# EUROPEAN PATENT APPLICATION

(11) **EP 4 678 642 A1**
(43) Date of publication of application: **14.01.2026**
(21) Application number: 24766338.8
(22) Date of filing: 29.02.2024
(51) Int. Cl.: C07D 471/04, C07D 401/14, A61K 45/06, A61P 35/00

(54) **NITROGEN-CONTAINING HETEROCYCLIC COMPOUND, PREPARATION METHOD THEREFOR, AND USE THEREOF**

(30) Priority: 03.03.2023 CN 202310201142; 27.10.2023 CN 202311421459
(71) Applicant: Bivision Pharmaceuticals, Inc, Nanjing, Jiangsu 211500 (CN)
(72) Inventor: WANG, Eric Yanjun, Nanjing, Jiangsu 211500 (CN); HE, Jinqiu, Nanjing, Jiangsu 211500 (CN); YU, Haihua, Nanjing, Jiangsu 211500 (CN); WANG, Kevin Yu, Nanjing, Jiangsu 211500 (CN); SUN, Yueguang, Nanjing, Jiangsu 211500 (CN); ZHAN, Jian, Nanjing, Jiangsu 211500 (CN); YU, Yaoming, Nanjing, Jiangsu 211500 (CN)
(74) Representative: Michalski Hüttermann & Partner Patentanwälte mbB
(86) International application number: PCT/CN2024/079446
(87) International publication number: WO 2024/183618

(57) **Abstract**

Disclosed in the present invention are a nitrogen-containing heterocyclic compound, a preparation method therefor, and a use thereof. Specifically disclosed are a compound represented by formula I, a pharmaceutically acceptable salt thereof, a solvate thereof, or a solvate of the pharmaceutically acceptable salt thereof. The compound can be used for the diagnosis and treatment of FAP-associated tumors, achieves high tumor uptake and long retention, and has a wide application prospect.

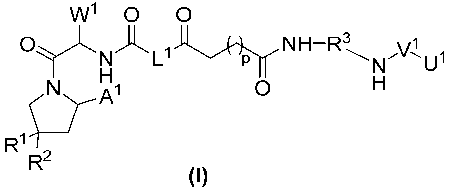

## Description

The present application claims the priorities of Chinese patent application 2023102011423 filed on March 03, 2023 and Chinese patent application 2023114214594 filed on October 27, 2023. The contents of the above Chinese patent applications are incorporated herein by reference in their entirety.

### TECHNICAL FIELD

The present disclosure relates to nitrogen-containing heterocyclic compounds, preparation methods therefor, and uses thereof.

### BACKGROUND

Chemotherapy remains widely used in the treatment of cancer patients and other diseases. Traditional anticancer chemotherapy drugs act on fundamental mechanisms of cell survival and cannot effectively distinguish between healthy and malignant cells. Moreover, these drugs do not efficiently reach or accumulate at the disease site after systemic administration. Non-specific mechanisms of action and low efficiency in tumor site localization are the reasons for side effects and poor therapeutic effects.

In order to treat diseases more effectively, the development of targeted drugs has been one of the hot topics in new drug research and development in recent years. These drugs can selectively localize at the disease site and exert their effects after systemic administration. Such drugs are substances formed by combining representative chemicals with therapeutic effects (such as cytotoxic drugs or radionuclides) and ligands with specificity for targeting cells. Disease-specific monoclonal antibodies, peptides, and small-molecule ligands are the preferred ligands for developing targeted drug products. For targeted applications, the use of small molecule ligands offers more rapid and efficient tumor penetration, lower immunogenicity, and lower manufacturing costs than larger molecules such as peptides and antibodies.

Tumors are complexes composed of tumor cells and their surrounding stromal cells and non-cellular components. The occurrence and progression of tumors represent a dynamic process of mutual promotion and co-evolution between tumor cells and their microenvironment (tumor microenvironment, TME). The tumor microenvironment is composed of many heterogeneous cell types, such as immune cells, including endothelial cells, cancer associated fibroblasts (CAFs), and their extracellular products. Among them, cancer associated fibroblasts (CAFs) are the most important stromal cells in the tumor microenvironment, accounting for about 50% of the total number of tumor tissue cells. CAFs play an important role in tumor growth, metastasis, drug resistance, treatment resistance, *etc.,* it is one of the hot topics in tumor diagnosis and treatment research in recent years.

A prominent feature of CAFs is the high expression of seprase or fibroblast activation protein (FAP). Both are the same cell-surface transmembrane serine proteases, possessing dual activities as dipeptidyl peptidase (DPP) and collagenase, capable of degrading dipeptides and type I collagen. FAP and dipeptidyl peptidase IV (PPIV) have similar structural domains and dipeptidyl peptidase activity and belong to the same serine protease family. However, FAP possesses unique endopeptidase activity, capable of cleaving gelatin, denatured type I collagen, and α2-antiplasmin, whereas DPPIV lacks this function, which is distinguished. FAP is selectively expressed on the surface of stromal fibroblasts in 90% or more of epithelial malignancies, including breast cancer, ovarian cancer, lung cancer, colorectal cancer, gastric cancer, pancreatic cancer, cutaneous melanoma, *etc.* FAP is typically not expressed in benign and precancerous epithelial tumors, such as colorectal adenomas, breast phyllodes tumors, and fibroadenomas. FAP is generally not expressed in normal human tissues, but only exists in the cervix and endometrium, and is expressed briefly during embryonic development. Numerous studies have shown that the high expression of FAP in CAFs of epithelial tumors is associated with poorer patient prognosis, indicating that its activity level is related to cancer progression as well as the metastasis and spread of cancer cells.

Radiopharmaceuticals are medical preparations composed of radioactive isotopes paired with molecular agents that target specific organs and tissues. They are radioactive drugs that can be used for imaging diagnosis and clinical treatment. Based on their uses, they can be categorized into diagnostic radiopharmaceuticals and therapeutic radiopharmaceuticals.

Diagnostic radiopharmaceuticals include two categories: drugs for organ imaging and drugs for functional determination. When combined with SPECT or PET, they enable the study of drug functions and metabolic processes *in vivo* at the molecular level, achieving rapid, non-destructive, and real-time imaging of physiological and pathological processes. This provides a means for truly early diagnosis and timely treatment.

Therapeutic radiopharmaceuticals refer to radioactive drugs that, when administered orally or intravenously to patients, highly selectively accumulate in diseased tissues. The rays radiated by radioisotopes produce local ionizing radiation biological effects, thereby inhibiting or destroying the diseased tissues to achieve therapeutic effects.

In recent years, the use of FAP as a target in tumor diagnosis and treatment has received widespread attention. In diagnosis, compared with FDG imaging, FAP-targeted imaging exhibits lower background activity in organs such as the brain and liver, while demonstrating higher detection rates for tumor lesions. However, to date, such probes have not shown particularly effective therapeutic effects, primarily because the biological activity of such compounds is often relatively low, resulting in limited uptake at the lesion sites. In addition, from the perspective of ligand selection, the binding of ligands and receptors showed a one-to-one correspondence, and the stability of binding to receptors was poor, and the low lesion uptake greatly limited the diagnosis and treatment effect. Therefore, using FAP as a specific molecular target with nuclear medical imaging is a promising strategy for several applications, including the early diagnosis of malignant tumors, accurate tumor staging, and both companion diagnostics and efficacy evaluation of cancer treatments.

### SUMMARY

The purpose of the present disclosure is to solve the problems currently existing in this technical field, such as insufficient affinity of compounds for FAP, excessively short retention time at target sites, or inadequate uptake at lesion sites. The present disclosure provides a nitrogen-containing heterocyclic compound, a preparation method therefor, and a use thereof, which has the advantages of simple preparation, good stability, high tumor uptake, long retention, *etc.,* making it suitable for clinical promotion and application.

The present disclosure provides a compound of formula I, a pharmaceutically acceptable salt thereof, a solvate thereof, or a solvate of the pharmaceutically acceptable salt thereof; 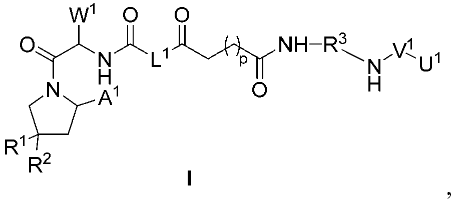
wherein R¹ and R² are independently hydrogen, halogen, C₁₋₆ alkyl, C₁₋₆ alkoxy, or hydroxyl, and R¹ and R² are not simultaneously C₁₋₆ alkyl, C₁₋₆ alkoxy, or hydroxyl;
A¹ is -CN, -B(OH)₂, -CONH₂, or -COOR^{a};
R^{a} is hydrogen or C₁₋₆ alkyl;
W¹ is hydrogen or C₁₋₄ alkyl;
L¹ is 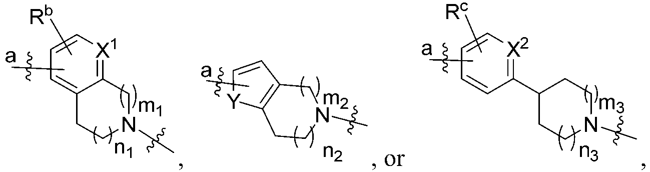 wherein the a-terminus is connected to the carbonyl group in the amide bond on the left side;
X¹ and X² are independently N or CH;
Y is O, S, or NH;
R^{b} and R^{c} are independently hydrogen, halogen, C₁₋₆ alkyl, or C₁₋₆ alkoxy;
n₁, n₂, and n₃ are independently 0, 1, or 2;
m₁, m₂, and m₃ are independently 0, 1, or 2;
p is 1, 2, 3, or 4;
R³ is independently C₁₋₆ alkylene;
V¹ is a chemical bond, -C(=O)-C₁₋₆ alkylene-NH-, or -C(=O)-CH₂-(OCH₂CH₂)ₛ-NH-, wherein s is 1, 2, 3, or 4, and the -NH- in -C(=O)-C₁₋₆ alkylene-NH- and -C(=O)-CH₂-(OCH₂CH₂)ₛ-NH- is connected to U¹;
U¹ is a group containing a radioactive metal ion or a group capable of optical imaging.

In some embodiments, R¹ and R² are independently hydrogen or halogen.

In some embodiments, Y is S.

In some embodiments, A¹ is CN.

In some embodiments, W¹ is hydrogen or C₁₋₄ alkyl.

In some embodiments, R^{b} and R^{c} are independently hydrogen.

In some embodiments, p is 1.

In some embodiments, V¹ is a chemical bond or -C(=O)-C₁₋₆ alkylene-NH-.

In some embodiments, the group containing a radioactive metal ion is composed of a radioactive metal ion and a group having a function of chelating a metal ion, wherein the radioactive metal ion is chelated with the group having a function of chelating a metal ion.

In some embodiments, the group having a function of chelating a metal ion is 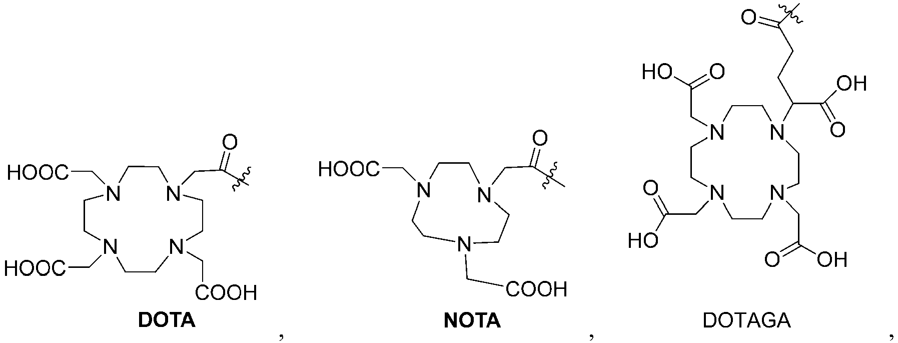 or 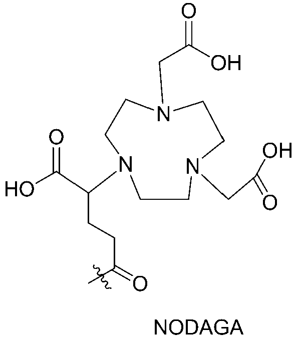 , for example, 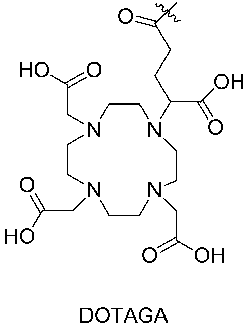 or

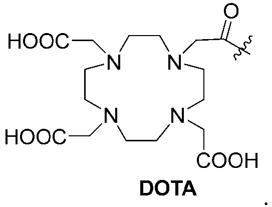

In some embodiments, the radioactive metal ion has one or more of the following effects: (i) tracing; (ii) delivery; (iii) imaging (e.g., PET imaging or SPECT imaging); (iv) therapy.

In some embodiments, the radioactive metal ion is ¹⁷⁷Lu³⁺, ⁶⁸Ga³⁺, ⁶⁴Cu²⁺, ²²⁵Ac³⁺, ⁹⁰Y³⁺, ⁸⁹Zr⁴⁺, ²¹²Pb²⁺, ²¹³Bi³⁺, or ²²⁷Th⁴⁺, such as¹⁷⁷Lu³⁺ or ⁶⁸Ga³⁺.

In some embodiments, the group capable of optical imaging may be a fluorescent group, such as cy3, cy5, or cy7.

In some embodiments, the compound of formula I is a compound of formula Ia:

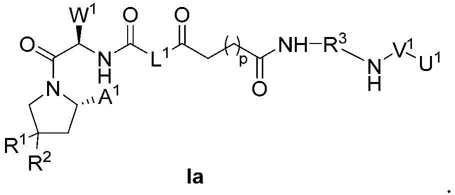

In some embodiments, R¹ and R² are independently hydrogen or halogen;
A¹ is CN;
W¹ is hydrogen or C₁₋₄ alkyl;
L¹ is 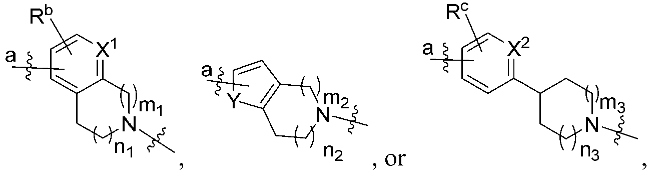 wherein the a-terminus is connected to the carbonyl group in the amide bond on the left side;
X¹ and X² are independently N or CH;
Y is S;
R^{b} and R^{c} are independently hydrogen;
n₁, n₂, and n₃ are independently 0, 1, or 2;
m₁, m₂, and m₃ are independently 0, 1, or 2;
p is 1;
R³ is C₁₋₆ alkylene;
V¹ is a chemical bond or -C(=O)-C₁₋₆ alkylene-NH-, wherein the -NH- in -C(=O)-C₁₋₆ alkylene-NH- is connected to U¹;
U¹ is a group containing a radioactive metal ion or a group capable of optical imaging.

In some embodiments, R¹ and R² are independently hydrogen or fluorine.

In some embodiments, W¹ is hydrogen or methyl.

In some embodiments, L¹ is 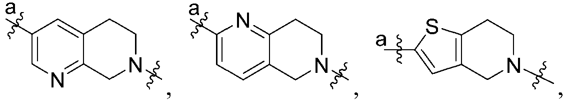 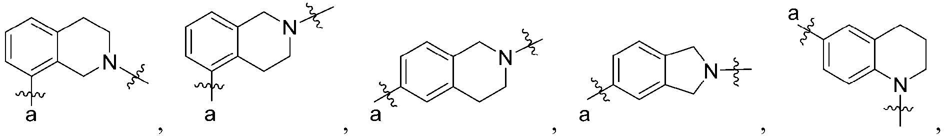 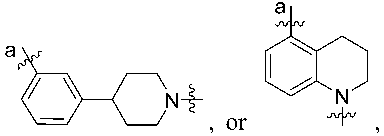 wherein the a-terminus is connected to the carbonyl group in the amide bond on the left side.

In some embodiments, R³ is -CH₂CH₂-.

In some embodiments, V¹ is a chemical bond or 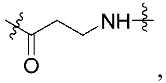 wherein the -NH- in 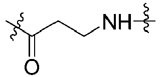 is connected to U¹.

In some embodiments, U¹ is 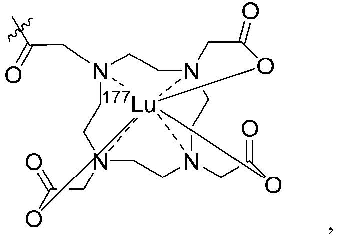 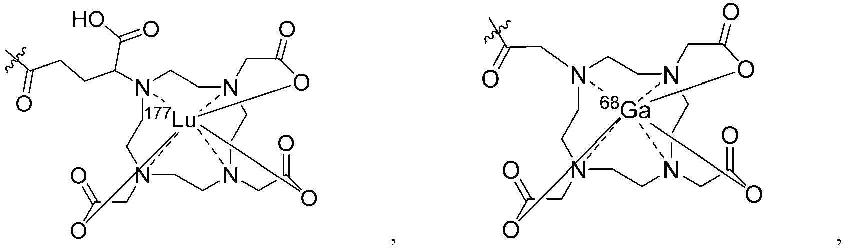 or

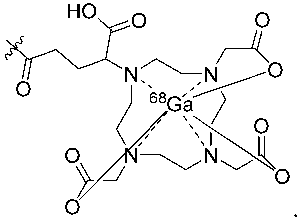

In some embodiments, the compound of formula I is a compound formed by chelating the radioactive metal ion with compound A, wherein the structure of compound A is any one of the following:

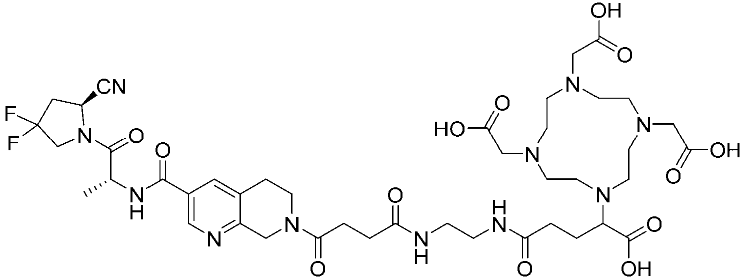

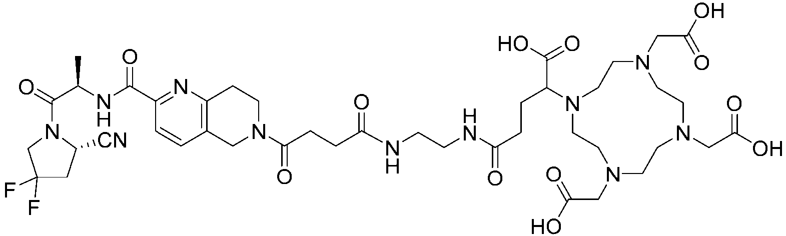

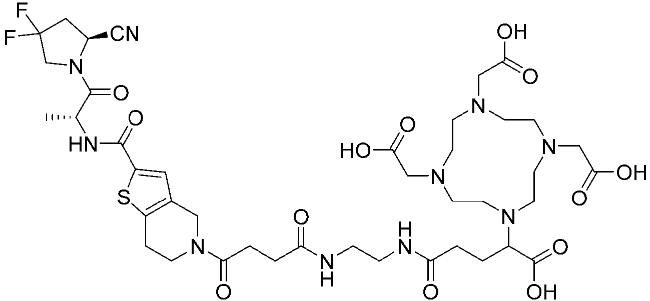

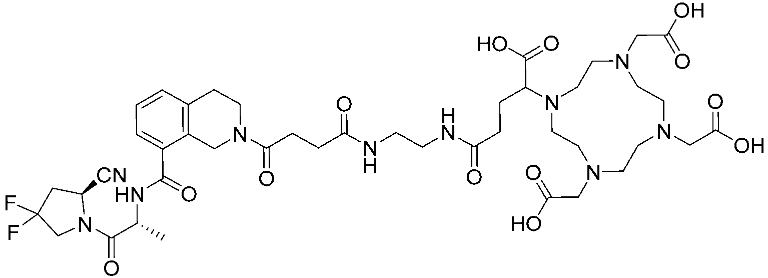

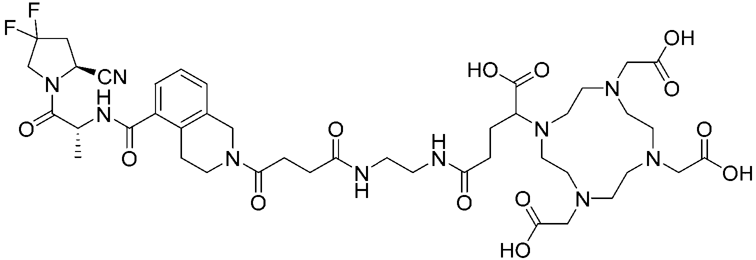

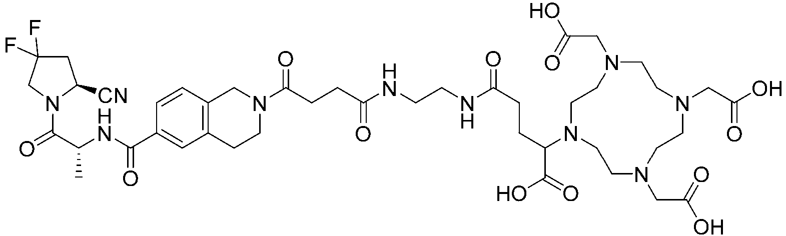

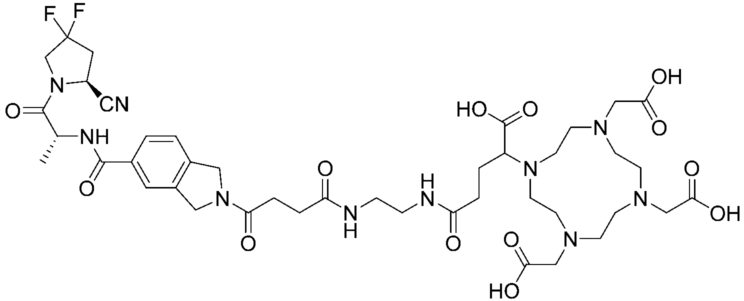

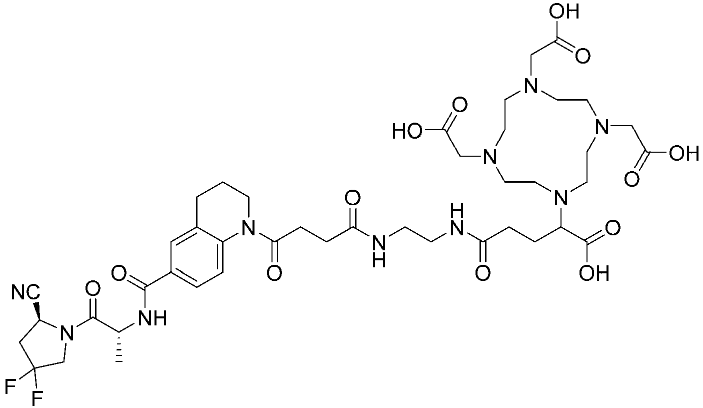

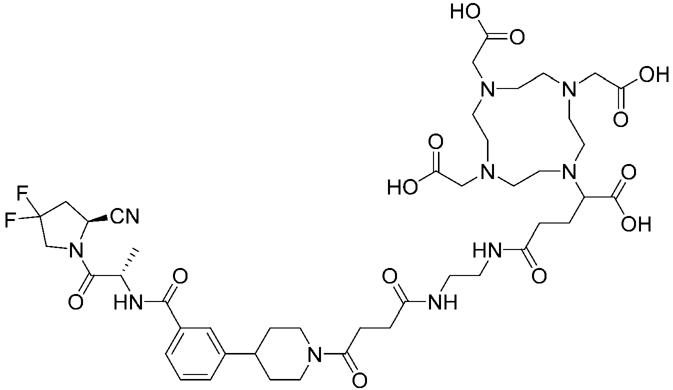

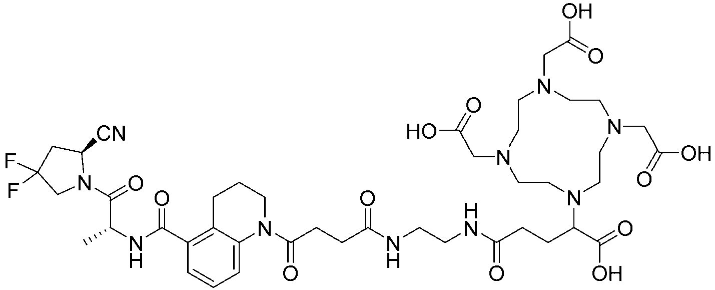

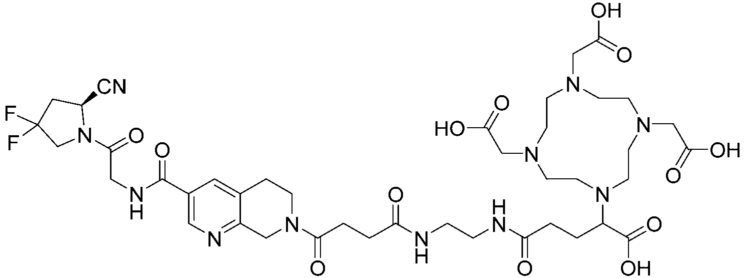

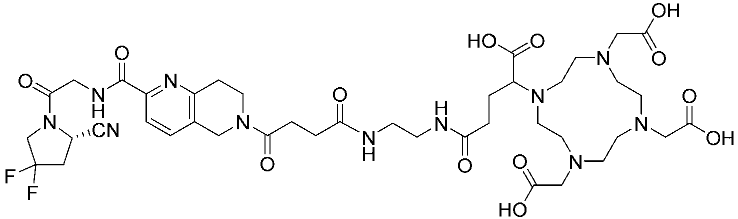

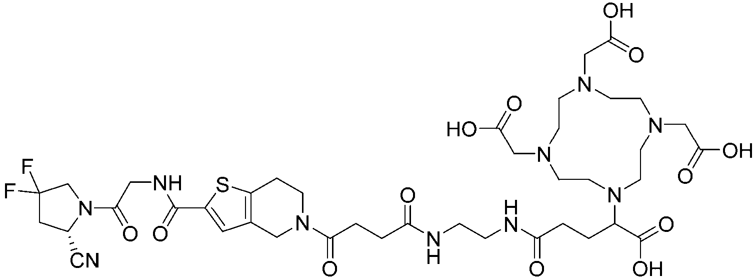

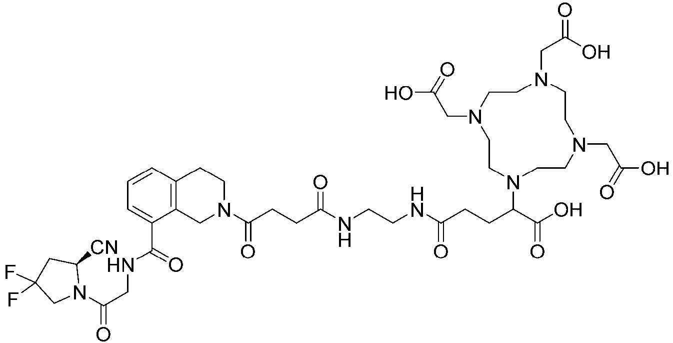

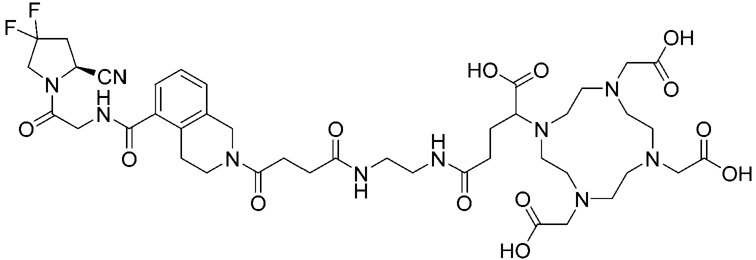

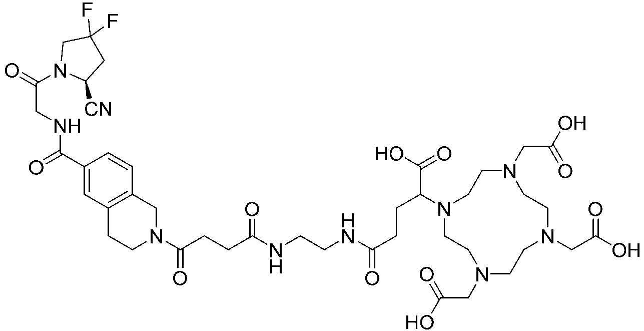

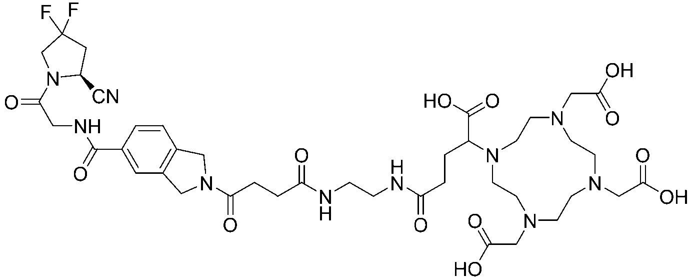

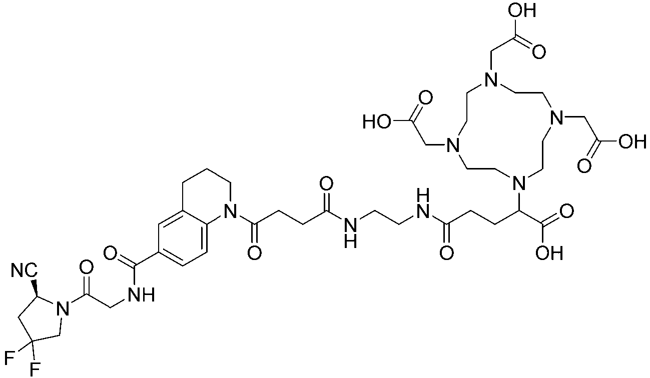

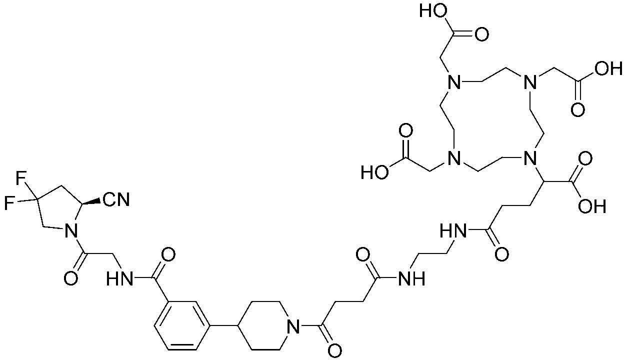

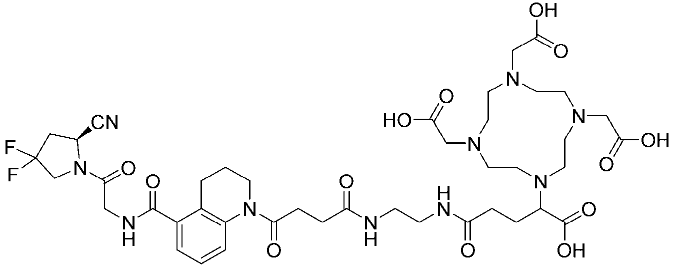

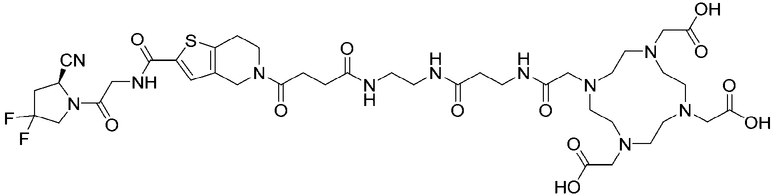

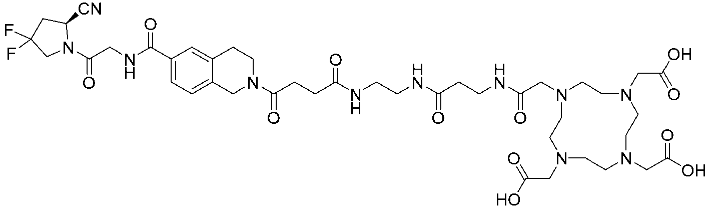

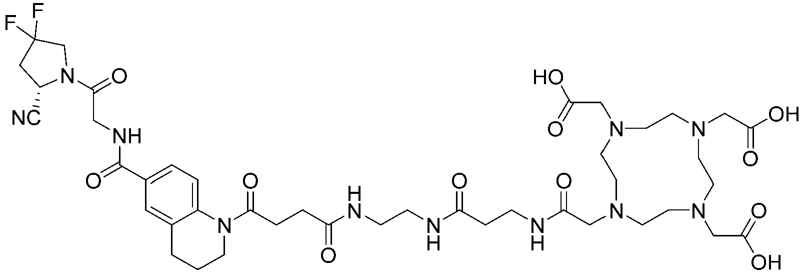

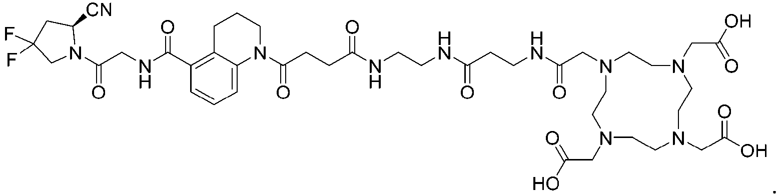

In some embodiments, the compound of formula I is a compound formed by chelating ¹⁷⁷Lu³⁺ with compound A, wherein the structure of compound A is as described above.

In some embodiments, the compound of formula I is a compound formed by chelating ⁶⁸Ga³⁺ with compound A, wherein the structure of compound A is as described above.

The present disclosure also provides a preparation method for the above compound of formula I, comprising the following steps: chelating the radioactive metal ion with the compound of formula II; 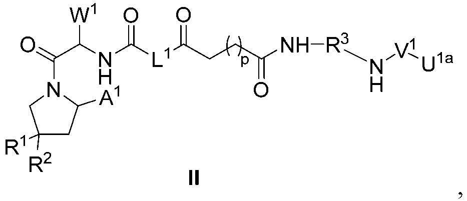 wherein U^{1a} is a group having a function of chelating a metal ion.

In the compound of formula II, the definition of the group having a function of chelating a metal ion may be as described above, for example, 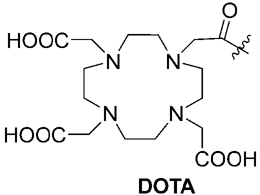 or

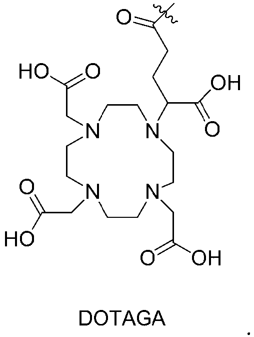

In the compound of formula II, the group having a function of chelating a metal ion is not chelated with a metal ion.

The present disclosure also provides a compound of formula II, a pharmaceutically acceptable salt thereof, a solvate thereof, or a solvate of the pharmaceutically acceptable salt thereof, 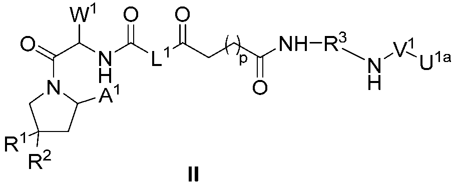 wherein U^{1a}, R¹, R², A¹, W¹, L¹, p, R³, and V¹ are as defined above.

In some embodiments, the compound of formula II is a compound of formula IIa:

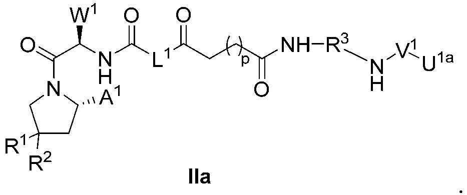

In some embodiments, the compound of formula II is any one of compound A as described above.

The present disclosure provides a compound of formula III, a pharmaceutically acceptable salt thereof, a solvate thereof, or a solvate of the pharmaceutically acceptable salt thereof; 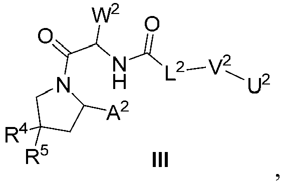
wherein R⁴ and R⁵ are independently hydrogen, halogen, C₁₋₆ alkyl, C₁₋₆ alkoxy, or hydroxyl, and R⁴ and R³ are not simultaneously C₁₋₆ alkyl, C₁₋₆ alkoxy, or hydroxyl;
A² is -CN, -B(OH)₂, -CONH₂, or -COOR^{d};
R^{d} is hydrogen or C₁₋₆ alkyl;
W² is hydrogen, C₁₋₄ alkyl, or HO-C₁₋₄ alkyl-;
L² is phenyl, phenyl substituted by one or more R^{e}, or 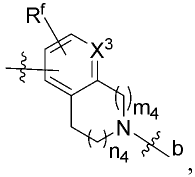 wherein the b-terminus is connected to V²;
R^{e} and R^{f} are independently hydrogen, halogen, C₁₋₆ alkyl, or C₁₋₆ alkoxy;
m₄ and n₄ are independently 0, 1, or 2;
X³ is CH or N;
V² is a chemical bond, -C₁₋₆ alkylene-NH-, or -C(=O)-C₁₋₆ alkylene-C(=O)-5- to 10-membered heterocycloalkyl, wherein the -NH-in -C₁₋₆ alkylene-NH- is connected to U², and the 5- to 10-membered heterocycloalkyl terminus in -C(=O)-C₁₋₆ alkylene-C(=O)-5- to 10-membered heterocycloalkyl is connected to U²;
U² is a group containing a radioactive metal ion or a group capable of optical imaging.

In some embodiments, R⁴ and R⁵ are independently hydrogen.

In some embodiments, A² is -B(OH)₂.

In some embodiments, R^{e} and R^{f} are independently hydrogen or halogen.

In some embodiments, X³ is CH.

In some embodiments, m₄ and n₄ are independently 1.

In some embodiments, when L² is phenyl or phenyl substituted by one or more R^{e}, then V² is -C₁₋₆ alkylene-NH-, wherein the -NH- in -C₁₋₆ alkylene-NH- is connected to U².

In some embodiments, when L² is 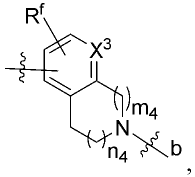 then V² is a chemical bond or -C(=O)-C₁₋₆ alkylene-C(=O)-5- to 10-membered heterocycloalkyl, wherein the 5- to 10-membered heterocycloalkyl terminus in -C(=O)-C₁₋₆ alkylene-C(=O)-5- to 10-membered heterocycloalkyl is connected to U².

In some embodiments, in the compound of formula III, the group containing a radioactive metal ion is composed of a radioactive metal ion and a group having a function of chelating a metal ion, wherein the radioactive metal ion is chelated with the group having a function of chelating a metal ion.

In some embodiments, in the compound of formula III, the group having a function of chelating a metal ion is 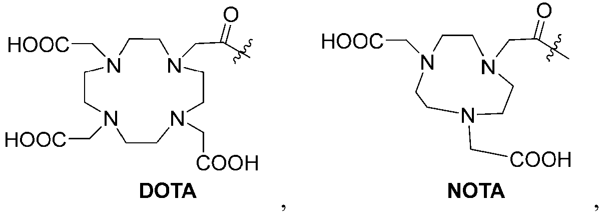 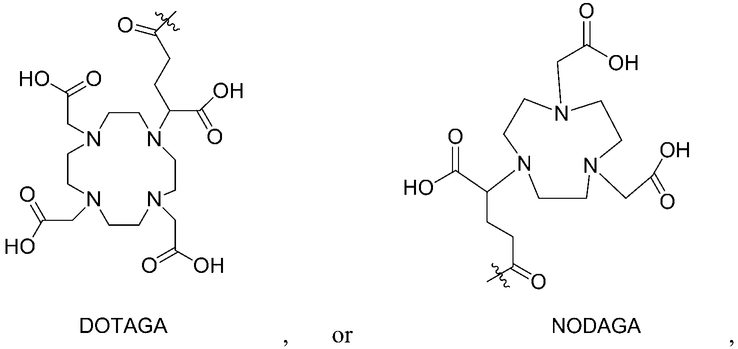 for example,

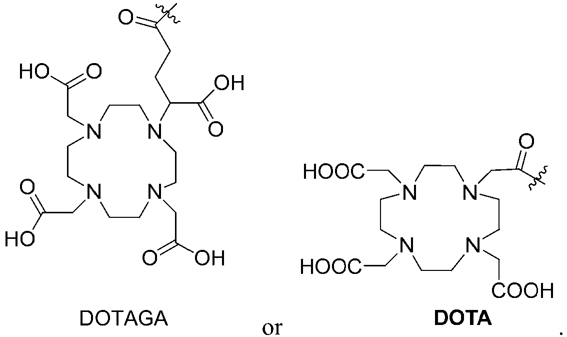

In some embodiments, in the compound of formula III, the radioactive metal ion has one or more of the following effects: (i) tracing; (ii) delivery; (iii) imaging (*e.g.*, PET imaging or SPECT imaging); (iv) therapy.

In some embodiments, in the compound of formula III, the radioactive metal ion is ¹⁷⁷Lu^{3+ 68}Ga³⁺, ⁶⁴Cu²⁺, ²²⁵Ac³⁺, ⁹⁰Y³⁺, ⁸⁹Zr⁴⁺, ²¹²Pb²⁺, ²¹³Bi³⁺, or ²²⁷Th⁴⁺, such as ¹⁷⁷Lu³⁺ or ⁶⁸Ga³⁺.

In some embodiments, in the compound of formula III, the group capable of optical imaging may be a fluorescent group, such as cy3, cy5, or cy7.

In some embodiments, the compound of formula III is a compound of formula IIIa:

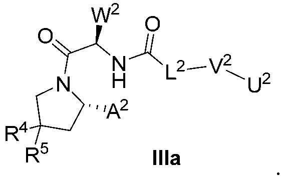

In some embodiments, R⁴ and R⁵ are independently hydrogen;
A² is -B(OH)₂;
W² is hydrogen, C₁₋₄ alkyl, or HO-C₁₋₄ alkyl-;
L² is phenyl, phenyl substituted by one or more R^{e}, or 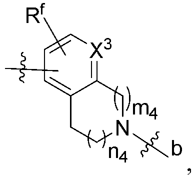 wherein the b-terminus is connected to V²;
R^{e} and R^{f} are independently hydrogen or halogen;
X³ is CH;
m₄ and n₄ are independently 1;
V² is a chemical bond, -C₁₋₆ alkylene-NH-, or -C(=O)-C₁₋₆ alkylene-C(=O)-5- to 10-membered heterocycloalkyl, wherein the -NH-in -C₁₋₆ alkylene-NH- is connected to U², and the 5- to 10-membered heterocycloalkyl terminus in -C(=O)-C₁₋₆ alkylene-C(=O)-5- to 10-membered heterocycloalkyl is connected to U²;
U² is a group containing a radioactive metal ion or a group capable of optical imaging.

In some embodiments, W² is methyl,

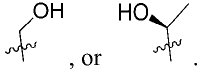

In some embodiments, L² is 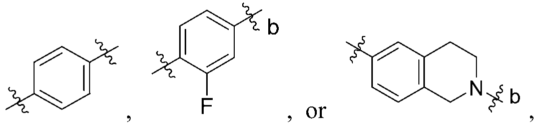 wherein the b-terminus is connected to V².

In some embodiments, V² is a chemical bond, -CH₂-NH-, or 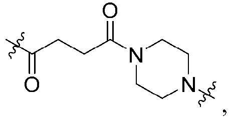 wherein the -NH- in -CH₂-NH- is connected to U², and the piperazine terminus in 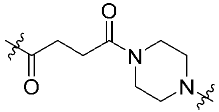 is connected to U².

In some embodiments, U² is 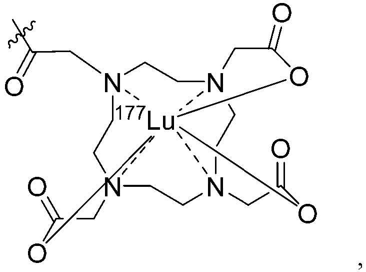 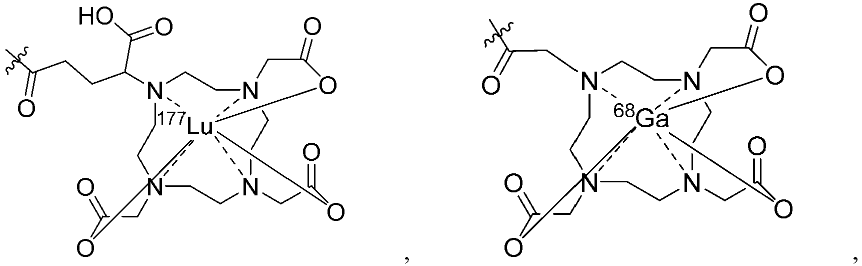 or

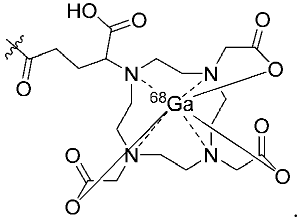

In some embodiments, the compound of formula III is a compound formed by chelating the radioactive metal ion with compound B, wherein the structure of compound B is any one of the following:

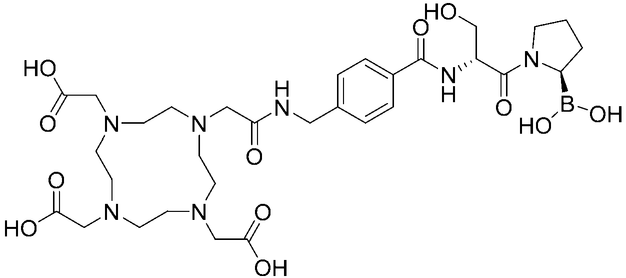

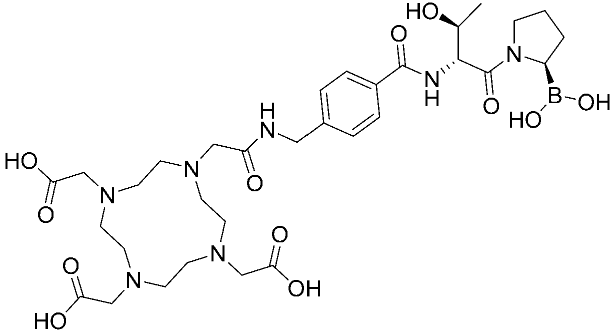

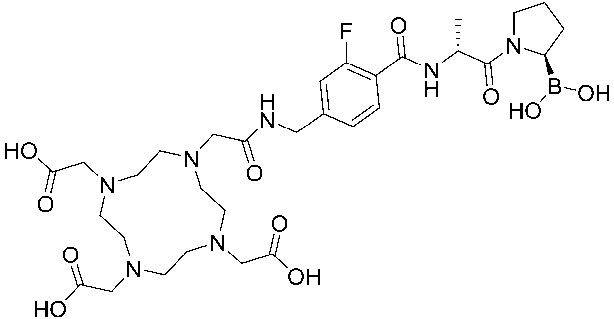

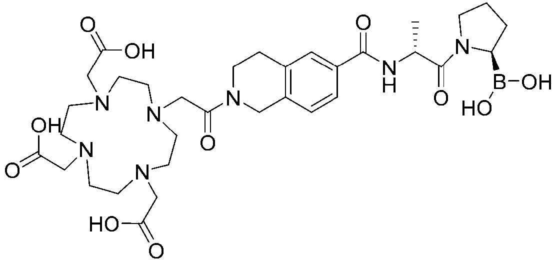

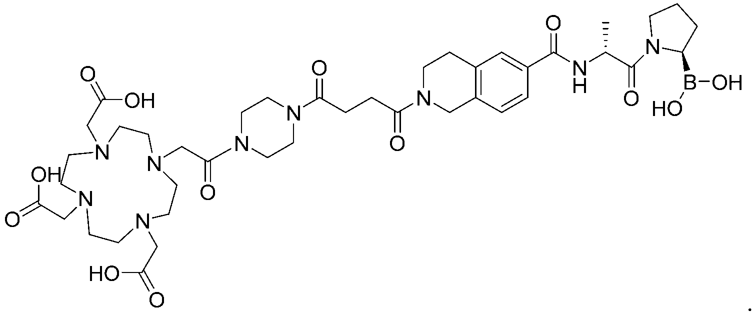

In some embodiments, the compound of formula III is a compound formed by chelating ¹⁷⁷Lu³⁺ with compound B, wherein the structure of compound B is as described above.

In some embodiments, the compound of formula III is a compound formed by chelating ⁶⁸Ga³⁺ with compound B, wherein the structure of compound B is as described above.

The present disclosure provides a preparation method for the above compound of formula III, comprising the following steps: chelating the radioactive metal ion with the compound of formula IV; 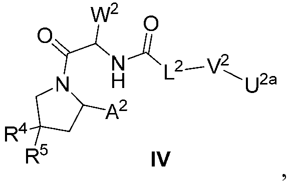 wherein U^{2a} is a group having a function of chelating a metal ion.

In some embodiments, in the compound of formula IV, the group having a function of chelating a metal ion is 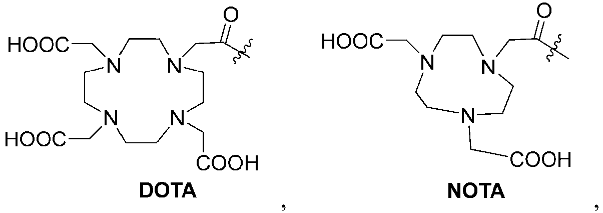 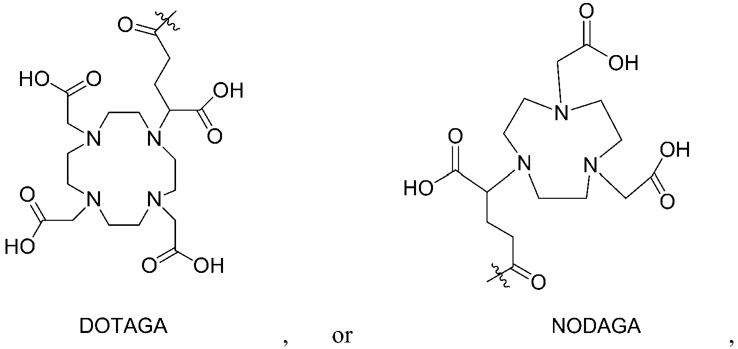 for example,

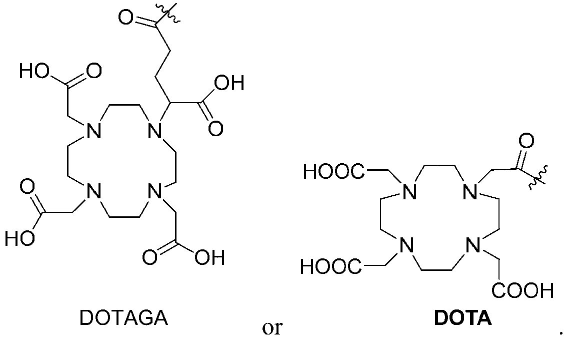

In the compound of formula IV, the group having a function of chelating a metal ion is not chelated with a metal ion.

The present disclosure provides a compound of formula IV, a pharmaceutically acceptable salt thereof, a solvate thereof, or a solvate of the pharmaceutically acceptable salt thereof, 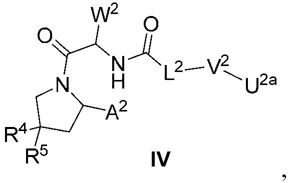 wherein R⁴, R⁵, A², W², L², V², and U^{2a} are as defined above.

In some embodiments, the compound of formula IV is a compound of formula IVa:

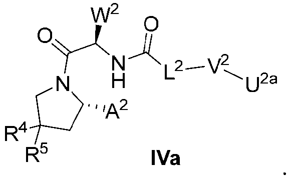

In some embodiments, the compound of formula IV is any one of compound B as described above.

The present disclosure provides a compound of formula V, a pharmaceutically acceptable salt thereof, a solvate thereof, or a solvate of the pharmaceutically acceptable salt thereof; 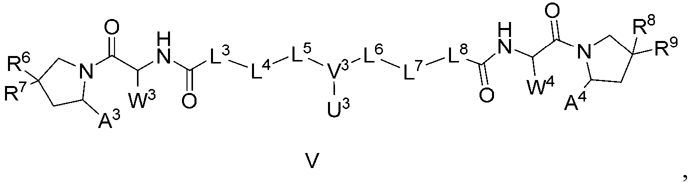
wherein R⁶, R⁷, R⁸, and R⁹ are independently hydrogen, halogen, C₁₋₆ alkyl, C₁₋₆ alkoxy, or hydroxyl, and R⁶ and R⁷ are not simultaneously C₁₋₆ alkyl, C₁₋₆ alkoxy, or hydroxyl, and R⁸ and R⁹ are not simultaneously C₁₋₆ alkyl, C₁₋₆ alkoxy, or hydroxyl;
A³ and A⁴ are independently -CN, -B(OH)₂, -CONH₂, or -COOR^{g};
R^{g} is hydrogen or C₁₋₆ alkyl;
W³ and W⁴ are independently hydrogen or C₁₋₄ alkyl;
L³ and L⁸ are independently phenyl, phenyl substituted by one or more R^{h}, 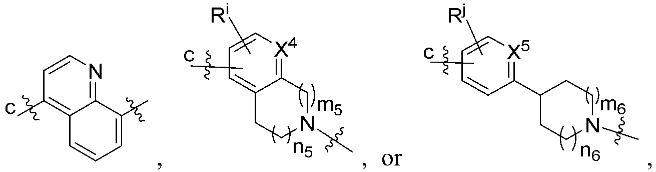 wherein the c-terminus is connected to the carbonyl group in the amide bond;
R^{h}, Rⁱ, and R^{j} are independently hydrogen, halogen, C₁₋₆ alkyl, or C₁₋₆ alkoxy;
X⁴ and X⁵ are independently CH or N;
m₅, m₆, n₅, and n₆ are independently 0, 1, or 2;
L⁴ and L⁷ are independently a chemical bond or C₁₋₆ alkylene;
L⁵ and L⁶ are independently 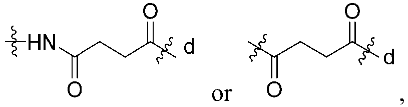 wherein the d-terminus is connected to V³;
V³ is 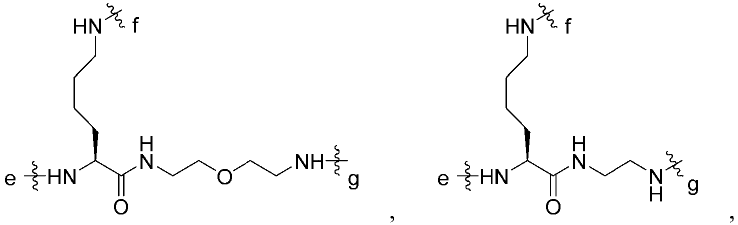 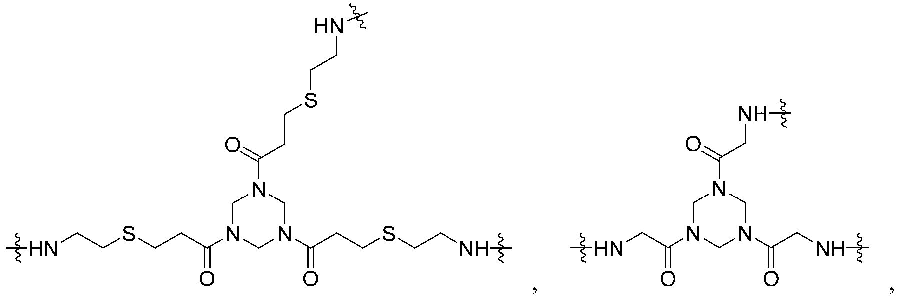 or 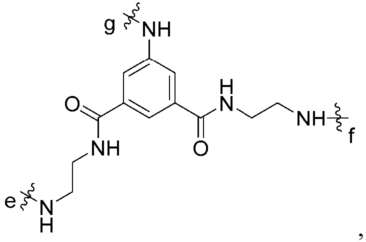 wherein the e-terminus is connected to L⁵ or L⁶, the f-terminus is connected to L⁵ or L⁶, and the g-terminus is connected to U³;
U³ is a group containing a radioactive metal ion or a group capable of optical imaging.

In some embodiments, R⁶, R⁷, R⁸, and R⁹ are independently hydrogen or halogen.

In some embodiments, A³ and A⁴ are independently -CN or -B(OH)₂.

In some embodiments, Rⁱ and R^{j} are independently hydrogen.

In some embodiments, X⁴ and X⁵ are independently CH.

In some embodiments, m₅, m₆, n₅, and n₆ are independently 1.

In some embodiments, when L³ and L⁸ are independently phenyl, then L⁴ and L⁷ are independently C₁₋₆ alkylene.

In some embodiments, when L³ and L⁸ are independently 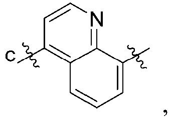 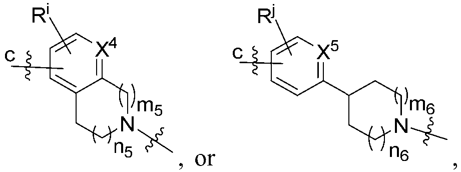 then L⁴ and L⁷ are independently a chemical bond.

In some embodiments, in the compound of formula V, the group containing a radioactive metal ion is composed of a radioactive metal ion and a group having a function of chelating a metal ion, wherein the radioactive metal ion is chelated with the group having a function of chelating a metal ion.

In some embodiments, in the compound of formula V, the group having a function of chelating a metal ion is 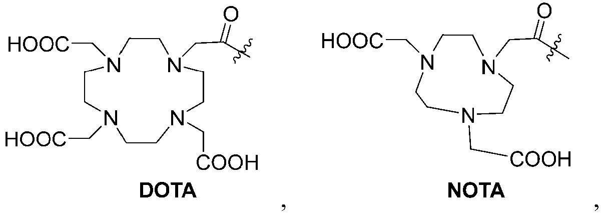 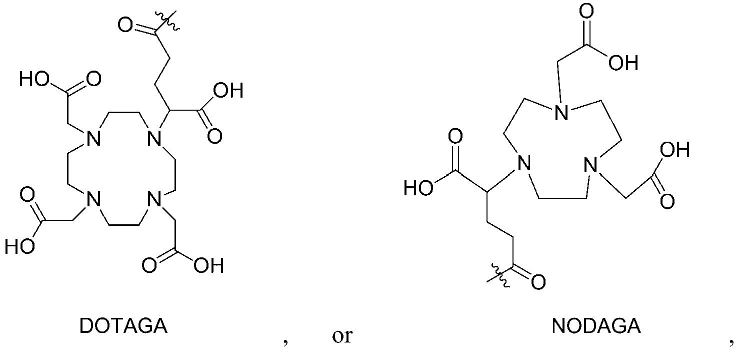 for example,

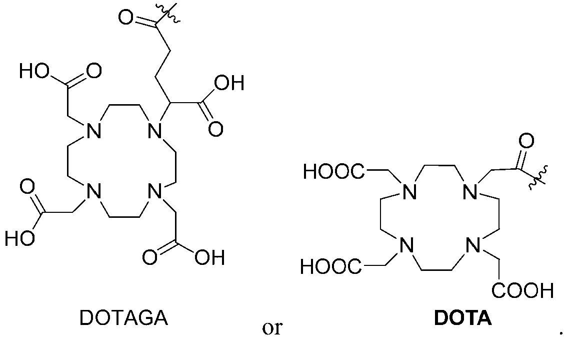

In some embodiments, in the compound of formula V, the radioactive metal ion has one or more of the following effects: (i) tracing; (ii) delivery; (iii) imaging (*e.g*., PET imaging or SPECT imaging); (iv) therapy.

In some embodiments, in the compound of formula V, the radioactive metal ion is ¹⁷⁷Lu³⁺, ⁶⁸Ga³⁺, ⁶⁴Cu²⁺, ²²⁵Ac³⁺, ⁹⁰Y³⁺, ⁸⁹Zr⁴⁺, ²¹²Pb²⁺, ²¹³Bi³⁺, or ²²⁷Th⁴⁺, such as ¹⁷⁷Lu³⁺ or ⁶⁸Ga³⁺.

In some embodiments, in the compound of formula V, the group capable of optical imaging may be a fluorescent group, such as cy3, cy5, or cy7.

In some embodiments, the compound of formula V is a compound of formula Va:

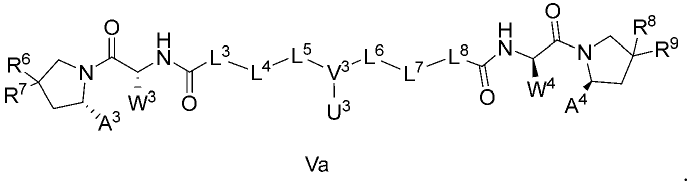

In some embodiments, R⁶, R⁷, R⁸, and R⁹ are independently hydrogen or halogen;
A³ and A⁴ are independently -CN or -B(OH)₂;
W³ and W⁴ are independently hydrogen or C₁₋₄ alkyl;
L³ and L⁸ are independently phenyl, 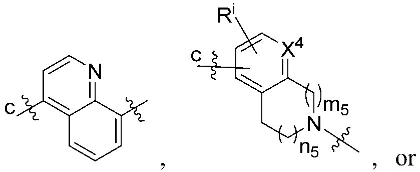 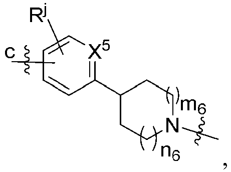 wherein the c-terminus is connected to the carbonyl group in the amide bond;
Rⁱ and R^{j} are independently hydrogen;
X⁴ and X⁵ are independently CH;
m₅, m₆, n₅, and n₆ are independently 1;
L⁴ and L⁷ are independently a chemical bond or C₁₋₆ alkylene;
L⁵ and L⁶ are independently 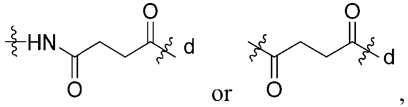 wherein the d-terminus is connected to V³;
V³ is 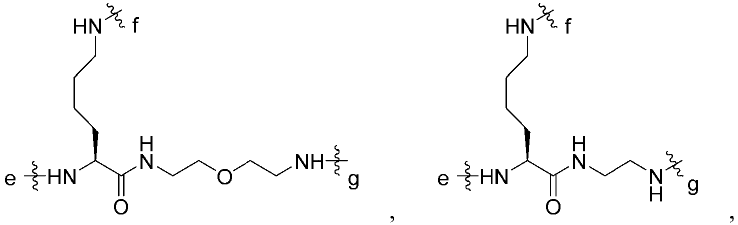 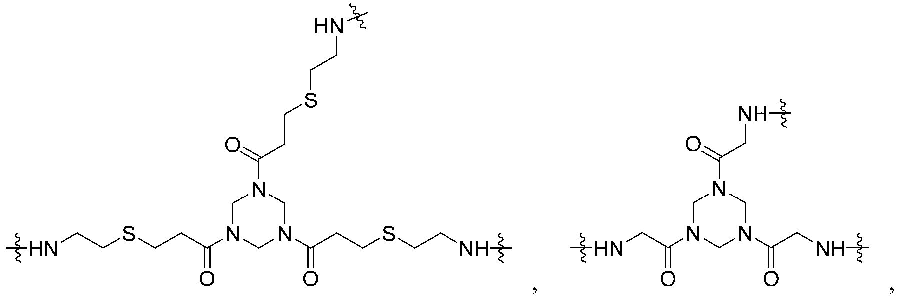 or 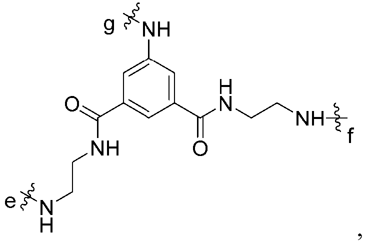 wherein the e-terminus is connected to L⁵ or L⁶, the f-terminus is connected to L⁵ or L⁶, and the g-terminus is connected to U³;
U³ is a group containing a radioactive metal ion or a group capable of optical imaging.

In some embodiments, R⁶, R⁷, R⁸, and R⁹ are independently hydrogen or fluorine.

In some embodiments, W³ and W⁴ are independently hydrogen or methyl.

In some embodiments, L³ and L⁸ are independently 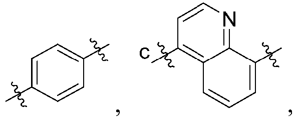 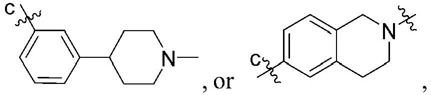 wherein the c-terminus is connected to the carbonyl group in the amide bond.

In some embodiments, -L³-L⁴-L⁵- is 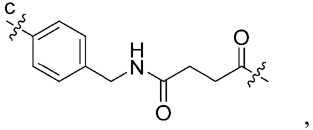 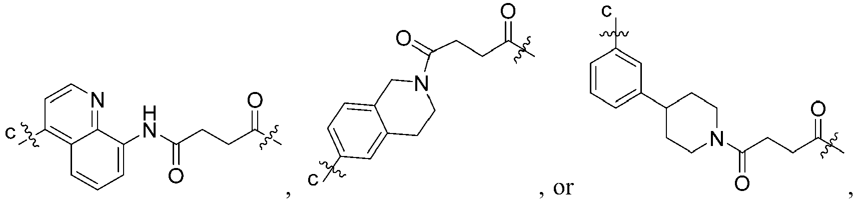 wherein the c-terminus is connected to the carbonyl group in the amide bond.

In some embodiments, -L⁶-L⁷-L⁸- is 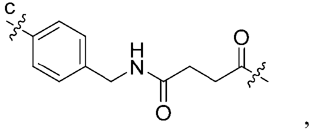 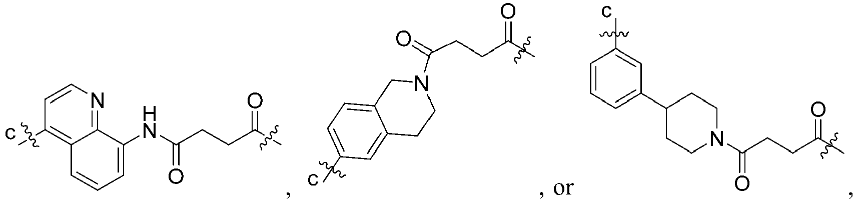 wherein the c-terminus is connected to the carbonyl group in the amide bond.

In some embodiments, U³ is

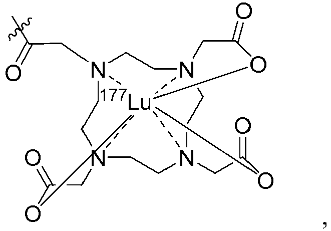

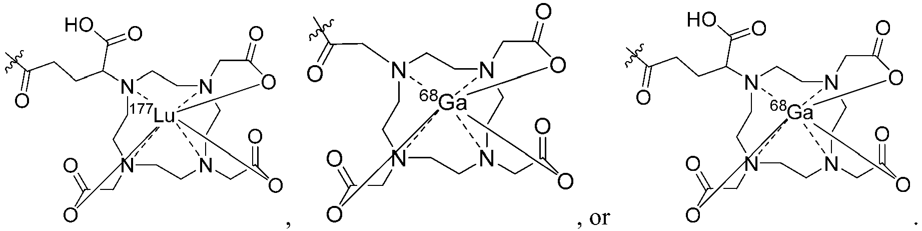

In some embodiments, the compound of formula V is a compound formed by chelating the radioactive metal ion with compound C, wherein the structure of compound C is any one of the following:

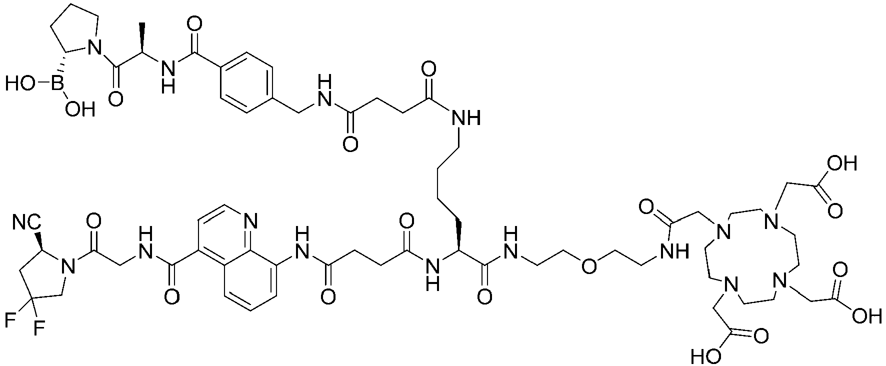

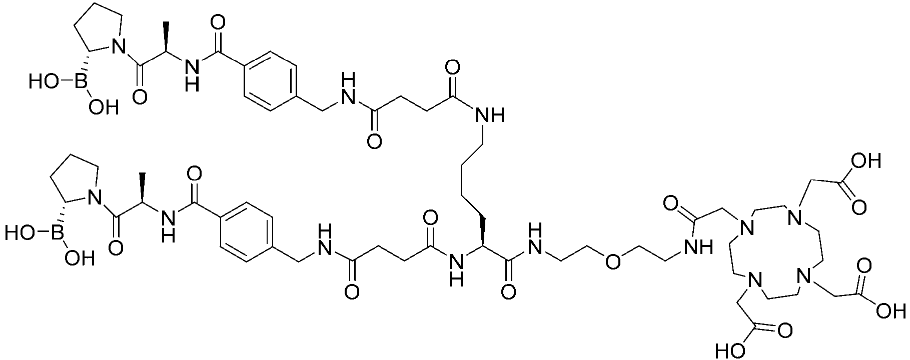

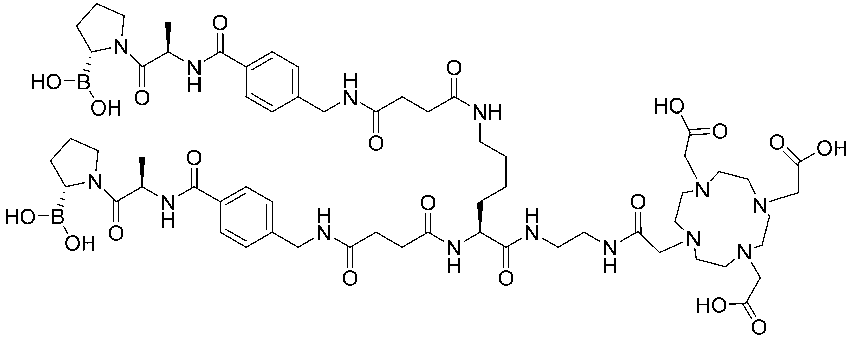

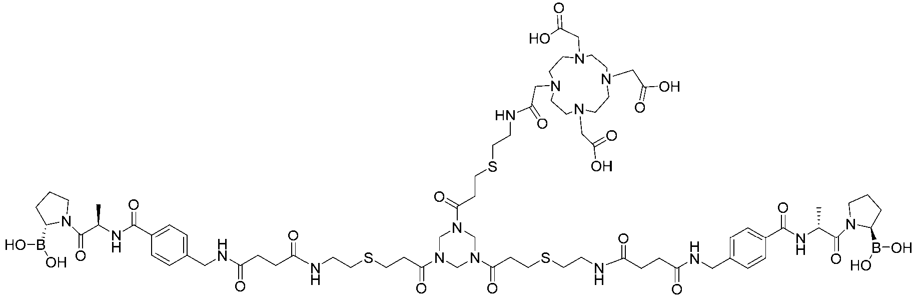

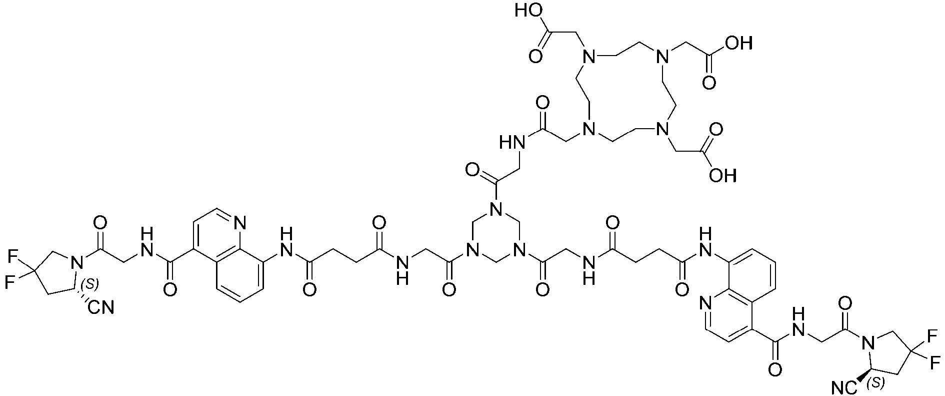

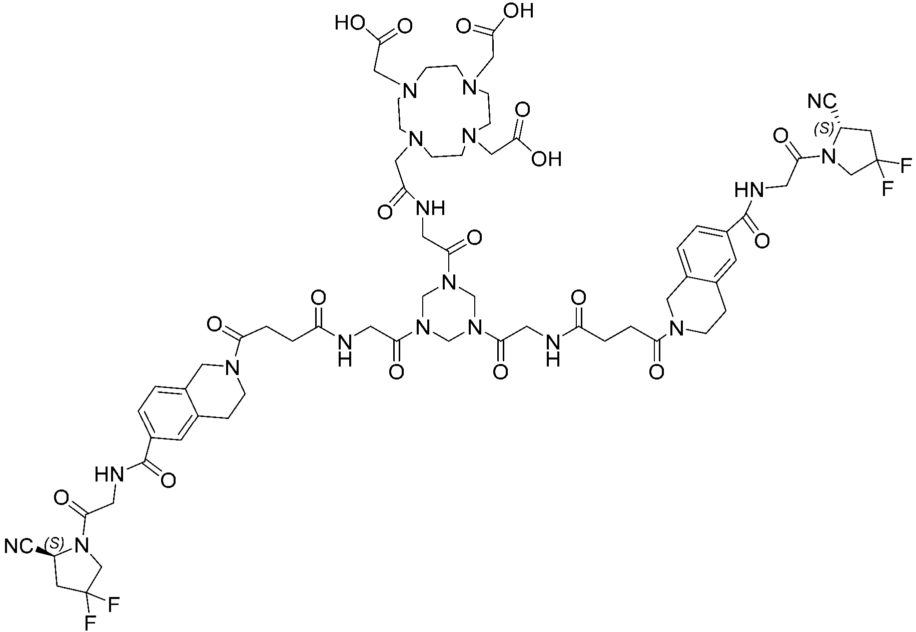

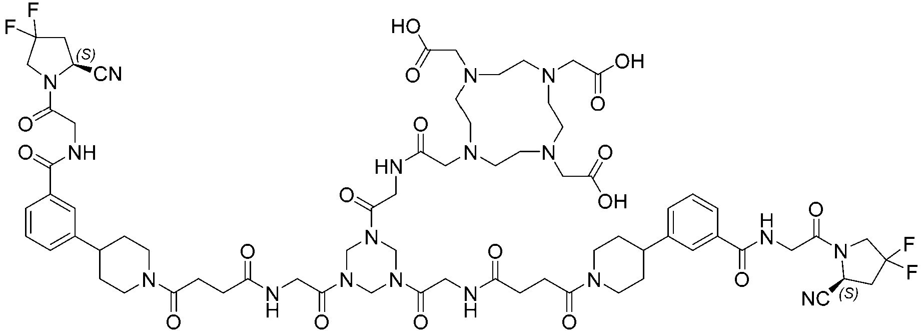

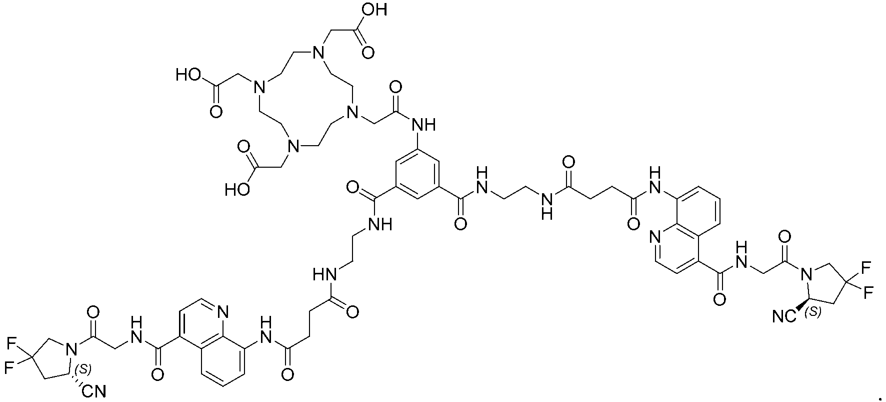

In some embodiments, the compound of formula V is a compound formed by chelating ¹⁷⁷Lu³⁺ with compound C, wherein the structure of compound C is as described above.

In some embodiments, the compound of formula V is a compound formed by chelating ⁶⁸Ga³⁺ with compound C, wherein the structure of compound C is as described above.

The present disclosure provides a preparation method for the above compound of formula V, comprising the following steps: chelating the radioactive metal ion with the compound of formula VI; 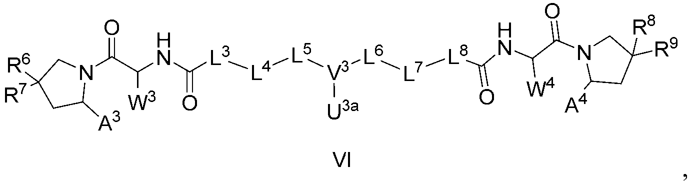 wherein U^{3a} is a group having a function of chelating a metal ion.

In some embodiments, in the compound of formula VI, the group having a function of chelating a metal ion is 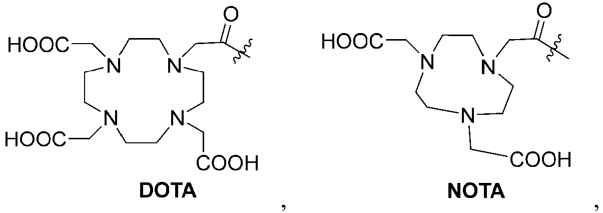 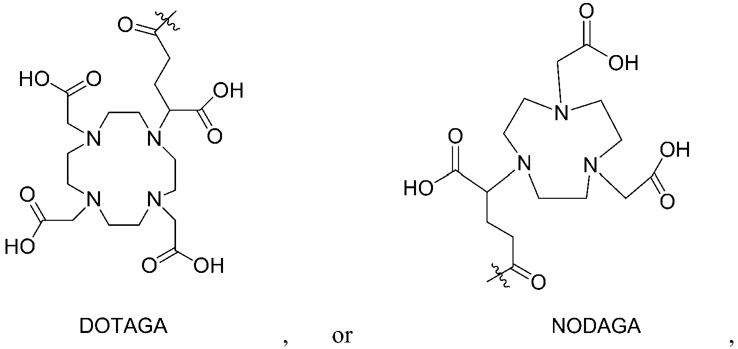 for example,

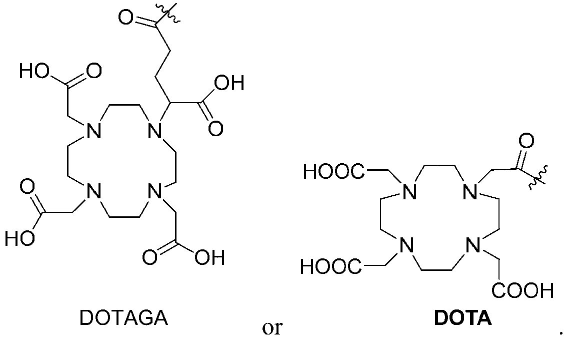

In the compound of formula VI, the group having a function of chelating a metal ion is not chelated with a metal ion.

The present disclosure provides a compound of formula VI, a pharmaceutically acceptable salt thereof, a solvate thereof, or a solvate of the pharmaceutically acceptable salt thereof, 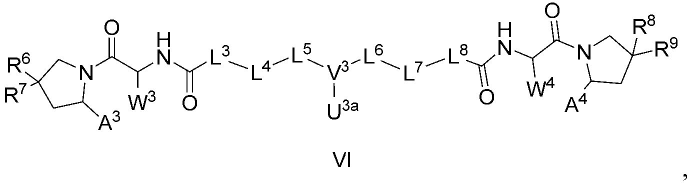 wherein R⁶, R⁷, R⁸, R⁹, A³, W³, W⁴, L³, L⁴, L⁵, L⁶, L⁷, L⁸, V³, and U^{3a} are as defined above.

In some embodiments, the compound of formula VI is a compound of formula VIa,

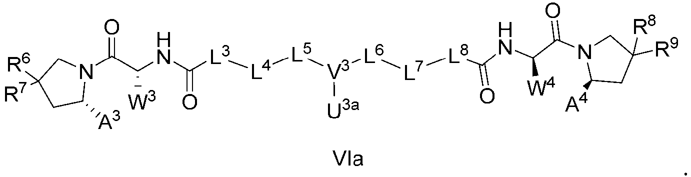

In some embodiments, the compound of formula VI is any one of compound C as described above.

The present disclosure provides a compound of formula VII, a pharmaceutically acceptable salt thereof, a solvate thereof, or a solvate of the pharmaceutically acceptable salt thereof; 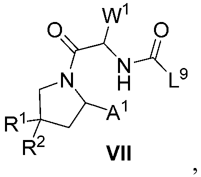
wherein L⁹ is 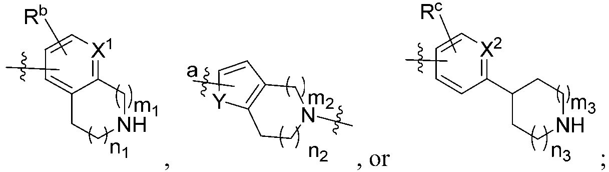
R¹, R², A¹, W¹, X¹, X², Y, R^{b}, R^{c}, m₁, m₂, m₃, n₁, n₂, and n₃ are as defined above.

In some embodiments, the compound of formula VII is a compound of formula VIIa:

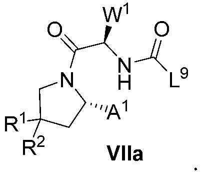

In some embodiments, L⁹ is

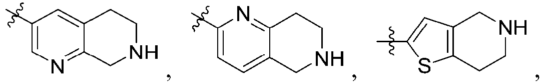

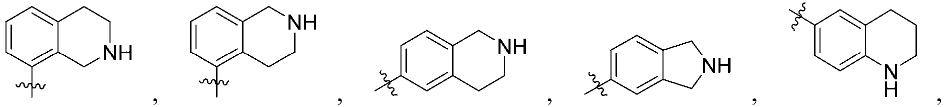

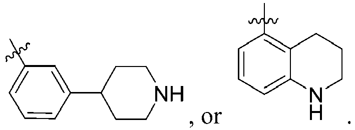

In some embodiments, the compound of formula VII is any one of the following compounds:

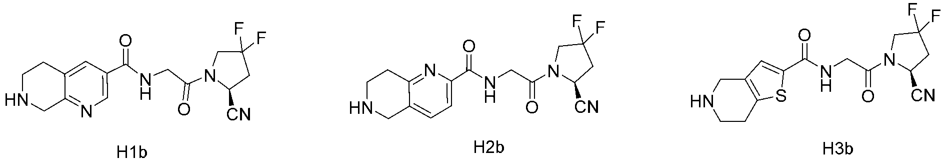

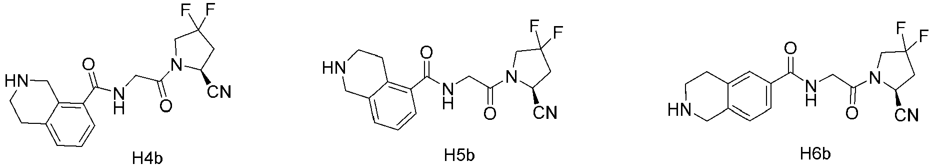

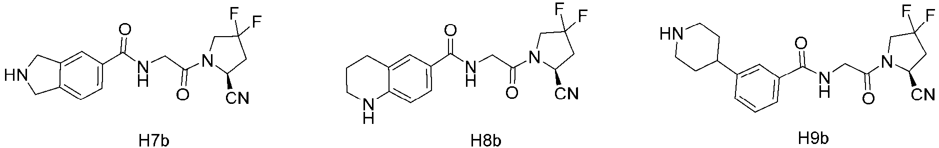

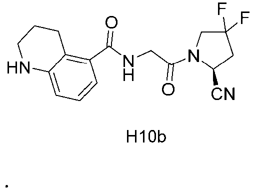

The present disclosure also provides a pharmaceutical composition comprising substance X and a pharmaceutically acceptable excipient; wherein substance X is substance Y, a pharmaceutically acceptable salt thereof, a solvate thereof, or a solvate of the pharmaceutically acceptable salt thereof, and substance Y is the compound of formula I, formula III, or formula V as described above.

In some embodiments, the pharmaceutical composition is a pharmaceutical composition for treating or diagnosing a tumor.

In some embodiments, the pharmaceutical composition is a pharmaceutical composition for tumor imaging.

In some embodiments, the tumor is an FAP-associated tumor, such as breast cancer, ovarian cancer, lung cancer, colorectal cancer, gastric cancer, pancreatic cancer, or cutaneous melanoma.

In some embodiments, the tumor is an FAP-positive solid tumor, such as breast cancer, ovarian cancer, lung cancer, colorectal cancer, gastric cancer, pancreatic cancer, or cutaneous melanoma.

In some embodiments, the substance X is a therapeutically effective amount of substance X.

The present disclosure also provides a use of substance X in the manufacture of a medicament; wherein the substance X is substance Y, a pharmaceutically acceptable salt thereof, a solvate thereof, or a solvate of the pharmaceutically acceptable salt thereof, and the substance Y is the compound of formula I, formula III, or formula V as described above;
the medicament is for treating or diagnosing a tumor, or tumor imaging.

In some embodiments, the medicament is for treating a tumor, and the radioactive metal ion is a radioactive metal ion that emits β-rays.

In some embodiments, when the medicament is for treating a tumor, the radioactive metal ion is ¹⁷⁷Lu³⁺

In some embodiments, when the medicament is for diagnosing a tumor, the radioactive metal ion is ⁶⁸Ga³⁺.

In some embodiments, the tumor is an FAP-associated tumor, such as breast cancer, ovarian cancer, lung cancer, colorectal cancer, gastric cancer, pancreatic cancer, or cutaneous melanoma.

In some embodiments, the tumor is an FAP-positive solid tumor, such as breast cancer, ovarian cancer, lung cancer, colorectal cancer, gastric cancer, pancreatic cancer, or cutaneous melanoma.

### Definitions and Explanations

Unless otherwise specified, the following terms and phrases used herein are intended to have the following meanings. A specific term or phrase that is not specifically defined should not be considered indefinite or unclear, but should be understood in its ordinary meaning. When a trade name appears herein, it is intended to refer to its corresponding commercial product or its active ingredient.

In the present disclosure, the term "substituted" or "substituent" means that a hydrogen atom in a group is replaced by a specified group. When the substitution position is not specified, substitution may occur at any position, provided that a stable or chemically feasible compound is formed. For example, a 5- to 12-membered heteroaryl substituted by one or more R^{3g} means that the hydrogen atom on the 5- to 12-membered heteroaryl is substituted by one or more R^{3g}, and when more than one R^{3g} is present, each R^{3g} is the same or different.

When any variable occurs in the constitution or structure of the compound more than once, the definition of the variable at each occurrence is independent. Thus, for example, if a group is substituted by one or more R^{e}, the R^{e} in each occurrence has independent options. Moreover, a combination of the substituent and/or the variant thereof is allowed only when the combination results in a stable compound.

When the linking groups listed in the present disclosure do not specify their direction of connection, the direction of connection may be arbitrary, including both left-to-right and right-to-left connections. For example, in -A-L-B, if the linking group L is -C-D- and the connection direction of L is not specified, then -A-L-B includes both -A-C-D-B and -A-D-C-B.

When one of the variables is selected as a chemical bond, it indicates that the two groups it connects are directly connected. For example, in A-L-Z, when L represents a chemical bond, the structure is actually A-Z.

In the present disclosure, the term "alkyl" refers to a saturated, straight or branched, monovalent hydrocarbon group. C₁₋₆ alkyl refers to an alkyl group having 1 to 6 carbon atoms, specifically methyl, ethyl, *n*-propyl, isopropyl, *n*-butyl, isobutyl, *sec*-butyl, or *tert*-butyl.

In the present disclosure, the term "alkylene" refers to a saturated, straight or branched divalent hydrocarbon group. C₁₋₆ alkylene refers to an alkylene group having 1 to 6 carbon atoms, specifically methylene, ethylene (*e.g.,* -CH₂CH₂-, -CH(CH₃)-), propylene (*e.g., -* CH₂CH₂CH₂-, -C(CH₃)₂-, -CH₂CH(CH₃)-), or butylene (*e.g.*, -CH₂CH₂CH₂CH₂-, - CH(CH₃)CH(CH₃)-, -CH₂CH(CH₃)CH₂-).

In the present disclosure, halogen refers to F, Cl, Br, or I.

The term "alkoxy" refers to the group R^{X}-O-, wherein R^{X} is the alkyl as defined above.

The term "heterocycloalkyl" refers to a saturated monocyclic ring having a specified number of ring atoms (*e.g.,* 5- to 10-membered), a specified number of heteroatoms (*e.g.,* 1, 2, or 3), and specified types of heteroatoms (one or more of N, O, and S). The heterocycloalkyl includes, but is not limited to, azetidinyl, tetrahydropyrrolyl, tetrahydrofuranyl, morpholinyl, piperidinyl, piperazinyl, *etc.*

The "-" at the terminus of a group indicates that the group is connected to other moieties in the molecule through this site.

The " 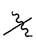 " in the structural moiety indicates that the structural moiety is connected to other moieties in the molecule through this site. For example, 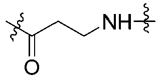 indicates that the carbonyl group and amino group are connected to other moieties in the molecule via " 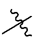 respectively.

The term "more than one" refers to 2, 3, 4, or 5.

The term "solvate of a pharmaceutically acceptable salt" refers to a substance formed by the combination of a compound with a pharmaceutically acceptable (relatively non-toxic, safe, and suitable for patient use) acid or base and a solvent (including but not limited to: water, methanol, ethanol, *etc*.)*,* wherein the pharmaceutically acceptable salt has the same meaning as the term "pharmaceutically acceptable salt" above, and the solvent is stoichiometric or non-stoichiometric. The solvate of the pharmaceutically acceptable salt includes, but is not limited to, hydrochloride monohydrate.

The term "therapeutically effective amount" refers to the amount of a compound administered to a patient that is sufficient to effectively treat the disease. The therapeutically effective amount will vary depending on the compound, the type of disease, the severity of the disease, the age of the patient, *etc.,* but can be adjusted by those skilled in the art as appropriate.

The term "pharmaceutical excipient" refers to the excipients and additives used in the production of medicaments and the preparation of prescriptions, encompassing all substances included in the pharmaceutical formulation other than the active ingredient.

The term "treatment" refers to any of the following: (1) alleviating one or more biological manifestations of a disease; (2) interfering with one or more points in the biological cascade that triggers the disease; (3) slowing the progression of one or more biological manifestations of the disease.

The term "prevention" refers to reducing the risk of disease occurrence.

The term "patient" refers to any animal that is or will be undergoing treatment, preferably a mammal, and most preferably a human. Mammals include, but are not limited to, cows, horses, sheep, pigs, cats, dogs, mice, rats, rabbits, guinea pigs, monkeys, humans, *etc.*

On the basis of common knowledge in the art, each of the above preferred conditions can be arbitrarily combined to obtain each preferred example of the present disclosure.

The reagents and raw materials used in the present disclosure are all commercially available.

The positive and progressive effects of the present disclosure are as follows: The present disclosure provides a class of nitrogen-containing heterocyclic compounds with high affinity for FAP, which can be used for the diagnosis and treatment of FAP-associated tumors (such as FAP-positive solid tumors). These compounds exhibit high tumor uptake and long retention, demonstrating broad application prospects.

### BRIEF DESCRIPTION OF THE DRAWINGS

FIG. 1 shows the lipid-water partition coefficients (CLogP) of compounds ¹⁷⁷Lu-JHH11 and ¹⁷⁷Lu-JHDB4.
FIG. 2 shows the results of the cell competitive binding experiment with compounds JHH8, JHDB8, JHH9, and the reference compound.
FIG. 3 shows the results of the cell competitive binding experiment with compounds JHH5, JHH4, JHH10, JHH8, H4b, H5b, H8b, and the reference compound.
FIG. 4 shows the tissue distribution results in tumor-bearing mice 24 hours after injection of compounds ¹⁷⁷Lu-JHH3 , ¹⁷⁷Lu-JHH8, ¹⁷⁷Lu-JHH9, ¹⁷⁷Lu-JHDB7, ¹⁷⁷Lu-JHDB8, and the reference compound.
FIG. 5 shows the imaging of compound ⁶⁸Ga-JHH7 in tumor-bearing mice.
FIG. 6 shows the imaging of compound ⁶⁸Ga-JHH11 in tumor-bearing mice.
FIG. 7 shows the tumor growth inhibition by the ¹⁷⁷Lu-labeled compounds in a tumor-bearing mouse model.
FIG. 8 shows the effect of the ¹⁷⁷Lu-labeled compounds on body weight in a tumor-bearing mouse model.

### DETAILED DESCRIPTION OF THE PREFERRED EMBODIMENT

The present disclosure will be described in detail below by way of examples, but the scope of the present disclosure is not limited thereto. Experimental methods that do not indicate specific conditions in the following examples should be selected according to conventional methods and conditions, or according to product specifications.

In the following examples, HATU refers to 2-(7-azabenzotriazol-1-yl)-*N,N,N',N'-*tetramethyluronium hexafluorophosphate, CAS 148893-10-1; DIPEA refers to *N,N-*diisopropylethylamine, CAS: 7087-68-5; HOSu refers to *N*-hydroxysuccinimide, CAS: 6066-82-6; DOTA-NHS refers to 1,4,7,10-tetraazacyclododecane-1,4,7,10-tetraacetic acid, 1-(2,5-dioxo-1-pyrrolidinyl) ester, CAS: 170908-81-3.

The LCMS detection method in the examples of the present disclosure is the following Analytical Method 1 or Analytical Method 2,

### Analytical Method 1:

Chromatographic column: SUNFIRE C18 (4.6×50 mm, 3.5 µm)
Mobile phase: H₂O (0.01% TFA) (A)/acetonitrile (0.01% TFA) (B)
Elution program: A gradient from 5% to 95% of B at 2.0 mL/min over 1.3 minutes
Column temperature: 50°C
Detection: UV (214, 4 nm) and MS (ESI, Positive mode, 110 to 1000 amu).

### Analytical Method 2:

Chromatographic column: Shim-pack VP-ODS, (4.6× 150 mm, 5 µm)
Mobile phase: H₂O (0.01% TFA) (A)/acetonitrile (0.01% TFA) (B)
Elution program: A gradient from 10% to 40% of B at 1.0 mL/min over 20 minutes, followed by a gradient from 40% to 70% of B from 20 to 24 minutes, then from 70% to 10% of B from 24 to 24.1 minutes, and maintaining 10% of B from 24.1 to 30 minutes.
Column temperature: 30°C
Detection: UV (214, 4 nm) and MS (ESI, Positive mode, 110 to 1000 amu).

### Synthesis of DOTA-GA-NH₂:

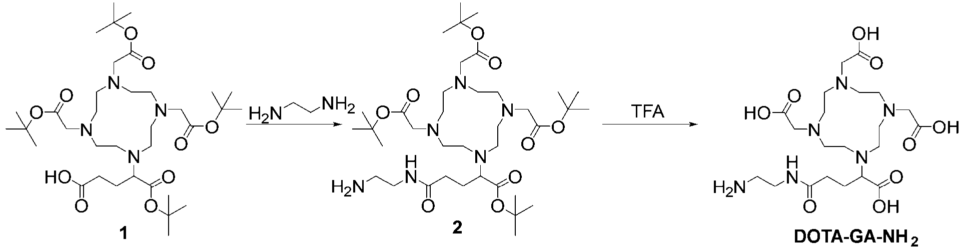

To a 100 mL round-bottom flask with a stir bar, DOTA-GA(tBu)₄ 1 (1.5 g, 2.1 mmol), HBTU (1.03 g, 2.73 mmol), and anhydrous MeCN (30 mL) were added, and finally pyridine (10 mL) was added. The reaction mixture was stirred at room temperature for 30 minutes, then drawn into a syringe and delivered via a syringe pump over 1 hour at a rate of 0.5 mL/min into a 100 mL round-bottom flask containing ethylenediamine (10 mL) and anhydrous MeCN (20 mL), and stirred at room temperature. The reaction mixture was poured into water (50 mL) and extracted with ethyl acetate (2×100 mL). The combined organic phases were washed with brine, dried over Na₂SO₄, filtered, and concentrated to obtain crude tri-tert-butyl 2,2',2"-(10-(5-((2-aminoethyl)amino)-1-(tert-butoxy)-1,5-dioxopentan-2-yl)-1,4,7,10-tetraazacyclododecane-1,4,7-triyl)triacetate **2** as a yellow oil (1.6 g). LCMS: m/z 743.5 [M+H]⁺, t_{R} = 1.014 min (Analytical Method 1)

The reaction mixture of tri-*tert*-butyl 2,2',2"-(10-(5-((2-aminoethyl)amino)-1-(*tert-*butoxy)-1,5-dioxopentan-2-yl)-1,4,7,10-tetraazacyclododecane-1,4,7-triyl)triacetate **2** (500 mg, 0.67 mmol) in TFA (10 mL) was stirred at room temperature overnight and concentrated. The residue was purified by reverse-phase HPLC (silica gel, 40 g, Biotage, 0 to 5% MeCN in water) to obtain 2,2',2"-(10-(4-((2-aminoethyl)amino)-1-carboxy-4-oxobutyl)-1,4,7,10-tetraazacyclododecane-1,4,7-triyl) DOTA-GA-NH₂ (300 mg, yield: 86.4%). LCMS: m/z 519.3 [M+H]⁺, t_{R} = 0.303 min (Analytical Method 1)

### Example 1 Synthesis of Compounds JHMe1-JHMe10

### Synthesis of Compound JHMe1 (General Synthetic Route 1) and Analysis Using Analytical Method 1:

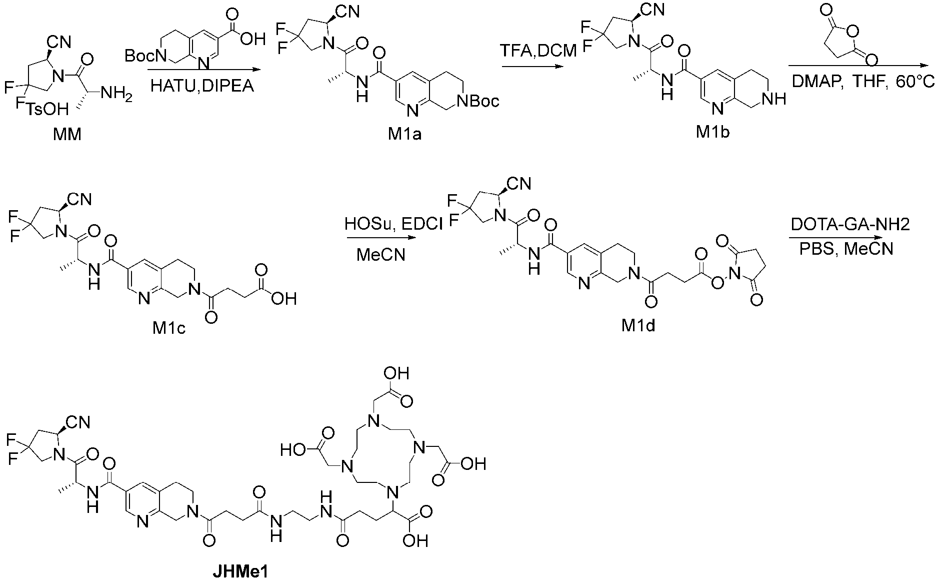

Synthesis of M1a: A solution of 7-(tert-butoxycarbonyl)-5,6,7,8-tetrahydro-1,7-naphthyridine-3-carboxylic acid (278 mg, 1.0 mmol), (S)-1-(D-alanyl)-4,4-difluoropyrrolidine-2-carbonitrile 4-methylbenzenesulfonate (412 mg, 1.1 mmol), HATU (494 mg, 1.3 mmol), DIPEA (387 mg, 3.0 mmol), DMF (4 mL), and DCM (16 mL) was stirred at room temperature for 1 hour. After concentration, the mixture was poured into water (100 mL) and extracted with EtOAc (30 mL×3). The organic layer was washed with water (30 mL×2) and brine (30 mL×1), dried over Na₂SO₄, concentrated under reduced pressure, and purified by flash silica gel chromatography with a gradient of MeOH/DCM (0% to 3%, 25 g, Biotage) to obtain 3-((*R*)-1-((*S*)-2-cyano-4,4-difluoropyrrolidin-1-yl)-1-oxopropan-2-yl)carbamoyl)-5,8-dihydro-1,7-naphthyridine-7(6*H*)-carboxylate (330 mg, 71.3%) as a pale yellow solid. LCMS: m/z 464.3 [M+H]⁺; t_{R} = 1.01 min.

Synthesis of M1b: To a solution of *tert*-butyl 3-(((*R*)-1-((*S*)-2-cyano-4,4-difluoropyrrolidin-1-yl)-1-oxopropan-2-yl)carbamoyl)-5,8-dihydro-1,7-naphthyridine-7(6*H*)-carboxylate (330 mg, 0.71 mmol) in DCM (5 mL), TFA (3 mL) was added. The mixture was stirred at room temperature for 1 hour and then concentrated to obtain crude *N*-((*R*)-1-((*S*)-2-cyano-4,4-difluoropyrrolidin-1-yl)-1-oxopropan-2-yl)-5,6,7,8-tetrahydro-1,7-naphthyridine-3-carboxamide as a yellow oil. LCMS: m/z 364.2 [M+H]⁺; t_{R} = 0.15 min.

Synthesis of M1c: A solution of*N*-((*R*)-1-((*S*)-2-cyano-4,4-difluoropyrrolidin-1-yl)-1-oxopropan-2-yl)-5,6,7,8-tetrahydro-1,7-naphthyridine-3-carboxamide (crude, 0.71 mmol), succinic anhydride (214 mg, 2.14 mmol), DMAP (43 mg, 0.36 mmol), and THF (10 mL) was stirred at 60°C for 6 hours. The mixture was concentrated under reduced pressure and purified by flash silica gel chromatography with a gradient of MeOH/DCM (0% to 5%, 25 g, Biotage) to obtain 4-(3-(((*R*)-1-((*S*)-2-cyano-4,4-difluoropyrrolidin-1-yl)-1-oxopropan-2-yl)carbamoyl)-5,8-dihydro-1,7-naphthyridin-7(6*H*)-yl)-4-oxobutanoic acid (190 mg, 57.6%) as a yellow solid. LCMS: m/z 464.3 [M+H]⁺; t_{R} = 0.86 min.

Synthesis of M1d: A solution of 4-(3-(((*R*)-1-((*S*)-2-cyano-4,4-difluoropyrrolidin-1-yl)-1-oxopropan-2-yl)carbamoyl)-5,8-dihydro-1,7-naphthyridin-7(6*H*)-yl)-4-oxobutanoic acid (190 mg, 0.41 mmol), HOSu (283 mg, 2.46 mmol), EDCI (236 mg, 1.23 mmol), and MeCN (5 mL) was stirred at room temperature for 6 hours. After concentration, the mixture was purified by flash silica gel chromatography with a gradient of MeOH/DCM (0 to 2%, 25 g, Biotage) to obtain 2,5-dioxopyrrolidin-1-yl 4-(3-(((*R*)-1-((*S*)-2-cyano-4,4-difluoropyrrolidin-1-yl)-1-oxopropan-2-yl)carbamoyl)-5,8-dihydro-1,7-naphthyridin-7(6*H*)-yl)-4-oxobutanoate (60 mg, 26.1%) as a white solid. LCMS: m/z 561.2 [M+H]⁺; t_{R} = 0.88 min.

Synthesis of JHM1: A PBS solution (1 mL) containing DOTA-GA-NH₂ (56 mg, 0.11 mmol) was neutralized with NaHCO₃, followed by dropwise addition of a MeCN solution (2 mL) of 2,5-dioxopyrrolidin-1-yl 4-(3-(((*R*)-1-((*S*)-2-cyano-4,4-difluoropyrrolidin-1-yl)-1-oxopropan-2-yl)carbamoyl)-5,8-dihydro-1,7-naphthyridin-7(6*H*)-yl)-4-oxobutanoate (60 mg, 0.11 mmol). The resulting reaction mixture was stirred at room temperature for 1 hour. The mixture was purified by Prep-HPLC to obtain 2,2',2"-(10-(1-carboxy-4-((2-(4-(3-(((*R*)-1-((*S*)-2-cyano-4,4-difluoropyrrolidin-1-yl)-1-oxopropan-2-yl)carbamoyl)-5,8-dihydro-1,7-naphthyridin-7(6*H*)-yl)-4-oxobutanamido)ethyl)amino)-4-oxobutyl)-1,4,7,10-tetraazacyclododecane-1,4,7-triyl)triacetic acid (18.2 mg, 17.6%) as a white solid. LCMS: m/z 965.3 [M+H]⁺; t_{R} = 0.87 min.

The synthesis of compounds JHM2 to JHM10 was carried out with reference to General Synthetic Route 1 and Analytical Method 1.

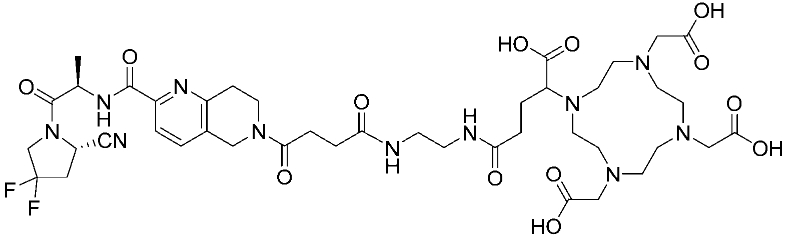

JHM2: white solid (13.6 mg, 4.4%). LCMS: *m*/*z* 482.8 [M/2+H]⁺; *t_{R} =* 0.94 min.

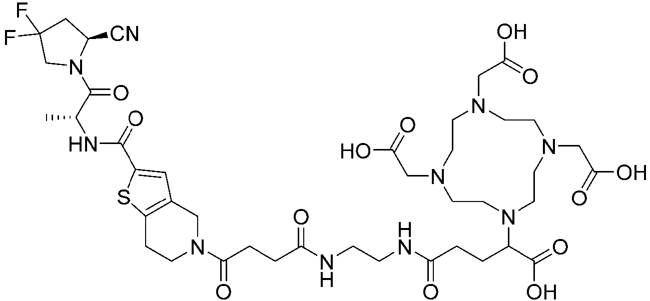

JHM3: white solid (22.2 mg, 13.0%). LCMS: *m*/*z* 485.4 [M/2+H]⁺; *t_{R} =* 0.90 min.

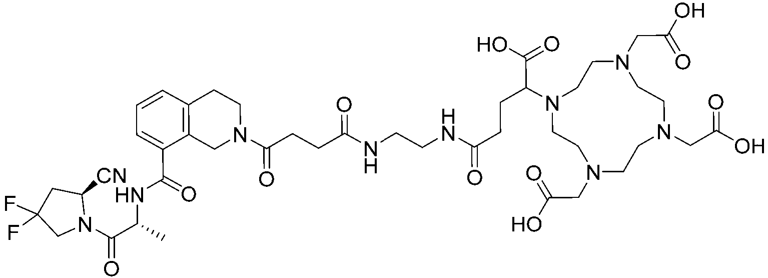

JHM4: white solid (44.4 mg, 15.9%). LCMS: *m*/*z* 482.4 [M/2+H]⁺; *t_{R} =* 0.90 min.

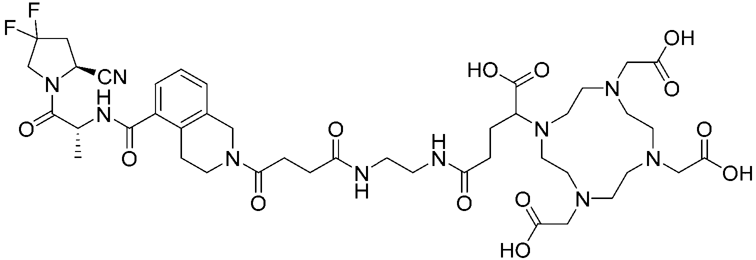

JHM5: white solid (6.0 mg, 3.5%). LCMS: *m*/*z* 482.3 [M/2+H]⁺; *t_{R} =* 0.94 min.

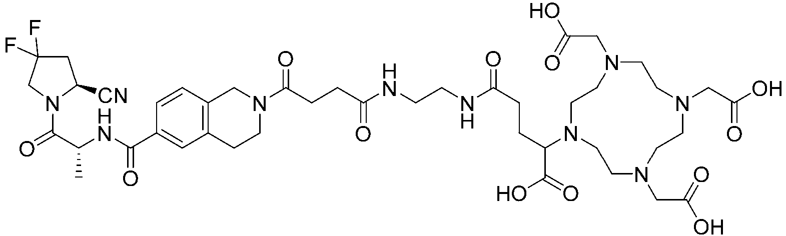

JHM6: white solid (20.4 mg, 13.2%). LCMS: *m*/*z* 482.4 [M/2+H]⁺; *t_{R} =* 0.95 min.

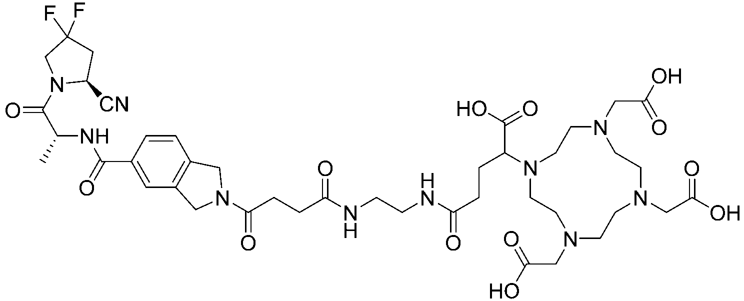

JHM7: white solid (13.6 mg, 7.2%). LCMS: *m*/*z* 950.3 [M+H]⁺; *t_{R}* = 0.93 min.

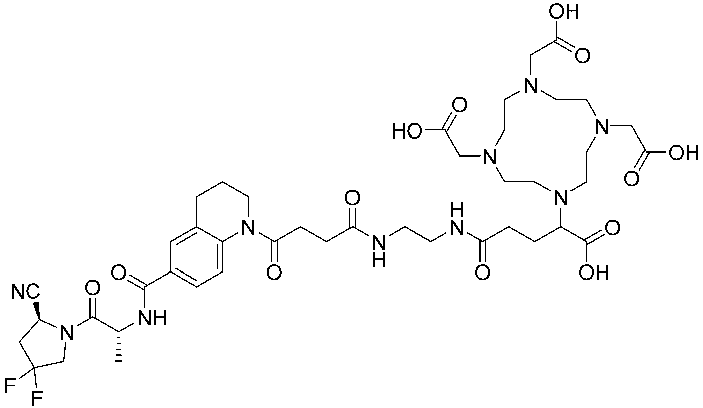

JHM8: white solid (17.5 mg, 5.0%). LCMS: *m*/*z* 482.1 [M/2+H]⁺; *t_{R} =* 1.24 min.

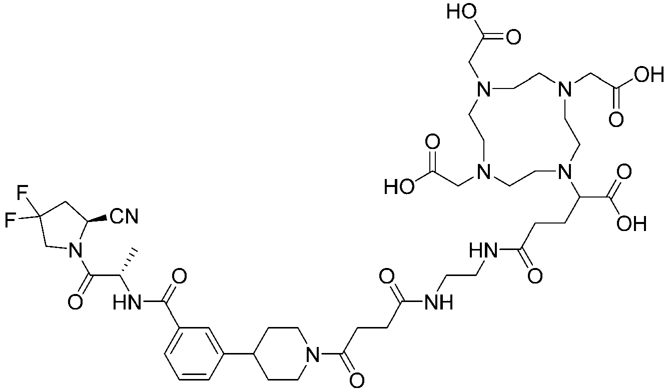

JHM9: white solid (53.8 mg, 27.7%). LCMS: *m*/*z* 991.0 [M+H]⁺; *t_{R}* = 0.84 min.

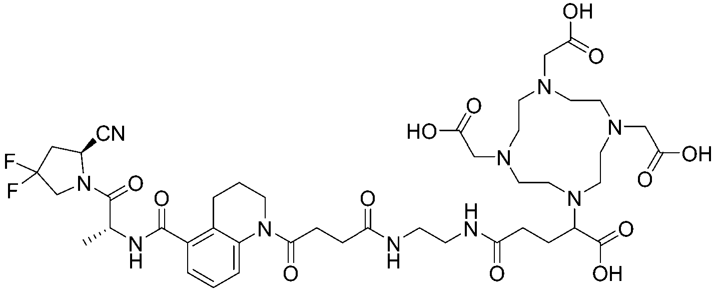

JHM10: white solid (8.6 mg, 1.9%). LCMS: *m*/*z* 963.5 [M+H]⁺; *t_{R}* = 0.89 min.

### Example 2 Synthesis of Compounds JHH1-JHH14

### Synthesis of Compound JHH1 (General Synthetic Route 2) and Analysis Using Analytical Method 1:

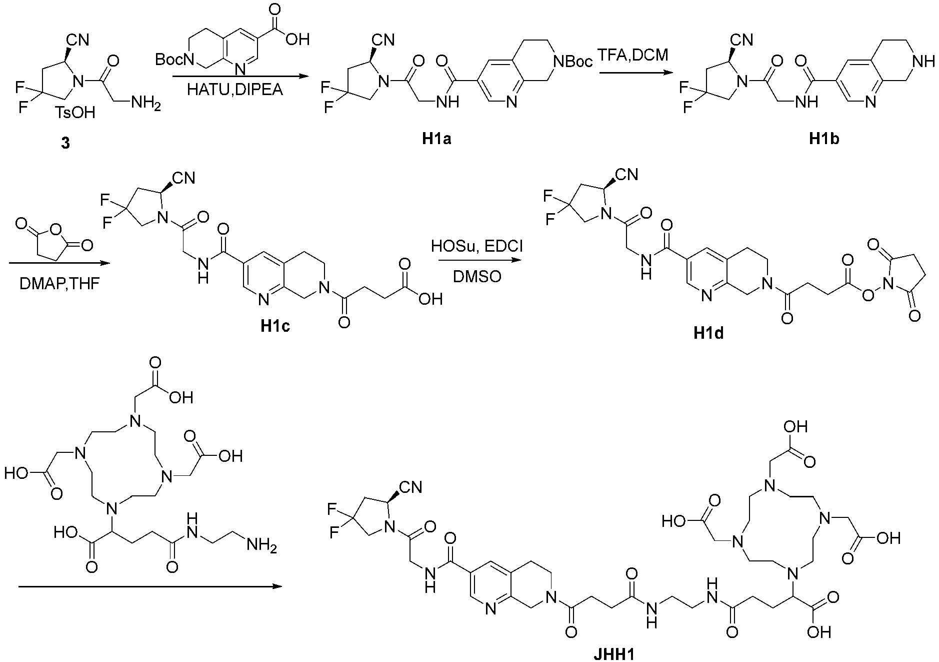

**Synthesis of H1a:** To a solution of 7-(*tert*-butoxycarbonyl)-5,6,7,8-tetrahydro-1,7-naphthyridine-3-carboxylic acid (385 mg, 1.38 mmol), (*S*)-4,4-difluoro-1-glycylpyrrolidine-2-carbonitrile benzenesulfonate (500 mg, 1.38 mmol), and HATU (632 mg, 1.66 mmol) in DCM (10 mL) and DMF (2 mL), DIEPA (537 mg, 4.15 mmol) was added at room temperature. The reaction mixture was stirred at room temperature for 2 hours. The reaction mixture was poured into water (50 mL) and extracted with ethyl acetate (2×100 mL). The combined organic phases were washed with brine, dried over Na₂SO₄, filtered, concentrated, and purified by flash chromatography (silica gel, 10 g, Biotage, 0 to 25% methanol in dichloromethane) to obtain *tert-*butyl (*S*)-3-((2-(2-cyano-4,4-difluoropyrrolidin-1-yl)-2-oxoethyl)carbamoyl)-5,8-dihydro-1,7-naphthyridine-7(6*H*)-carboxylate as a yellow oil (500 mg, yield: 80%). LCMS: m/z 450.3 [M+H]⁺, t_{R} = 1.021 min.

**Synthesis of H1b:** To a solution of *tert-*butyl (*S*)-3-((2-(2-cyano-4,4-difluoropyrrolidin-1-yl)-2-oxoethyl)carbamoyl)-5,8-dihydro-1,7-naphthyridine-7(6*H*)-carboxylate (500 mg, 1.11 mmol) in DCM (10 mL), TFA (5 mL) was added. The reaction mixture was stirred at room temperature for 1 hour. The cooled mixture was then concentrated to obtain crude (*S*)-*N*-(2-(2-cyano-4,4-difluoropyrrolidin-1-yl)-2-oxoethyl)-5,6,7,8-tetrahydro-1,7-naphthyridine-3-carboxamide as a yellow oil. LCMS: m/z 350.2 [M+H]⁺, t_{R} = 0.342 min.

**Synthesis of H1c:** A solution of (*S*)-*N*-(2-(2-cyano-4,4-difluoropyrrolidin-1-yl)-2-oxoethyl)-5,6,7,8-tetrahydro-1,7-naphthyridine-3-carboxamide (500 mg, 1.11 mmol), succinic anhydride (666 mg, 6.66 mmol), and DMAP (67.8 mg, 0.55 mmol) in THF (10 mL) was stirred at 60°C for 6 hours. The mixture was then purified by flash chromatography (silica gel, 10 g, Biotage, 0 to 25% methanol in dichloromethane) to obtain (*S*)-4-((2-(2-cyano-4,4-difluoropyrrolidin-1-yl)-2-oxoethyl)carbamoyl)-5,8-dihydro-1,7-naphthyridin-7(6*H*)-yl)-4-oxobutanoic acid as a yellow oil (300 mg, yield 60.2%). LCMS: m/z 450.2 [M+H]⁺, t_{R} = 0.803 min.

**Synthesis of H1d:** A solution of 2(*S*)-4-(2-(2-cyano-4,4-difluoropyrrolidin-1-yl)-2-oxoethyl)carbamoyl)-5,8-dihydro-1,7-naphthyridin-7(6*H*)-yl)-4-oxobutanoic acid (250 mg, 0.55 mmol), EDCI (135 mg, 0.72 mmol), and HOSu (75 mg, 0.66 mmol) in MeCN (5 mL) was stirred at room temperature overnight. The resulting solution was poured into water (50 mL) and extracted with ethyl acetate (2×50 mL). The combined organic phases were washed with brine, dried over Na₂SO₄, filtered, and concentrated to obtain crude 2,5-dioxopyrrolidin-1-yl (*S*)-4-(3-((2-(2-cyano-4,4-difluoropyrrolidin-1-yl)-2-oxoethyl)carbamoyl)-5,8-dihydro-1,7-naphthyridin-7(6*H*)-yl)-4-oxobutanoate (108 mg). LCMS: m/z 547.2 [M+H]⁺, t_{R} = 0.869 min.

**Synthesis of JHH1:** To a solution of 2,5-dioxopyrrolidin-1-yl (*S*)-4-(3-((2-(2-cyano-4,4-difluoropyrrolidin-1-yl)-2-oxoethyl)carbamoyl)-5,8-dihydro-1,7-naphthyridin-7(6*H*)-yl)-4-oxobutanoate (100 mg, 0.18 mmol) in MeCN (2 mL), 2 mL of PBS solution containing DOTA-GA-NH₂ (139 mg, 0.27 mmol) was added. The reaction mixture was stirred at room temperature for 30 minutes. The residue was purified by preparative high-performance liquid chromatography to obtain 2,2',2"-(10-(1-carboxy-4-((2-(4-(3-((2-((S)-2-cyano-4,4-difluoropyrrolidin-1-yl)-2-oxoethyl)carbamoyl)-5,8-dihydro-1,7-naphthyridin-7(6*H*)-yl)-4-oxobutanamido)ethyl)amino)-4-oxobutyl)-1,4,7,10-tetraazacyclododecane-1,4,7-triyl)triacetic acid (25 mg, yield: 14.6%). LCMS: m/z 950.3 [M+H]⁺, t_{R} = 0.739 min.

The synthesis of compounds JHH2 to JHH10 was carried out with reference to General Synthetic Route 2 and Analytical Method 1, wherein H2b, H3b, H4b, H5b, H6b, H7b, H8b, and H9b were analyzed using Analytical Method 2.

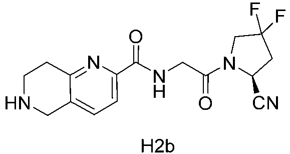

H2b: white solid (20.1 mg, 34.1%). LCMS: *m*/*z* 350.4 [M+H]⁺, *t*_{R} = 8.501 min.

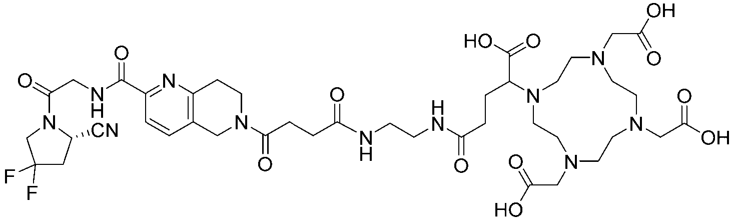

JHH2: white solid (42.3 mg, 18.7%). LCMS: *m*/*z* 951.3 [M+H]⁺, *t*_{R} = 0.899 min.

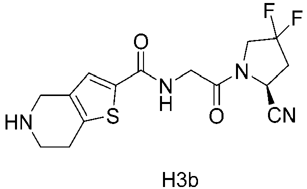

H3b: white solid (20.4 mg, 32.7%). LCMS: *m*/*z* 354.6 [M+H]⁺, *t*_{R} = 9.270 min.

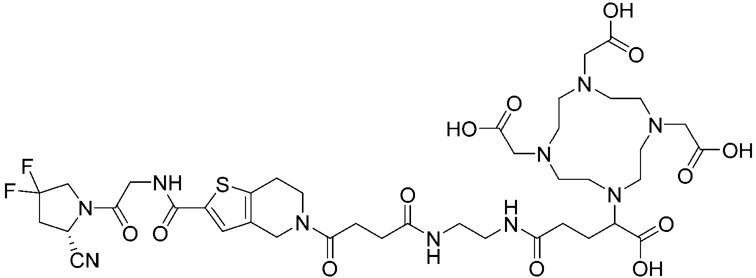

JHH3: white solid (29 mg, 6.9%). LCMS: *m*/*z* 478.4 [M+H]⁺, *t*_{R} = 0.846 min.

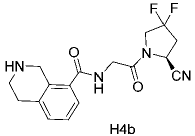

H4b: white solid (22.6 mg, 36.8%). LCMS: *m*/*z* 348.7 [M+H]⁺, *t*_{R} *=* 10.231 min.

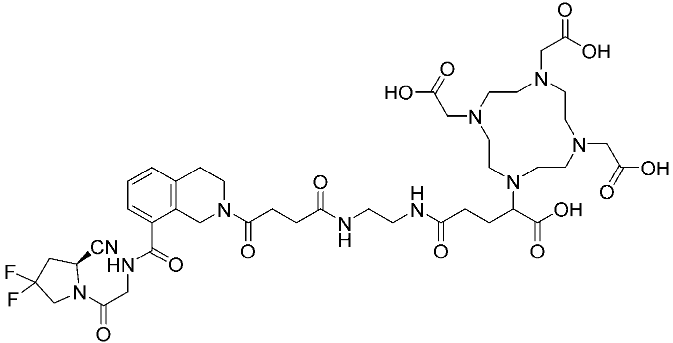

JHH4: white solid (44.4 mg, 18.7%). LCMS: *m*/*z* 475.3 [M+H]⁺, *t*_{R} = 0.841 min.

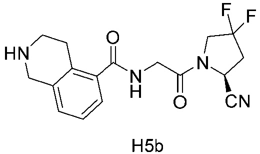

H5b: white solid (18.62 mg, 18.02%). LCMS: *m*/*z* 348.91 [M+H]⁺, *t*_{R} = 11.54 min.

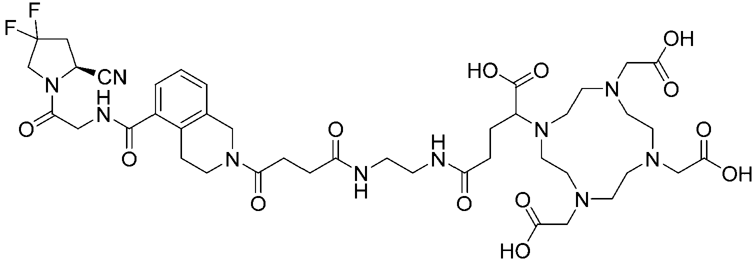

JHH5: white solid (3 mg, 0.87%). LCMS: *m*/*z* 475.4 [M+H]⁺, *t*_{R} = 0.896 min.

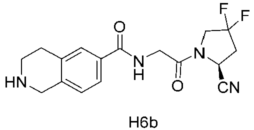

H6b: white solid (26.2 mg, 59.7%). LCMS: *m*/*z* 349.4 [M+H]⁺, *t*_{R} = 9.184 min.

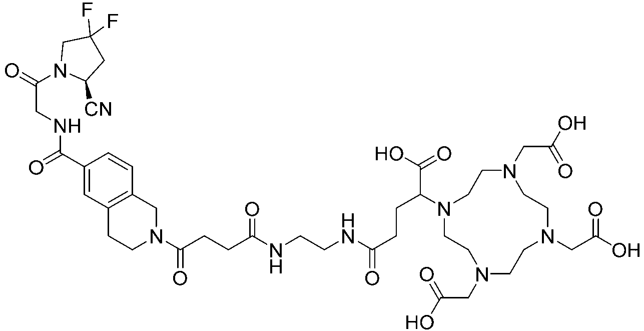

JHH6: white solid (2 mg, 4.25%). LCMS: *m*/*z* 949.3 [M+H]⁺, *t*_{R} =0.912 min.

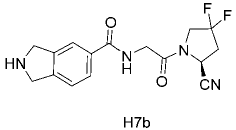

H7b: white solid (11.36 mg, 12.07%). LCMS: *m*/*z* 335.38 [M+H]⁺, *t*_{R} = 10.70 min.

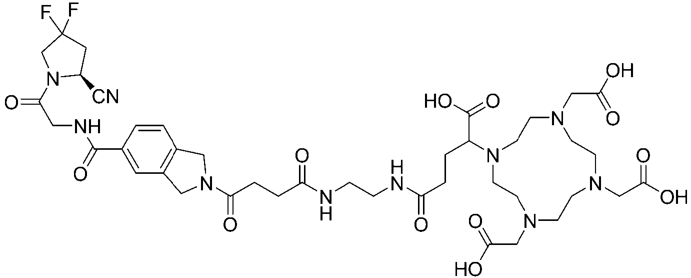

JHH7: white solid (59 mg, 17.1%). LCMS: *m*/*z* 935.3 [M+H]⁺, *t*_{R} = 0.888 min.

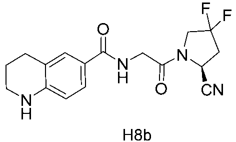

H8b: white solid (12.09 mg, 30.04%). LCMS: *m*/*z* 371.28 [M+Na]⁺, *t*_{R} = 12.125 min.

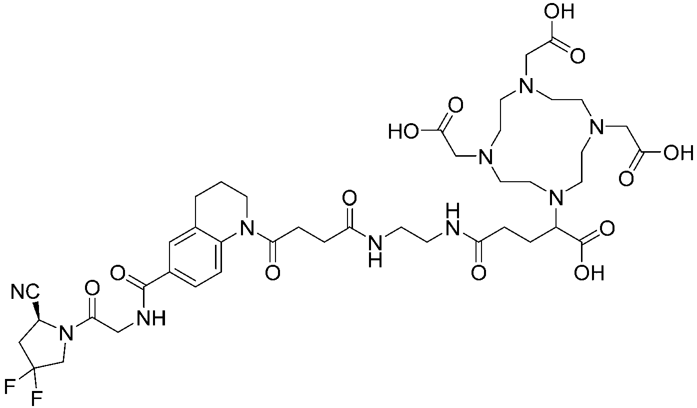

JHH8: white solid (10.4 mg, 2.34%). LCMS: *m*/*z* 950.3 [M+H]⁺, *t*_{R} = 0.923 min.

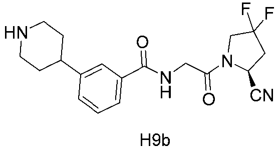

H9b: white solid (8.13 mg, 20.5%). LCMS: *m*/*z* 376.76 [M+H]⁺, *t*_{R} = 11.87 min.

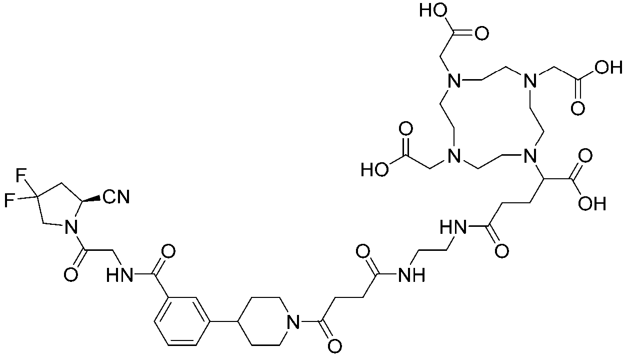

JHH9: white solid (17.9 mg, 10.8%). LCMS: *m*/*z* = 489.3 [M+H]⁺, *t*_{R} *=* 0.957 min.

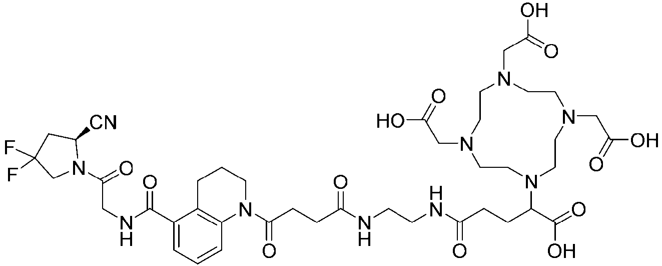

JHH10: white solid (23.3 mg, 5.45%). LCMS: *m*/*z* = 950.3 [M+H]⁺, *t*_{R} = 0.768 min.

Using DOTA-NHS instead of DOTAGA, the following compounds JHH11-JHH14 were synthesized following General Synthesis Route 2 and Analytical Method 1:

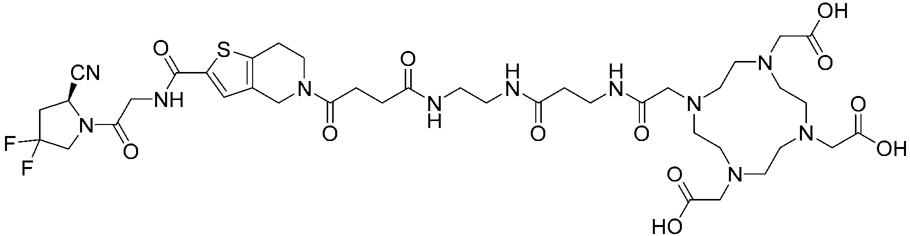

JHH11: white solid (21.7 mg, 14.2%). LCMS: *m*/*z* 954.0 [M+H]⁺; *t*_{R} = 0.78 min.

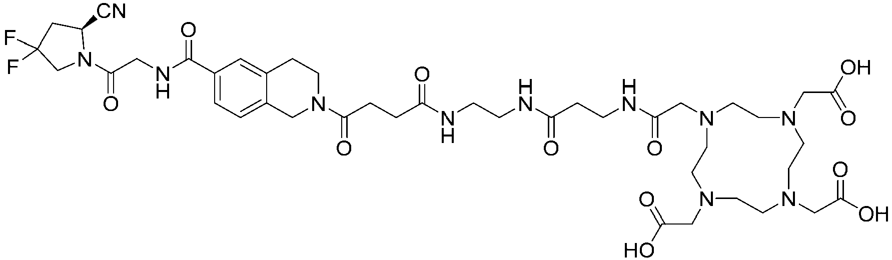

JHH12: white solid (48.9 mg, 28.1%). LCMS: *m*/*z* 948.3 [M+H]⁺; *t_{R}* = 0.92 min.

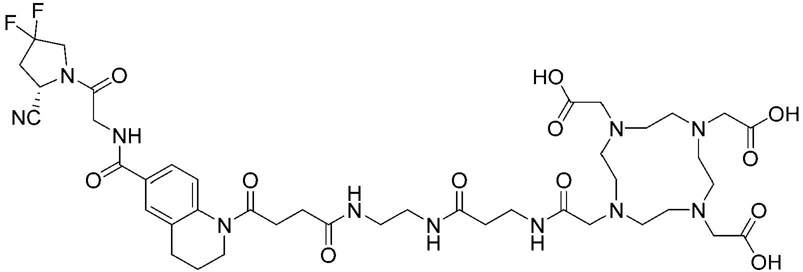

JHH13: white solid (44.6 mg, 28.1%). LCMS: *m*/*z* 948.1 [M+H]⁺; *t_{R}* = 0.79 min.

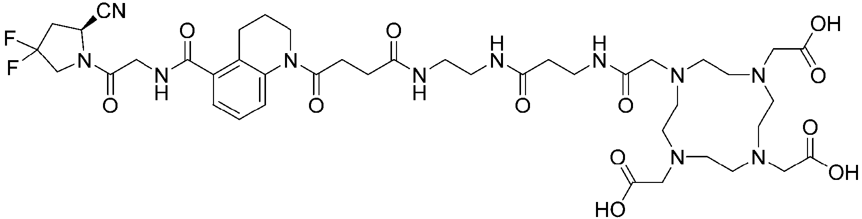

JHH14: white solid (65.5 mg, 26.9%). LCMS: *m*/*z* 948.0 [M+H]⁺; *t_{R}* = 0.77 min.

### Example 3 Synthesis of Compounds JHB1-JHB5

### Synthesis of Compound JHB1

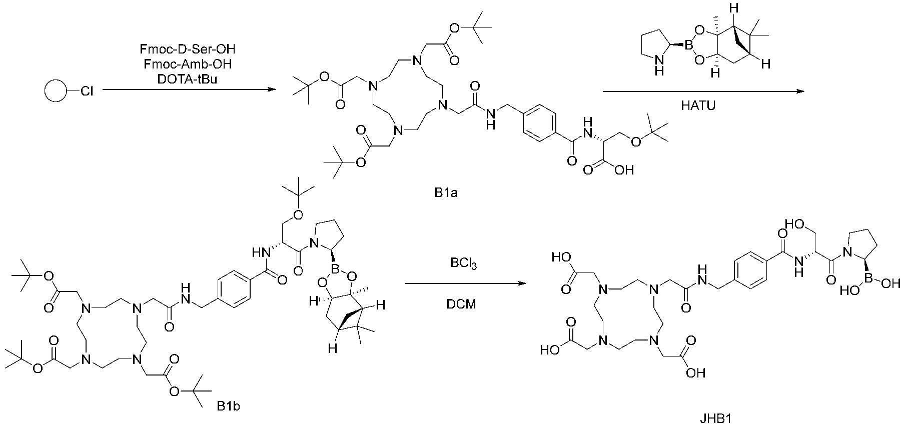

Synthesis of B1a: 1 g of CTC resin (Xi'an Sunresin New Materials Co., Ltd.) (0.65 mmol) was added to a peptide synthesis tube, followed by the addition of DCM (20 mL) for swelling for 20 minutes, and the solvent was then removed under reduced pressure. Fmoc-D-Ser(tBu)-OH (249 mg, 1.95 mmol) was dissolved in DCM (15 mL) and added to the peptide tube, followed by the addition of DIEA (0.3 mL), and the reaction was carried out for 2 hours. MeOH (1.5 mL) was added, and the reaction was carried out for 15 minutes. The solvent was removed under reduced pressure, and the resin was washed with DMF (20 mL) for 5 times. 20% PIP/DMF (20 mL) was added, and the reaction was carried out for 10 minutes. This step was repeated once, and then the resin was washed with DMF (20 mL) for 6 times. Fmoc-Amb-OH (720 mg, 1.95 mmol) and DOTA-tBu (1.1 g, 1.98 mmol) were sequentially coupled to the resin using PyBOP and TBTU as condensation reagents, respectively. The resin was cleaved 5 times with 1% TFA/DCM (20 mL), and the cleavage solution was rotary evaporated to dryness. The crude product was purified to obtain 270 mg of the product (B1a).

Synthesis of B1b: B1a (90 mg, 0.104 mmol), borate ester (29 mg, 0.115 mmol), and HATU (43.7 mg, 0.115 mmol) were dissolved in DMF (0.5 mL), followed by the addition of DIEA (39.6 mg, 0.3 mmol). The reaction was stirred for 2 hours. The reaction was monitored by LCMS until completion, and the reaction mixture was directly purified to obtain the product (B1b, 60 mg).

Synthesis of JHB1: B1b (60 mg, 0.056 mmol) was dissolved in DCM (2 mL) at -78°C, and BCl₃ (2 mL) was added dropwise. The reaction was stirred for 1 hour. The reaction was monitored by LCMS until completion, and the product (JHB1) was obtained by preparative purification (ACN: H₂O = 0 to 30%, 40 min). HPLC: 93.73%, *t_{R}* = 10.717 min (Analytical Method 2), MS: [M+H-H₂O]⁺ = 704.79.

The synthesis of compounds JHB2 to JHB5 was carried out with reference to the synthetic route of JHB1.

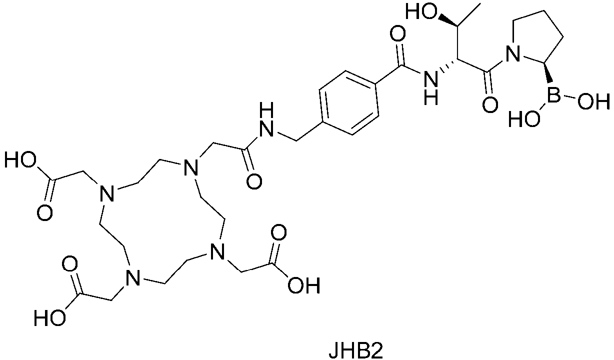

JHB2: HPLC: 91.94%, *t_{R}* = 8.864 min (Analytical Method 2), MS: [M+H-2H₂O]⁺ = 700.73.

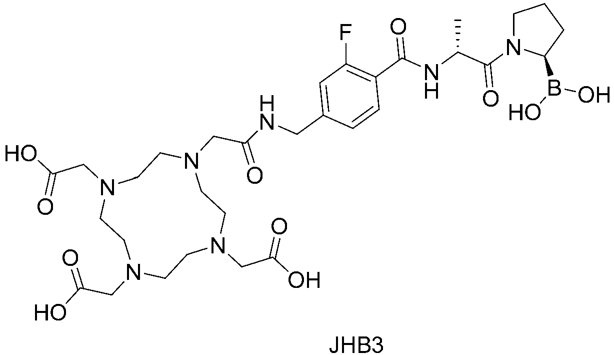

JHB3: HPLC: 97.84%, *t_{R}* = 13.828 min (Analytical Method 2), MS: [M+H-H₂O]⁺ = 706.47.

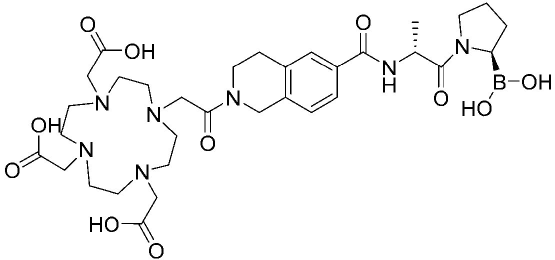

JHB4: HPLC: 96.1%, LCMS: *m*/*z* 715.3 [M+H-H₂O]⁺; *t_{R}* = 0.77 min (Analytical Method 1)

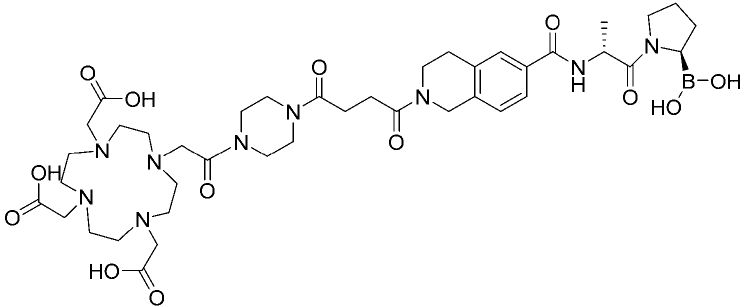

JHB5: HPLC: 100%, LCMS: *m*/*z* 883.4 [M+H-H₂O]⁺; *t_{R}* = 0.88 min (Analytical Method 1)

### Example 4 Synthesis of Compound JHDB1 (General Synthetic Route 4) and Analysis Using Analytical Method 1:

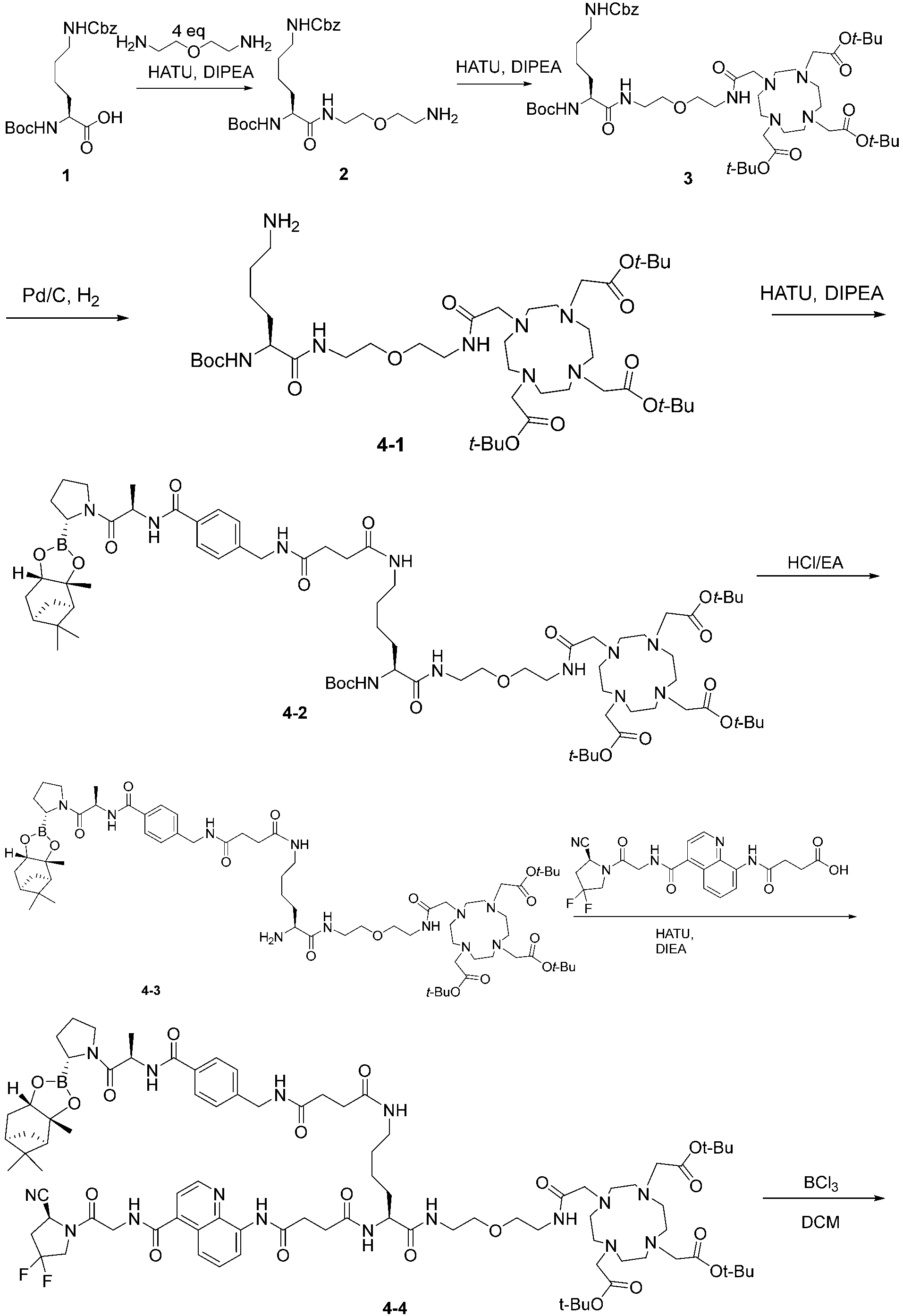

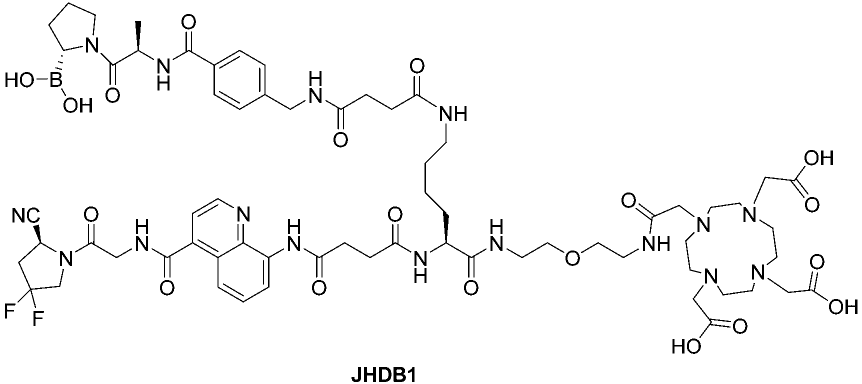

### 6-(2-(2-Aminoethoxy)ethyl)amino-6-oxohexane-1,5-diyl)(S)-dicarbamate (2):

To a 100 mL round-bottom flask with a stir bar, *N*⁶-((benzyloxy)carbonyl)-*N*²-(*tert-*butoxycarbonyl)-*L*-lysine (2 g, 5.25 mmol), HATU (2.6 g, 6.82 mmol), and anhydrous MeCN (30 mL) were added, and finally pyridine (10 mL) was added. The reaction mixture was stirred at room temperature for 30 minutes, then drawn into a syringe and delivered via a syringe pump over 1 hour at a rate of 0.5 mL/min into a 100 mL round-bottom flask containing 2,2'-oxybis(ethyl-1-amine) (3.28 g, 31.5 mmol) and anhydrous MeCN (20 mL), and stirred at room temperature. The reaction mixture was poured into water (50 mL) and extracted with ethyl acetate (2×100 mL). The combined organic phases were washed with brine, dried over Na₂SO₄, filtered, and concentrated to obtain crude benzyl *tert-*butyl (6-((2-(2-aminoethoxy)ethyl)amino)-6-oxohexane-1,5-diyl)(*S*)-dicarbamate as a yellow oil (1.7 g). LCMS: m/z 467.3 [M+H]⁺, t_{R} = 1.06 min.

**2,2,2-(10-(9-((*tert*-Butoxycarbonyl)amino)-3,10,18-trioxa-1-phenyl-2,14-dioxa-4,11,17-triazanonadecan-19-yl)-1,4,7,10-tetraazacyclododecane-1,4,7-triyl(*S*)-triacetate (3):** To a solution of 2-(4,7,10-tris(2-(*tert*-butoxy)-2-oxoethyl)-1,4,7,10-tetraazacyclododecan-1-yl)acetic acid (1.6 g, 2.9 mmol), 6-((2-(2-aminoethoxy)ethyl)amino)-6-oxohexane-1,5-diyl (*S*)-dicarbamate (1.7 g, 3.6 mmol), and HATU (1.65 g, 4.32 mmol) in DMF (20 mL), DIEA (1.4 g, 10.8 mmol) was added. The reaction mixture was stirred at room temperature for 2 hours. The reaction mixture was poured into water (50 mL) and extracted with ethyl acetate (2×100 mL). The combined organic phases were washed with brine, dried over Na₂SO₄, filtered, concentrated, and purified by flash chromatography (silica gel 10 g, Biotage, 0 to 25% methanol in dichloromethane) to obtain 2,2',2"-(10-(9-((*tert*-butoxycarbonyl)amino)-3,10,18-trioxa-1-phenyl-2,14-dioxa-4,11,17-triazanonadecan-19-yl)-1,4,7,10-tetraazacyclododecane-1,4,7-triyl)(*S*)-triacetate as a yellow oil. LCMS: m/z 511.5 [1/2M+H]⁺, t_{R} = 1.17 min.

**2,2,2-(10-(6-(4-Aminobutyl)-2,2-dimethyl-4,7,15-trioxa-3,11-dioxa-5,8,14-triazahexadecan-16-yl)-1,4,7,10-tetraazacyclododecane-1,4,7-triyl(*S*)-triacetate (4-1):** To a solution of 2,2,2,2-(10-(9-((*tert*-butoxycarbonyl)amino)-3,10,18-trioxa-1-phenyl-2,14-dioxa-4,11,17-triazanonadecan-19-yl)-1,4,7,10-tetraazacyclododecane-1,4,7-triyl)(*S*)-triacetate (800 mg, 0.78 mmol) in MeOH (20 mL), Pd/C (400 mg) was added. The mixture was stirred under hydrogen at room temperature for 2 hours. The mixture was filtered through diatomite, and the filtrate was concentrated to obtain 2,2',2"-(10-(6-(4-aminobutyl)-2,2-dimethyl-4,7,15-trioxa-3,11-dioxa-5,8,14-triazahexadecan-16-yl)-1,4,7,10-tetraazacyclododecane-1,4,7-triyl)(S)-triacetate as a yellow oil (600 mg, yield: 86.4%). LCMS: m/z 444.5 [1/2M+H]⁺, t_{R} = 1.04 min.

**2,2,2-(10-((12*S*)-12-(*tert*-Butoxycarbonyl)amino)-3,6,13,21-tetraoxa-1-(4-((2*R*)-1-oxo-1-((2*R*)-2-((3a*S*,4*S*,6*S*)-3a,5,5-trimethylhexahydro-4,6-methanobenzo[*d*][1,3,2]dioxaborol-2-yl)pyrrolidin-1-yl)propan-2-yl)carbamoyl)phenyl)-17-oxa-2,7,14,20-tetraazadocosan-22-yl)-1,4,7,10-tetraazacyclododecane-1,4,7-triyl)triacetate (4-2):** To a solution of compound (4-1) (300 mg, 0.33 mmol), 4-oxo-4-((2*R*)-1-oxo-1-((2*R*)-2-((3a*S*,4*S*,6S)-3a,5,5-trimethylhexahydro-4,6-methanobenzo[*d*][1,3,2]dioxaborol-2-yl)pyrrolidin-1-yl)propan-2-yl)amino)butanoic acid (187 mg, 0.33 mmol), and HATU (155 mg, 0.40 mmol) in DMF (3 mL), DIEA (109 mg, 0.84 mmol) was added at room temperature. The reaction mixture was stirred at room temperature for 2 hours. The reaction mixture was poured into water (50 mL) and extracted with ethyl acetate (2×100 mL). The combined organic phases were washed with brine, dried over Na₂SO₄, filtered, concentrated, and purified by flash chromatography (silica gel 10 g, Biotage, 0 to 25% MeOH/DCM) to obtain compound (4-2) (80 mg, yield: 16.6%). LCMS: m/z 712.0 [1/2M+H]⁺, t_{R} = 1.21 min.

**2,2,2-(10-((12S)-12-Amino-3,6,13,21-tetraoxa-1-(4-((2*R*)-1-oxo-1-((2*R*)-2-((2*R*)-2-((3a*S*,4*S*,6*S*)-3a,5,5-trimethylhexahydro-4,6-methanobenzo[*d*][1,3,2]dioxaborol-2-yl)pyrrolidin-1-yl)prop-2-yl)carbamoyl)phenyl)-17-oxa-2,7,14,20-tetraazadocosan-22-yl)-1,4,7,10-tetraazacyclododecane-1,4,7-triyl)triacetate (4-3):** To a solution of compound (4-2) (80 mg, 0.056 mmol) in DCM (2 mL), 1 M HCl/EA (2 mL) was added. The reaction mixture was stirred at 0°C for 30 minutes. The mixture was then dried under a stream of nitrogen to obtain crude compound (4-3) as a yellow oil. LCMS: m/z 662.0 [1/2M+H]⁺, t_{R} = 1.14 min.

**2,2,2-(10-(12*S*)-12-(4-(4-(2-(*S*)-2-Cyano-4,4-difluoropyrrolidin-1-yl)-2-oxoethyl)amino)amino)-4-oxobutanamido)-3,6,13,21-tetraoxa-1-(4-((2*R*)-1-oxo-1-((2*R*)-2-((3a*S*,4*S*,6*S*)-3a,5,5-trimethylhexahydro-4,6-methanobenzo[*d*][1,3,2]dioxaborol-2-yl)pyrrolidin-1-yl)propan-2-yl)carbamoyl)phenyl)-17-oxa-2,7,14,20-tetraazadocosan-22-yl)-1,4,7,10-tetraazacyclododecane-1,4,7-triyl)triacetate (4-4):** To a solution of compound (4-3) (46 mg, 0.03 mmol), 2,5-dioxopyrrolidin-1-yl (*S*)-4-((4-((2-(2-cyano-4,4-difluoropyrrolidin-1-yl)-2-oxoethyl)carbamoyl)quinolin-8-yl)amino)-4-oxobutanoate (16 mg, 0.03 mmol), and HATU (16 mg, 0.04 mmol) in DMF (2 mL), DIEA (13.4 mg, 0.09 mmol) was added at room temperature. The reaction mixture was stirred at room temperature for 2 hours. The reaction mixture was poured into water (50 mL) and extracted with ethyl acetate (2×100 mL). The combined organic phases were washed with brine, dried over Na₂SO₄, filtered, concentrated, and purified by flash chromatography (silica gel 10 g, Biotage, 0 to 25% methanol in dichloromethane) to obtain compound (4-4) as a yellow oil. LCMS: m/z 882.6 [1/2M+H]⁺, t_{R} = 1.21 min.

**2,2,2-(10-(*R*)-1-((*R*)-1-(*R*)-2-Boronopyrrolidin-1-yl)-1-oxopropan-2-yl)carbamoyl)phenyl)-12-(4-((2-((*S*)-2-cyano-4,4-difluoropyrrolidin-1-yl)-2-oxoethylcarbamoyl)quinolin-8-yl)amino)-4-oxobutanamido)-3,6,13,21-tetraoxa-17-oxa-2,7,14,20-tetraazadocosan-22-yl)-1,4,7,10-tetraazacyclododecane-1,4,7-triyl)(JHDB1):** To a solution of compound **(4-4)** (20 mg, 0.01 mmol) in DCM (3 mL), BCl₃ (1 mL) was added at -78°C. The reaction mixture was stirred overnight at room temperature. The resulting solution was added to water (50 mL), and the pH was adjusted to >7 with sodium bicarbonate. The mixture was purified by preparative high-performance liquid chromatography to obtain JHDB1 (2.0 mg, yield: 12.5%). LCMS: m/z 722.4 [1/2M+H]⁺, t_{R} = 0.78 min.

### Example 5 Synthesis of Compound JHDB2 (General Synthetic Route 5) and Analysis Using Analytical Method 1:

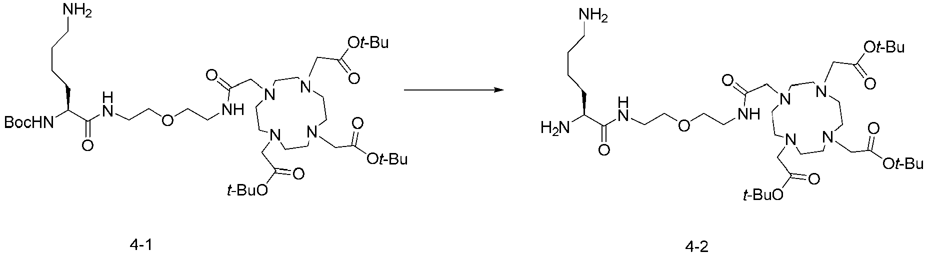

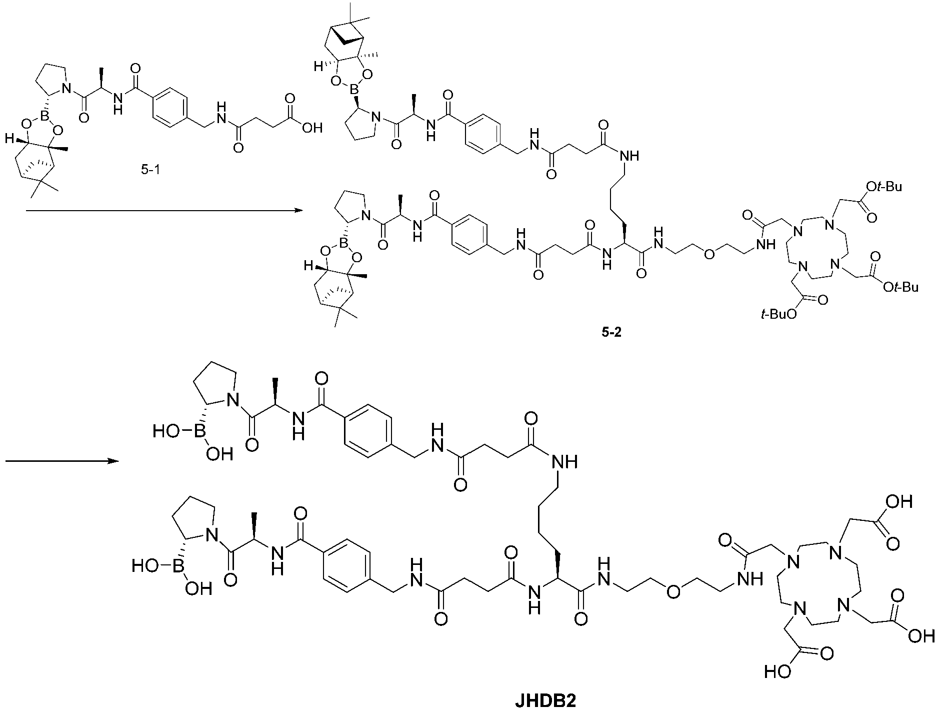

**2,2,2,10-(12*S*)-3,6,13,21-Tetraoxa-1-(4-(2*R*)-1-oxo-1-(2*R*)-2-(3a*S*,)4*S*,6*S*)-3a,5,5-trimethylhexahydro-4,6-methanobenzo[*d*][1,3,2]dioxaborol-2-yl)pyrrolidin-1-yl)propan-2-yl)carbamoyl)phenyl)-12-(4-oxo-4-(2*R*)-1-((2*R*)-2-((2*R*)-2-((3a*S*,4*S*,6*S*)-3a,5,5-trimethylhexahydro-4,6-methanobenzo[*d*][1,3,2]dioxaborol-2-yl)pyrrolidin-1-yl)propan-2-yl)amino)amino)butanamido)-17-oxa-2,7,14,20-tetraazadocosan-22-yl)-1,4,7,10-tetraazacyclododecane-1,4,7-triyl)triacetate (5-2):** To a solution of 2,2',2"-(10-(2-(2-(2-(2,6-diaminohexanamido)ethoxy)ethoxy)ethyl)amino)-2-oxoethyl)-1,4,7,10-tetraazacyclododecane-1,4,7-triyl)(*S*)-triacetate (125 mg, 0.159 mmol), 4-((4-((2*R*)-1-oxo-1-((2*R*)-2-((3a*S*,4*S*,6*S*)-3a,5,5-trimethylhexahydro-4,6-methanobenzo[*d*][1,3,2]dioxaborol-2-yl)pyrrolidin-1-yl)propan-2-yl)amino)amino)benzyl)butanoic acid (175 mg, 0.318 mmol), and HATU (90 mg, 0.238 mmol) in DMF (2 mL), DIEA (61.6 mg, 0.477 mmol) was added at room temperature. The reaction mixture was stirred at room temperature for 2 hours. The reaction mixture was poured into water (50 mL) and extracted with ethyl acetate (2×100 mL). The combined organic phases were washed with brine, dried over Na₂SO₄, filtered, concentrated, and purified by flash chromatography (silica gel 10 g, Biotage, 0 to 25% methanol in dichloromethane) to obtain compound (5-2) as a yellow oil. LCMS: m/z 929.6 [1/2M+H]⁺, t_{R} = 1.28 min.

**2,2,2-(10-((*S*)-12-(4-(4-((*R*)-1-(*R*)-2-Boronopyrrolidin-1-yl)-1-oxopropan-2-yl)carbamoyl)benzyl)amino)-4-oxobutanamido)-1-(4-(*R*)-1-(*R*)-2-boronopyrrolidin-1-yl)-1-oxopropan-2-yl)carbamoyl)phenyl)-3,6,13,21-tetraoxa-17-oxa-2,7,14,20-tetraazadocosan-22-yl)-1,4,7,10-tetraazacyclododecane-1,4,7-triyl) (JHDB2):** To a solution of compound (5-2) (70 mg, 0.037 mmol) in DCM (3 mL), BCl₃ (2 mL) was added at -78°C. The reaction mixture was stirred overnight at room temperature. The resulting solution was added to water (50 mL), and the pH was adjusted to >7 with sodium bicarbonate. The mixture was purified by preparative high-performance liquid chromatography to obtain JHDB2 (13.9 mg, yield: 12.5%). LCMS: m/z 462.5 [1/3M+H]⁺, t_{R} = 0.921 min.

The synthesis of compound JHDB3 was carried out following the synthetic route of JHDB2, and LCMS analysis was performed using Analytical Method 2.

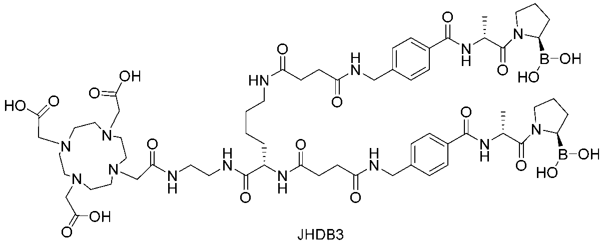

**JHDB3:** (11 mg, yield: 10.5%). LCMS: *m*/*z* 662.5 [M-3H₂O] 2H⁺, *t*_{R} = 12.59 min.

### Example 6 Synthesis of Compound JHDB4 (General Synthetic Route 6) and Analysis Using Analytical Method 1:

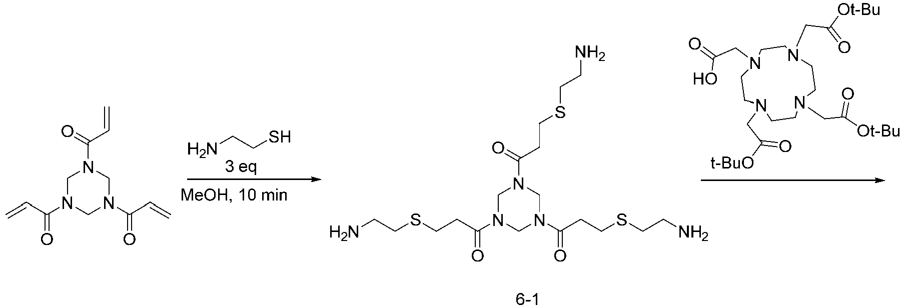

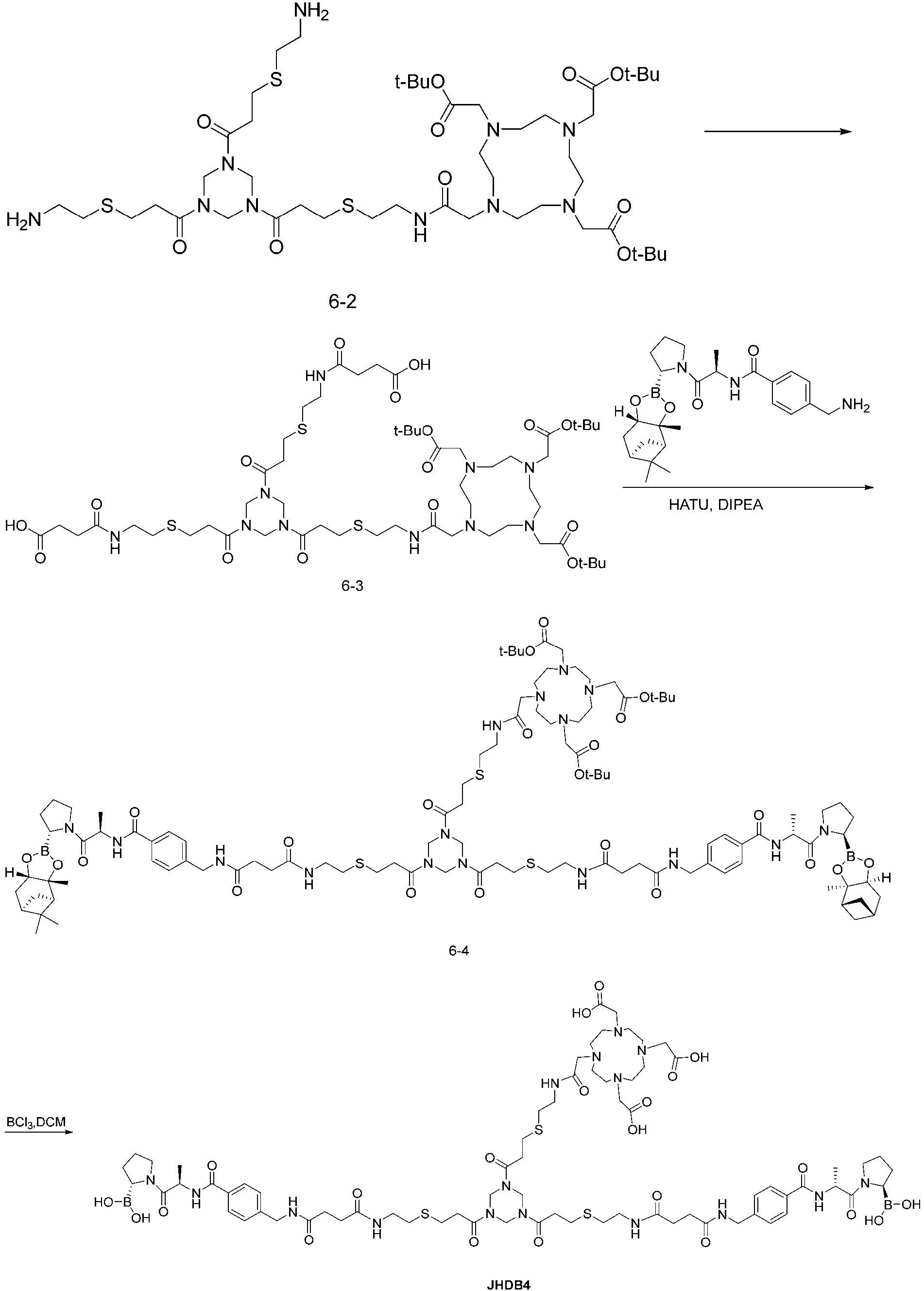

**Synthesis of 1,1,1,5-Triazinan-1,3,5-triyl)tris(3-((2-aminoethyl)thio)propan-1-one) (6-1):** To a methanol (50 mL) solution of 1,3,5-triacryloylhexahydro-1,3,5-triazine (5 g, 20 mmol), 2-mercaptoethanol (4.62 g, 60 mmol) and n-propylamine (5 drops) were added. The mixture was stirred for 30 minutes and concentrated to obtain 1,1',1"-(1,3,5-triazinane-1,3,5-triyl)tris(3-((2-aminoethyl)thio)propan-1-one) (9.8 g, quantitative) as a yellow solid. LCMS: m/z 481.4 [M+H]⁺; t_{R} = 0.21 min.

**Synthesis of 2,2',2"-(10-(2-((2-(3-(3,5-bis(3-((2-aminoethyl)thio)propanoyl)-1,3,5-triazinan-1-yl)-3-oxopropyl)thio)ethyl)amino)-2-oxoethyl)-1,4,7,10-tetraazacyclododecane-1,4,7-triyl)triacetate (6-2):** A solution of 2-(4,7,10-tris(2-(*tert-*butoxy)-2-oxoethyl)-1,4,7,10-tetraazacyclododecan-1-yl)acetic acid (573 mg, 1.0 mmol), HBTU (417 mg, 1.1 mmol), and Py (7.11 g, 90 mmol) in MeCN (20 mL) was stirred at room temperature for 0.5 hours. A solution of 1,1',1"-(1,3,5-triazinane-1,3,5-triyl)tris(3-((2-aminoethyl)thio)propan-1-one) (1.44 g, 3.0 mmol) in MeCN (10 mL) was added dropwise. The mixture was stirred at room temperature for 1 hour. After concentration, the residue was purified by Prep-HPLC to obtain compound (6-2) (250 mg, 24.2%) as a white solid. LCMS: m/z 518.4 [M/2+H]⁺; t_{R} = 0.98 min.

**Synthesis of 4,4'-(((((5-(3-((2-(2-(4,7,10-tris(2-(*tert*-butoxy)-2-oxoethyl)-1,4,7,10-tetraazacyclododecan-1-yl)acetamido)ethyl)thio)propanoyl)-1,3,5-triazinane-1,3-diyl)bis(3-oxopropane-3,1-diyl))bis(sulfanediyl))bis(ethane-2,1-diyl))bis(azanediyl))bis(4-oxobutanoic acid) (6-3):** A solution of compound (6-2) (250 mg, 0.24 mmol), succinic anhydride (48 mg, 0.48 mmol), and TEA (73 mg, 0.72 mmol) in MeCN (5 mL) was stirred for 1 hour. The mixture was concentrated under reduced pressure and purified by reverse-phase column chromatography with a gradient of MeCN/H₂O (0% to 30%, 25 g, Biotage) to obtain compound (6-3) (80 mg, 26.8%) as a white solid. LCMS: m/z 618.5 [M/2+H]⁺; t_{R} = 1.07 min.

**Synthesis of 2,2',2"-tri-*tert*-butyl-(10-(2-((2-(3-(3,5-bis(3-(2-(2-(4-oxo-4-(4-((2*R*)-1-oxo-1-((2*R*)-2-((3a*S*,4*S*,6*S*)-3a,5,5-trimethylhexahydro-4,6-methanobenzo[*d*][1,3,2]dioxaborol-2-yl)pyrrolidin-1-yl)propyl)amino)butanamido)ethyl)thio)propanoyl)-1,3,5-triazinan-1-yl)-3-oxopropyl)thio)ethyl)amino)-2-oxoethyl)-1,4,7,10-tetraazacyclododecane-1,4,7-triyl)triacetate (6-4):** Compound (6-3) (80 mg, 0.06 mmol), 4-aminomethyl-*N*-((2*R*)-1-oxo-1-((2*R*)-2-((3a*S*,4*S*,6*S*)-3a,5,5-trimethylhexahydro-4,6-methanobenzo[*d*][1,3,2]dioxaborol-2-yl)pyrrolidin-1-yl)propan-2-yl)benzamide (59 mg, 0.13 mmol), HATU (62 mg, 0.16 mmol), and DIEA (50 mg, 0.39 mmol) were stirred in DMF (2 mL) and DCM (8 mL) for 1 hour. After concentration, the mixture was diluted with EtOAc (10 mL), washed with water (5 mL×3) and brine (5 mL×1), dried over Na₂SO₄, and concentrated. The residue was purified by preparative high-performance liquid chromatography to obtain compound (6-4) (55 mg, 40.3%) as a white solid. LCMS: m/z 702.9 [M/3+H]⁺; t_{R} = 1.28 min.

**2,2,2-(10-(2-((3-(3,5-Bis(3-(2-(4-(4-((*R*)-1-((*R*)-2-boronopyrrolidin-1-yl)-1-oxopropan-2-yl)amino)-4-oxobutanamido)ethyl)thio)propionyl)-1,3,5-triazinan-1-yl)-3-oxopropyl)thio)ethyl)amino)-2-oxoethyl)-1,4,7,10-tetraazacyclododecane-1,4,7-triyl)triacetic acid (JHDB4):** To a solution of compound (6-4) (55 mg, 0.03 mmol) in anhydrous DCM (5 mL) under a nitrogen atmosphere, BCl₃ (1.0 M in dichloromethane) (0.52 mL, 0.52 mmol) was added dropwise. The mixture was stirred at -78°C for 0.5 hours, then stirred at room temperature for 3 hours. The mixture was concentrated under reduced pressure at room temperature. The residue was diluted with water (3 mL) and neutralized. The mixture was purified by Prep-HPLC to obtain JHDB4 (10.0 mg, 22.9%) as a white solid. LCMS: m/z 545.3 [(M-34)/3+H]⁺; t_{R} = 0.75 min.

### Example 7 Synthesis of Compound JHDB5 (General Synthetic Route 7) and Analysis Using Analytical Method 2:

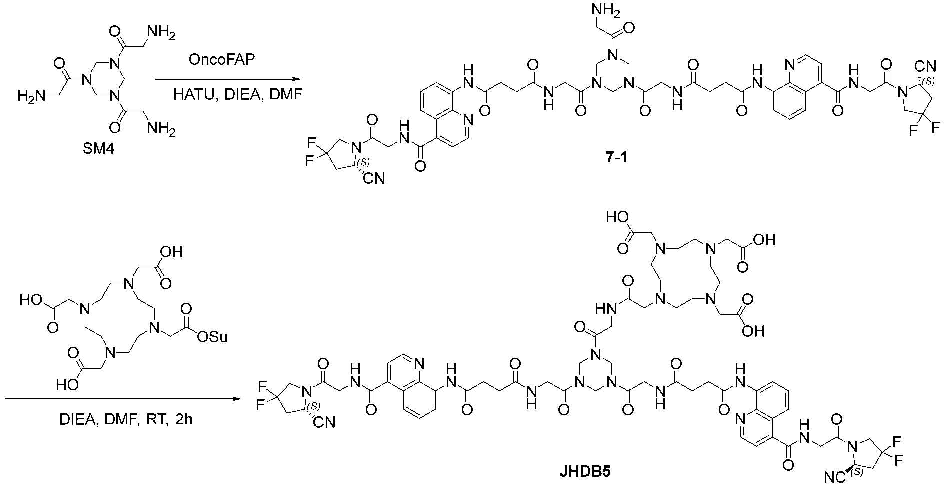

Intermediate 7-1 was prepared from SM4 and OncoFAP (for the synthesis of OncoFAP, refer to: PNAS 2021, 118, 16 e2101852118 or Eur J Nucl Med Mol Imaging, 2022, 49(6): 1822-1832) via General Synthetic Route 2, and coupled with DOTA, and then purified by preparative liquid phase to obtain compound JHDB5 (15.2 mg, 25%) as a white solid. LCMS: 1528.68 [M+H⁺]⁺, t_{R} = 10.75 min.

The compounds JHDB6 and JHDB7 were prepared by utilizing General Synthetic Route 7 (Analytical Method 2), where different intermediates were coupled with SM4 and then coupled with DOTA.

### JHDB6

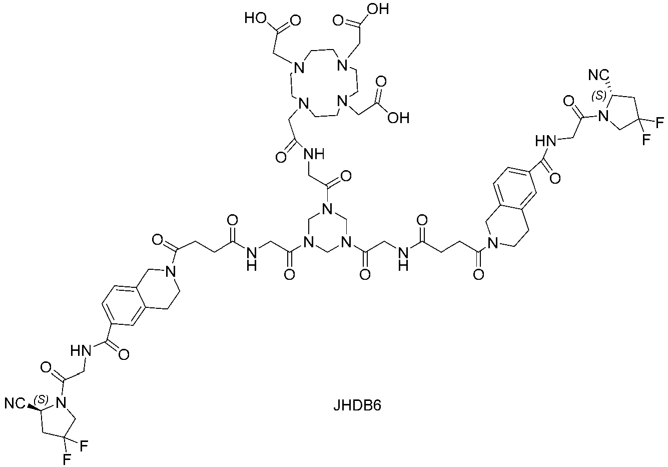

White solid (10.2 mg, 24%), LCMS: 754.06 [M/2+H⁺]⁺, t_{R} = 11.41 min.

### JHDB7

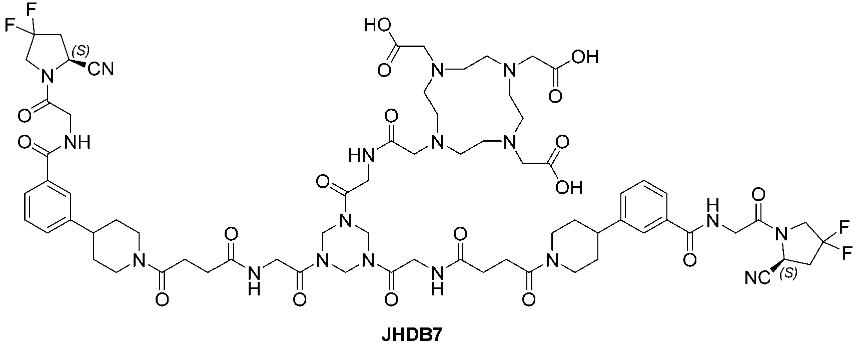

White solid (28.6 mg, 42%), LCMS: 782.12 [M/2+H⁺]⁺, t_{R} = 13.06 min.

### Example 8 Synthesis of Compound JHDB8 (General Synthetic Route 8)

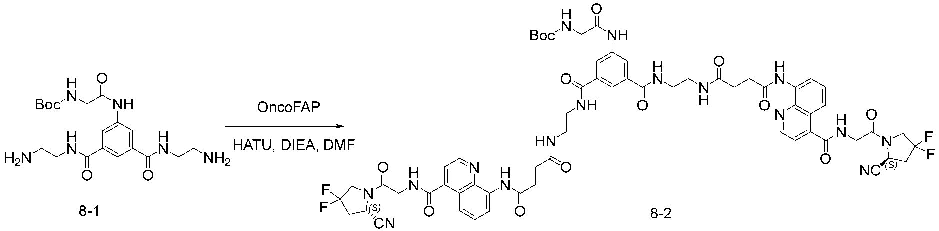

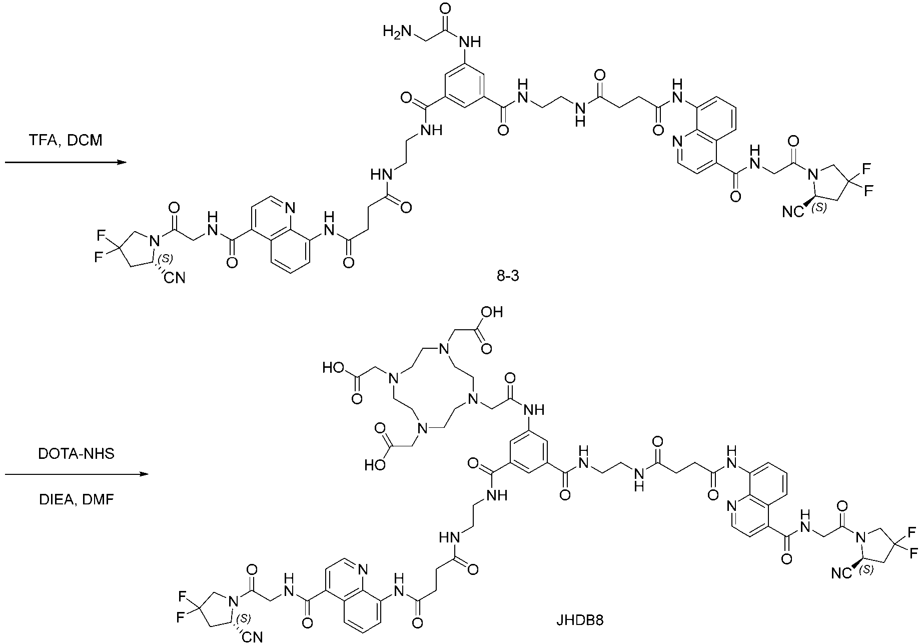

8-1 was coupled with OncoFAP to obtain compound 8-2, which was deprotected to obtain compound 8-3. Compound 8-3 was reacted with DOTA-NHS (CAS: 170908-81-3) to obtain crude JHDB8. After preparative liquid phase purification, pure JHDB8 was obtained as a white solid (435 mg, 100%). LCMS: 1592.60 [M+H⁺]⁺, t_{R} = 11.68 min. (Analytical Method 2)

### Biological Assay:

### 1. In Vitro Activity Assay

The Biacore 8K (Cytiva) instrument was used to detect ligand binding of the FAP protein (Sinobiological). The FAP protein was captured on the SA chip. Before ligand immobilization (flow paths 1 and 2, flow rate of 10 µL/min), the FAP protein (10 µg/mL, flow rate of 5 µL/min, injection time of 600 s) was immobilized on flow path 2 using running buffer, and the sensor surface was conditioned with three consecutive injections of 1 M NaCl in 50 mM NaOH. After each ligand injection, additional washing was carried out with isopropanol in 1 M NaCl and 50 mM NaOH (flow paths 1 and 2, flow rate of 10 µL/min, injection time of 60 s).

The test compounds were dissolved in 100% dimethyl sulfoxide and diluted to 10 mM, then further diluted in the assay buffer (PBS, pH 7.4, 1 mM TCEP (tris(2-hydroxyethyl)phosphine), 0.05% P20, 2% dimethyl sulfoxide) to appropriate maximum concentrations. The analyte was run under the following conditions: analysis temperature = 15°C, analysis step = all set to LMW kinetics; cycle type = single cycle (90 s contact time, 1800 s dissociation time, 30 µL/min flow rate, flow channels 1, 2); flow channel detection = 2-1). Data evaluation was performed using Biacore Insight Evaluation Software, and the data were fitted to a 1:1 binding model.

The Biacore results are shown in Table 1: pK_{D} = -LogK_{D}, where K_{D} is the binding affinity of the compound to the FAP protein as measured by Biacore. The binding affinity is expressed by K_{D} (M) = K_{d}(1/s)/Kₐ(1/Ms). Where A: pK_{D}>8, B: 7<pK_{D}<8, C: 6<pK_{D}<7, D: pK_{D}<6.

**Table 1. Biacore Test Results**

| Compound | pK_{D} |
|---|---|
| JHM1 | C |
| JHH1 | B |
| JHH2 | B |
| JHH3 | A |
| JHH5 | A |
| JHH6 | C |
| JHH7 | A |
| JHH8 | A |
| JHH9 | A |
| JHH10 | A |
| JHH11 | A |
| JHH12 | A |
| JHH13 | B |
| JHDB2 | C |
| JHDB6 | A |
| JHDB7 | B |
| JHB1 | B |

### 2. Selective Assay

The specificity of the synthesized compounds against recombinant human dipeptidyl peptidase IV (DPPIV), fibroblast activation protein (FAP), or prolyl oligopeptidase (PREP) was expressed by their half-maximal inhibitory concentration (IC₅₀) against the proteases. The specific steps are:
1) The synthesized compound was dissolved in DMSO to a final concentration of 100 mM.
   a) For FAP assay: The test compound was diluted to a 1 mM solution using a buffer of 50 mM Tris, 140 mM NaCl at pH 7.5.
   b) For DPPIV assay: The test compound was diluted to a 1 mM solution using a buffer of 25 mM Tris, 250 mM NaCl at pH 7.5.
   c) For PREP assay: The test compound was diluted to a 1 mM solution using a 140 mM NaCl buffer at pH 8.0.
2) The prepared 1 mM test compound solution was serially diluted (1:10) into a row of a 96-well plate using the corresponding buffer mentioned above.
3) The substrate was prepared as a DMSO stock solution (FAP and PREP: 2.5 mM Z-Gly-Pro-AMC (VWR, Cat. No. I-1145.0050BA) in DMSO; DPPIV: 100 mM Gly-Pro-AMC (VWR, Cat. No. 100042-646) in DMSO), and subsequently the substrate stock solution was diluted 20-fold using the corresponding buffer mentioned above.
4) The enzyme was diluted into the appropriate assay buffer. The final concentrations of DPPIV, FAP, and PREP were 0.1, 1.2, and 0.6 nm, respectively. To each required well in columns 2-10, 180 µL was added separately. Column 1 (A, B, C) was prepared with 200 µL of the appropriate assay buffer as a control. Column 1 (D, E, F, G, H) was prepared using 20 µL of appropriate assay buffer and 180 µL of enzyme as an inhibitor-free control.
5) Where appropriate, 20 µL of the test compounds from the dilution plate prepared in step 2 were added to columns 2-10 of the assay plate. Each sample was tested in triplicate. Each sample was incubated at room temperature for 10 minutes, with the plate being shaken for the first 2 minutes.
6) To each well, 10 µL of the 20x substrate prepared in step 3 was added and incubated at room temperature for 15 minutes, with the plate being shaken for the first 2 minutes.
7) Fluorescence was measured at λex: 380, λem: 460.

The results of the above tests indicated that the synthesized compound exhibited good selectivity for FAP. Where A: pIC50 >8, B: 7<pIC50<8, C: 6<pIC50<7, D: pIC50<6

**Table 2. Compound Selectivity**

| Compound | pIC50 (FAP) | pIC50 (Prep) | pIC50 (DPPIV) |
|---|---|---|---|
| JHH5 | B | D | D |

### 3. ClogP Determination

¹⁷⁷Lu-JHH11 and ¹⁷⁷Lu-JHDB4 were labeled with ¹⁷⁷Lu using the "7. General ¹⁷⁷Lu Labeling Experimental Method" described below. After labeling, a certain amount of each compound solution was mixed with ultrapure water, and the activity was measured after thorough mixing.

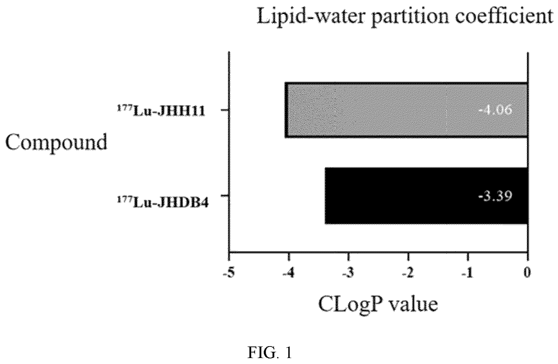

Two EP tubes were prepared, each containing 100 µL of saturated n-octanol aqueous solution and 80 µL of pure water. Then, 20 µL of the above ¹⁷⁷Lu-labeled compound solution was added to each tube. After shaking and mixing for 2 hours, the tubes were centrifuged at room temperature at 2000 rpm/min for 5 minutes. From each tube, 20 µL was taken from the upper layer (lipid layer) and the lower layer (aqueous layer), respectively, and the gamma counts were measured. The results are shown in FIG. 1.

### 4. Cell Competitive Binding Assay

(1) HT1080 8# cells in the logarithmic growth phase were prepared into a cell suspension, and the cell density was adjusted to 1×10⁴/mL. 1 mL of the suspension was seeded into a 24-well cell culture plate. The plate was incubated overnight in a 37°C incubator.
(2) The cell culture medium was aspirated, and the cells were washed once with PBS, followed by the addition of 975 µL of additive-free medium.
(3) To each well, 25 µL of ¹⁷⁷Lu-radiolabeled FAPi-46 (Nanchang Tanzhen Biotechnology Co., Ltd., CAS: 2374782-04-2) at a fixed concentration (final concentration in culture medium: 1.35 µCi/mL) and varying concentrations (final concentrations in culture medium: 1000 ng/mL, 100 ng/mL, 10 ng/mL, 1 ng/mL, 0.1 ng/mL, 0.01 ng/mL, 0.001 ng/mL, 0 ng/mL) of unlabeled test compounds JHH8, JHDB8, and JHH9 were added.
(4) The mixture was incubated on ice for 2 hours.
(5) The cells were washed three times with ice-cold PBS.
(6) The cells were lysed with 0.5 mL of 1 M sodium hydroxide, washed twice with 0.5 mL of PBS, and the sodium hydroxide solution (0.5 mL) and PBS solutions (0.5 mL×2) were collected for uptake counting.

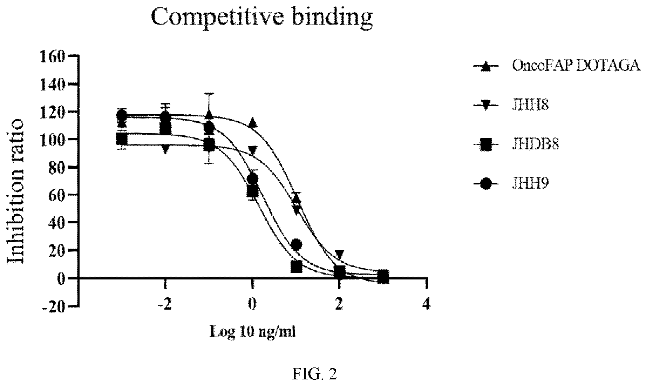

The control compound was OncoFAP-DOTAGA (structural formula: 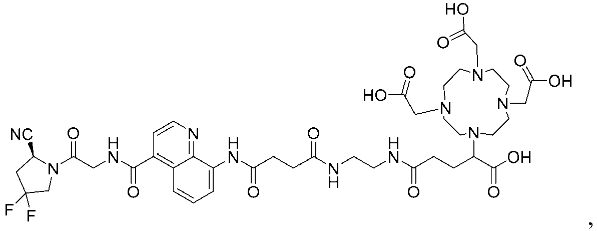 synthesized according to the references PNAS 2021, 118, 16 e2101852118 or Eur J Nucl Med Mol Imaging, 2022, 49(6): 1822-1832), and the results are shown in FIG. 2.

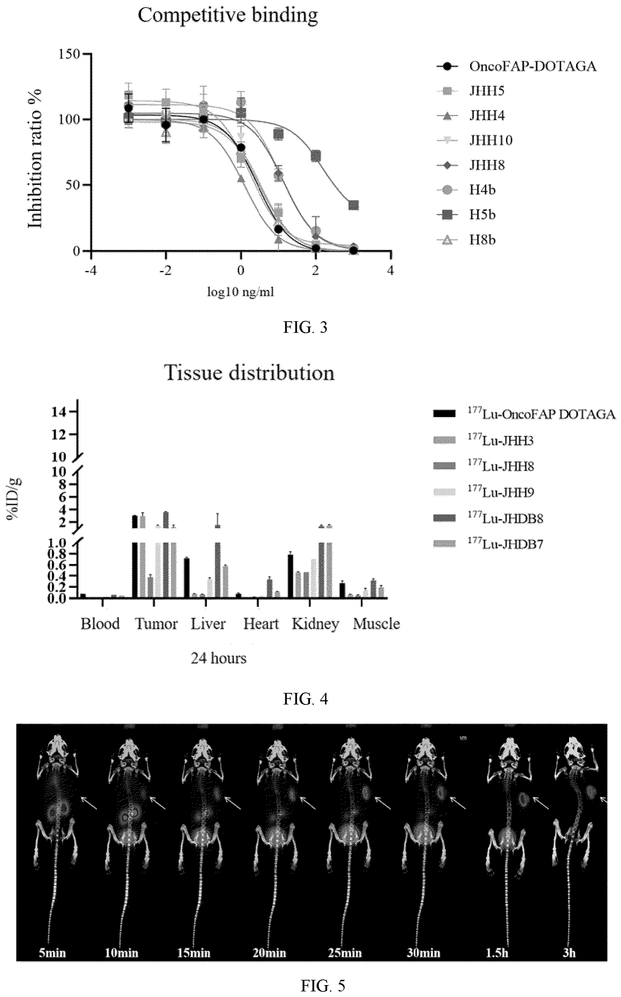

The cell competitive binding of compounds JHH5, JHH4, JHH10, JHH8, H4b, H5b, and H8b was tested in the same batch using the above method, and the experimental results are shown in Table 3 and FIG. 3.

**Table 3. Competitive Binding of Compounds**

| | OncoFAP-DOTAGA | JHH5 | JHH4 | JHH10 | JHH8 | H4b | H5b | H8b |
|---|---|---|---|---|---|---|---|---|
| EC₅₀ (ng/mL) | 2.699 | 1.726 | 1.231 | 3.38 | 13.77 | 11.89 | 157.5 | 3.219 |
| Molecular weight | 959.39 | 948.98 | 948.98 | 948.98 | 948.98 | 347.71 | 348.35 | 348.35 |
| nmol/L | 2.81 | 1.82 | 1.30 | 3.56 | 14.51 | 34.20 | 452.13 | 9.24 |

### 5. Tissue Distribution

Tissue distribution in FAP-positive tumor-bearing mice: 2×10⁶ HT1080 2# FAP cells (in 50% Matrigel, Corning) were subcutaneously inoculated into the right shoulder of mice (Balb/c Nude, Vital River) aged approximately 6-9 weeks. When the tumors grew to approximately 150-350 mm³ in size, the following "7. General ¹⁷⁷Lu Labeling Experimental Method" was used to label JHH3, JHH8, JHH9, JHDB7, and JHDB8 compounds. Subsequently, ¹⁷⁷Lu-JHH3, ¹⁷⁷Lu-JHH8, ¹⁷⁷Lu-JHH9, ¹⁷⁷Lu-JHDB7, ¹⁷⁷Lu-JHDB8, and ¹⁷⁷Lu-OncoFAP DOTAGA radiolabeled compounds were injected into the mice via the tail vein (approximately 3.7 MBq/mouse). 24 hours after administration, the animals were euthanized by CO₂ inhalation, and blood and organs (liver, kidney, muscle, tumor) were collected after euthanasia.

Blood was collected from the inferior vena cava, and immediately after collection, 100 µL was quantitatively transferred to a designated centrifuge tube (weighed). After organ collection, the samples were rinsed twice with deionized water and dried, then placed in preweighed tubes and weighed again to calculate the sample weight. The samples were measured on the day of collection. All blood samples and tissue samples were measured for radioactive counts using a gamma counter. The results are shown in FIG. 4. The results showed that after intravenous injection into animals, the labeled compound was distributed to various organs, primarily excreted via renal metabolism. Compared to the reference compound, ¹⁷⁷Lu-JHH3 exhibited higher uptake in tumor tissues while showing lower uptake in other organs.

### 6. ⁶⁸Ga Labeling and Animal Imaging Assay

A. 1.0 mg of JHH7 precursor was weighed and dissolved in 0.1 M sodium acetate buffer to prepare a 1.0 mg/mL precursor solution.
B. The Ge/Ga generator was eluted stepwise with 5 mL of 0.1 M HCl, and the highest activity fraction (0.5 mL) was collected. 0.5 mL of metal-free 0.1 M sodium acetate buffer (pH = 4.5) was added. In a 1.5 mL centrifuge tube as the reaction tube, a specific amount of precursor [1000 (molecular weight)/14.94 µL (precursor solution)/mCi (radionuclide)] was added, vortex-mixed for 10 s, and heated at 95°C, 800 rpm for 15 min.
C. The C18 Sep-Pak was activated with anhydrous ethanol, rinsed thoroughly with pure water, and the excess water was removed.
D. The solution after the reaction was passed through the C18 Sep-Pak, rinsed with pure water, and the excess water was removed. Subsequently, the C18 Sep-Pak was eluted with ethanol, collecting approximately 10 tubes with 3 drops per tube.
E. The final preparation should be a clear solution, prepared fresh for immediate use and limited to the same day.

### PCT/CT scan

1) The PET/CT was powered on according to the standard operating procedures, the scanning software was launched, and daily calibration was performed.
2) Animal anesthesia preparation was conducted, where tumor-bearing mice (supplied by Shanghai Alamo Pharmaceutical Technology Co., Ltd., HT1080 cell line with high FAP expression) were anesthetized using isoflurane.
3) After the loss of righting reflex in the tumor-bearing mice, the imaging agent was injected via the tail vein.
4) After drug administration, dynamic scanning at 1 hour and static scanning was performed at 3 hours were performed for 10 minutes.
5) During the process, animal body weight, injection dose, injection time, and residual dose were recorded according to the record sheet, and the time of measuring the injection dose and the time of measuring the residual dose were separately recorded.
6) The uptake in animal tumors, muscles, and other organs was analyzed using PMOD software.

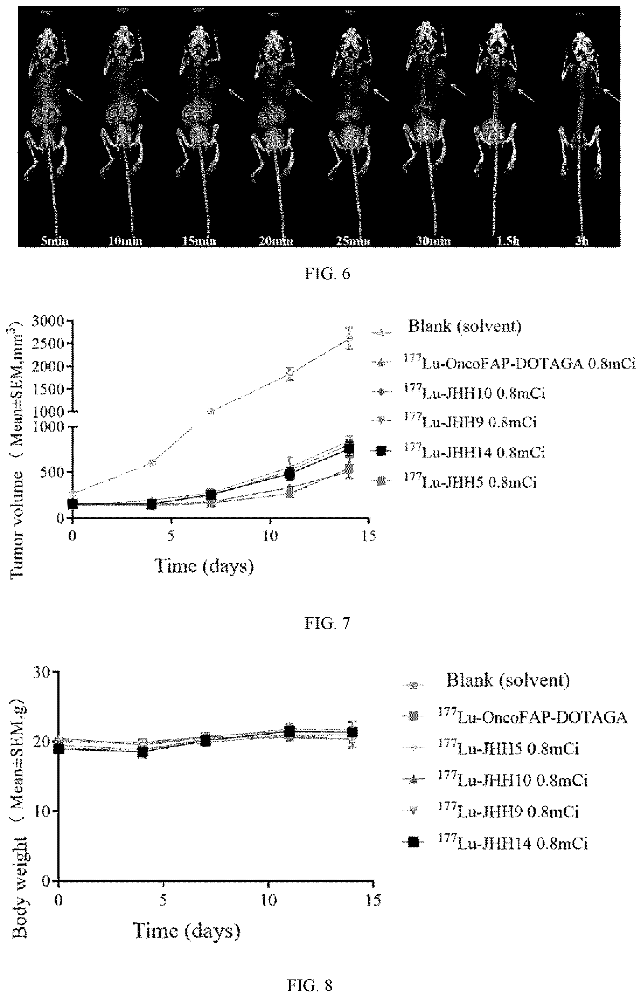

⁶⁸Ga-labeled compounds JHH7 and JHH11 were imaged in tumor-bearing mice, and the results are shown in FIG. 5 and FIG. 6, with the imaging data shown in Tables 4 and 5.

**Table 4. Imaging Data of ⁶⁸Ga-JHH7 in Tumor-Bearing Mice**

| E12 | 5 min | 10 min | 15 min | 20 min | 25 min | 30 min | 1.5 h | 3 h |
|---|---|---|---|---|---|---|---|---|
| Brain | 0.90 | 0.82 | 0.59 | 0.55 | 0.48 | 0.46 | 0.23 | 0.12 |
| Heart | 4.61 | 2.19 | 1.43 | 1.01 | 0.94 | 0.72 | 0.45 | 0.21 |
| Liver | 1.71 | 0.88 | 0.50 | 0.38 | 0.23 | 0.25 | 0.52 | 0.43 |
| Lung | 1.78 | 0.94 | 0.79 | 0.45 | 0.28 | 0.37 | 0.54 | 0.55 |
| Kidney | 6.73 | 5.59 | 3.87 | 3.35 | 2.74 | 2.88 | 0.71 | 0.55 |
| Muscle | 0.81 | 1.56 | 1.42 | 1.30 | 1.29 | 0.97 | 0.17 | 0.19 |
| Bone | 0.17 | 0.28 | 0.30 | 0.18 | 0.17 | 0.10 | 0.53 | 0.14 |
| Bladder | 3.02 | 49.75 | 84.10 | 109.69 | 145.55 | 157.22 | 89.40 | 7.50 |
| Tumor | 0.91 | 2.47 | 3.36 | 3.88 | 4.70 | 5.00 | 6.40 | 5.20 |
| Intestine | 0.47 | 0.21 | 0.23 | 0.22 | 0.15 | 0.16 | 0.92 | 0.60 |

**Table 5. Imaging Data of ⁶⁸Ga-JHH11 in Tumor-Bearing Mice**

| E15 | 5 min | 10 min | 15 min | 20 min | 25 min | 30 min | 1.5 h | 3 h |
|---|---|---|---|---|---|---|---|---|
| Brain | 0.98 | 0.66 | 0.59 | 0.55 | 0.45 | 0.41 | 0.03 | 0.04 |
| Heart | 8.61 | 4.33 | 3.22 | 2.72 | 2.29 | 1.98 | 0.20 | 0.12 |
| Liver | 5.43 | 3.56 | 2.78 | 2.44 | 2.16 | 2.05 | 0.78 | 0.82 |
| Lung | 3.12 | 2.08 | 1.97 | 1.88 | 1.47 | 1.48 | 0.58 | 0.25 |
| Kidney | 13.43 | 15.81 | 20.15 | 13.33 | 8.42 | 5.54 | 1.00 | 0.46 |
| Muscle | 0.82 | 1.22 | 1.16 | 0.98 | 0.75 | 0.65 | 0.37 | 0.08 |
| Bone | 0.92 | 1.68 | 1.17 | 1.08 | 1.22 | 0.82 | 0.50 | 0.02 |
| Bladder | 4.24 | 17.03 | 27.04 | 45.38 | 60.89 | 73.70 | 265.50 | 4.50 |
| Tumor | 1.06 | 2.47 | 3.26 | 3.87 | 4.30 | 4.60 | 4.40 | 2.30 |
| Intestine | 1.96 | 1.99 | 1.66 | 1.54 | 1.30 | 1.22 | 0.62 | 0.08 |

The above results indicated that after intravenous injection into tumor-bearing mice, the labeled compounds were rapidly distributed to various organs of the mice and then quickly excreted from the body over time, with renal metabolism being the primary metabolic pathway. The uptake of the labeled compounds in FAP-positive tumors gradually increased over time. ⁶⁸Ga-JHH7 reached its peak uptake in the tumor 1.5 hours after injection into the mice, followed by a gradual decline in uptake. At 3 hours, a certain amount of uptake in the tumor was still observed. ⁶⁸Ga-JHH11 compound reached its peak tumor uptake at 30 minutes after injection into the mice and was rapidly metabolized and excreted from the mice, and tumor uptake was still observed at 3 hours.

### 7. General ¹⁷⁷Lu Labeling Experimental Method: Using JHH5 as an Example

1) The precursor compound **JHH5** (1 mg) was weighed and dissolved in 0.5 M pH 4.0 ascorbic acid buffer to prepare a 0.1 mg/mL solution.
2) Labeling was performed at a nuclide: precursor ratio of 1:7-10 (molar ratio): 2 mCi ¹⁷⁷LuCl₃ and the calculated amount of precursor based on real-time specific activity were added to the labeling buffer system (0.5 M pH 4.0 ascorbic acid buffer), adjusting the reaction system to a volume of 0.15 mL.
3) The reaction was carried out at 95°C for 30 minutes on a constant-temperature heater. After completion of the reaction, the product was analyzed by iTLC and Radio-HPLC.
4) iTLC eluting solution: 1% EDTA, stationary phase: silica gel 254, developed to 1 cm from the top of the stationary phase, taking approximately 20 minutes, with a sample loading volume of 0.5 µL.
5) If the iTLC and Radio-HPLC results reached 95%, the labeled product was considered qualified.
6) The reaction mixture was transferred to a vial, and the reaction tube was rinsed with 0.15 mL of normal saline. The rinse solution was also added to the vial.
7) 6.7 µL of DTPA solution (2.5 mM) was added and mixed well for later use. 1 mg of DTPA was dissolved in 1 mL of normal saline to prepare a DTPA stock solution (concentration of 2.5 mM), which was mixed well for later use.

The final preparation should be a clear solution, prepared fresh for immediate use and limited to the same day.

The radiochemical purity and labeling rate were calculated based on peak area.

As shown in Table 6, the ¹⁷⁷LuCl₃ solution was developed to the top of the silica gel plate in the developing agent, and iTLC indicated that the purity of the labeled compound was >99.9%.

**Table 6**

| Region: | ¹⁷⁷Lu | Detector: | Front | | |
|---|---|---|---|---|---|
| Test sample | Start (mm) | End (mm) | Retardation factor (Rf) | Area (Counts) | %(ROI) |
| JHH5 | 0.0 | 33.0 | 0.120 | 70508 | 99.97 |
| ¹⁷⁷Lu | 79.6 | 100.0 | 0.908 | 18 | 0.03 |
| 2 peaks | | 70526 | 100.00 | | |
| Total area: | 70593 counts | | | | |
| Average background: | 0 counts | | | | |

### 8. Therapeutic Experiment of ¹⁷⁷Lu-labeled Compounds

Balb/c Nude tumor-bearing mice (supplier: Shanghai Alamo Pharmaceutical Technology Co., Ltd., HT1080 cell line with high FAP expression) aged approximately 6-8 weeks were randomly assigned to 13 experimental groups based on tumor volume, with 5 mice per group. Animal body weight and tumor size were measured, and administration was initiated on the grouping day starting with Group 1 (control group: normal saline). General health and appearance were observed daily after the start of the experiment. Animal body weight and tumor size were measured before each sample collection time point. Any abnormal observations detected during the entire study period were required to be recorded in the raw data.

The synthesized compounds were labeled with ¹⁷⁷Lu and subsequently utilized in the therapeutic assay on mouse tumor models. The results, as shown in FIG. 7 and Table 7, demonstrated that ¹⁷⁷Lu-labeled JHH1, JHH5, JHH9, JHH14 exhibited dose-dependent therapeutic effects in this model. These ¹⁷⁷Lu-labeled compounds displayed a good inhibitory effect on tumor growth. Furthermore, no significant changes in mouse body weight (FIG. 8) were observed throughout the experimental period.

**Table 7**

| **Test sample** | **Tumor volume (mm³)** | | | | **P** | **P** |
|---|---|---|---|---|---|---|
| | **Before administration** | **Group administration** | **TGI^{TV} (%)** | **T/C** | **Vs blank** | **Vs ¹⁷⁷Lu-OncoFAP - DOTAGA 0.8mCi** |
| | | **Day 14** | | | | |
| Blank (solvent) | 261.57±10.65 | 2,612.56±193.05 | - | 100.00 % | - | <0.0001 |
| ¹⁷⁷Lu-OncoFAP-DOTAGA 0.8mCi | 137.00±12.07 | 844.66±41.70 | 69.90% | 32.33% | <0.0001 | |
| ¹⁷⁷Lu-JHH10 0.8mCi | 141.47±12.20 | 506.26±26.61 | 84.48% | 19.38% | <0.0001 | 0.0005 |
| ¹⁷⁷Lu-JHH5 0.8mCi | 148.04±9.78 | 545.74±94.21 | 83.08% | 20.89% | <0.0001 | 0.002 |
| ¹⁷⁷Lu-JHH9 0.8mCi | 155.24±14.81 | 800.87±42.08 | 72.54% | 30.65% | <0.0001 | 0.6372 |
| ¹⁷⁷Lu-JHH14 0.8mCi | 153.87±16.47 | 755.85+58.74 | 74.39% | 28.93% | <0.0001 | 0.3402 |

| | | | | | | |
|---|---|---|---|---|---|---|
| **TGI^{TV}:** Relative tumor inhibition rate (TGI^{TV}) : TGI^{TV} (%) = [1-(Ti-T0)/(Vi-V0)]×100%; (Ti: average tumor volume of the treatment group on day i of administration, T0: average tumor volume of the treatment group on day 0 of administration; Vi: average tumor volume of the solvent control group on day i of administration, V0: average tumor volume of the solvent control group on day 0 of administration) T/C% is one of the most commonly used indicators for tumor response to treatment: T/C% = T/C * 100%; (T represents the average tumor volume of the treatment group at a specific time point; C represents the average tumor volume of the control group at a specific time point.) | | | | | | |

The differences between the treatment groups (tumor volumes of the second, third, fourth, and fifth groups) and the control group (tumor volume of the first group) and between the tumor volumes of the first, third, fourth, and fifth groups and the tumor volume of the second group were analyzed using the two-way ANOVA Fisher's LSD test method. P-values less than 0.05 were considered statistically significant. Both statistical significance and biological significance were taken into account during the result analysis.

## Claims

1. A compound of formula I, a pharmaceutically acceptable salt thereof, a solvate thereof, or a solvate of the pharmaceutically acceptable salt thereof; 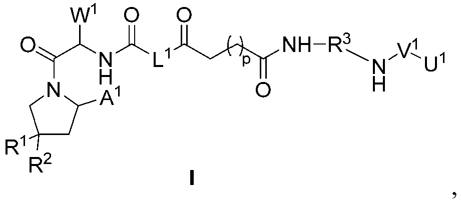
wherein R¹ and R² are independently hydrogen, halogen, C₁₋₆ alkyl, C₁₋₆ alkoxy, or hydroxyl, and R¹ and R² are not simultaneously C₁₋₆ alkyl, C₁₋₆ alkoxy, or hydroxyl;
A¹ is -CN, -B(OH)₂, -CONH₂, or -COOR^{a};
R^{a} is hydrogen or C₁₋₆ alkyl;
W¹ is hydrogen or C₁₋₄ alkyl;
L¹ is 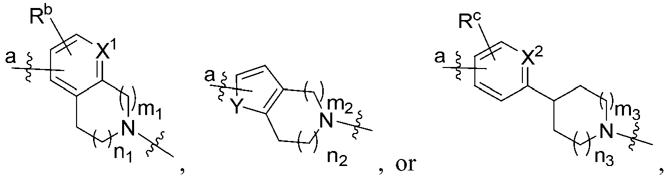 wherein the a-terminus is connected to the carbonyl group in the amide bond on the left side;
X¹ and X² are independently N or CH;
Y is O, S, or NH;
R^{b} and R^{c} are independently hydrogen, halogen, C₁₋₆ alkyl, or C₁₋₆ alkoxy;
n₁, n₂, and n₃ are independently 0, 1, or 2;
m₁, m₂, and m₃ are independently 0, 1, or 2;
p is 1, 2, 3, or 4;
R³ is independently C₁₋₆ alkylene;
V¹ is a chemical bond, -C(=O)-C₁₋₆ alkylene-NH-, or -C(=O)-CH₂-(OCH₂CH₂)ₛ-NH-, wherein s is 1, 2, 3, or 4, and the -NH- in -C(=O)-C₁₋₆ alkylene-NH- and -C(=O)-CH₂-(OCH₂CH₂)ₛ-NH- is connected to U¹;
U¹ is a group containing a radioactive metal ion or a group capable of optical imaging.

2. The compound of formula I, the pharmaceutically acceptable salt thereof, the solvate thereof, or the solvate of the pharmaceutically acceptable salt thereof according to claim 1, wherein the compound of formula I satisfies one or more of the following conditions:
(1) R¹ and R² are independently hydrogen or halogen;
(2) A¹ is CN;
(3) W¹ is hydrogen or C₁₋₄ alkyl;
(4) R^{b} and R^{c} are independently hydrogen;
(5) p is 1;
(6) Y is S;
(7) V¹ is a chemical bond or -C(=O)-C₁₋₆ alkylene-NH-;
(8) the group containing a radioactive metal ion is composed of a radioactive metal ion and a group having a function of chelating a metal ion, wherein the radioactive metal ion is chelated with the group having a function of chelating a metal ion;
(9) the group capable of optical imaging is a fluorescent group.

3. The compound of formula I, the pharmaceutically acceptable salt thereof, the solvate thereof, or the solvate of the pharmaceutically acceptable salt thereof according to claim 2, wherein the compound of formula I satisfies one or more of the following conditions:
(1) the group having a function of chelating a metal ion is 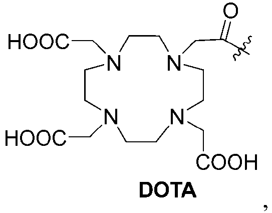 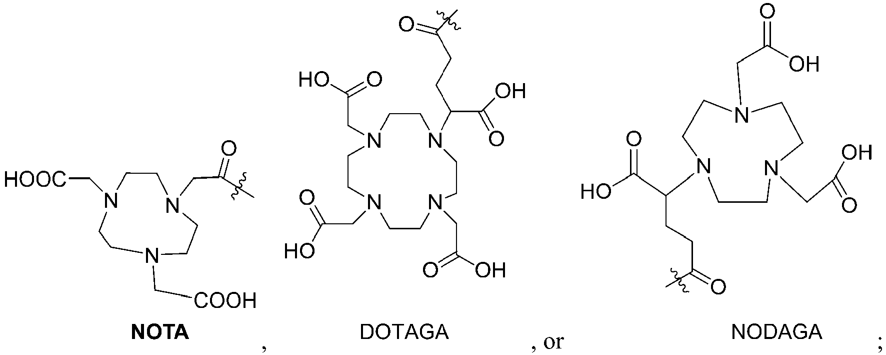
(2) the radioactive metal ion has one or more of the following effects: (i) tracing; (ii) delivery; (iii) imaging; (iv) therapy;
(3) the radioactive metal ion is ¹⁷⁷Lu³⁺, ⁶⁸Ga³⁺, ⁶⁴Cu²⁺, ²²⁵Ac³⁺, ⁹⁰Y³⁺, ⁸⁹Zr⁴⁺, ²¹²Pb²⁺, ²¹³Bi³⁺, or ²²⁷Th⁴⁺;
(4) the group capable of optical imaging is cy3, cy5, or cy7;
(5) the compound of formula I is a compound of formula Ia:

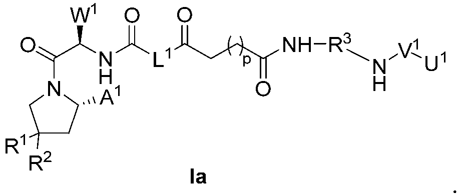

4. The compound of formula I, the pharmaceutically acceptable salt thereof, the solvate thereof, or the solvate of the pharmaceutically acceptable salt thereof according to claim 1, wherein R¹ and R² are independently hydrogen or halogen;
A¹ is CN;
W¹ is hydrogen or C₁₋₄ alkyl;
L¹ is 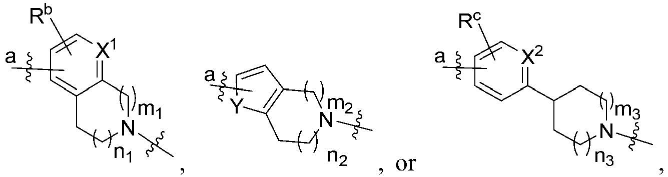 wherein the a-terminus is connected to the carbonyl group in the amide bond on the left side;
X¹ and X² are independently N or CH;
Y is S;
R^{b} and R^{c} are independently hydrogen;
n₁, n₂, and n₃ are independently 0, 1, or 2;
m₁, m₂, and m₃ are independently 0, 1, or 2;
p is 1;
R³ is C₁₋₆ alkylene;
V¹ is a chemical bond or -C(=O)-C₁₋₆ alkylene-NH-, wherein the -NH- in -C(=O)-C₁₋₆ alkylene-NH- is connected to U¹;
U¹ is a group containing a radioactive metal ion or a group capable of optical imaging.

5. The compound of formula I, the pharmaceutically acceptable salt thereof, the solvate thereof, or the solvate of the pharmaceutically acceptable salt thereof according to claim 1, wherein the compound of formula I satisfies one or more of the following conditions:
(1) R¹ and R² are independently hydrogen or fluorine;
(2) W¹ is hydrogen or methyl;
(3) L¹ is 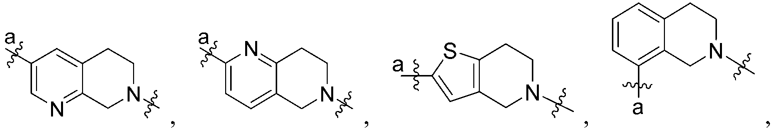 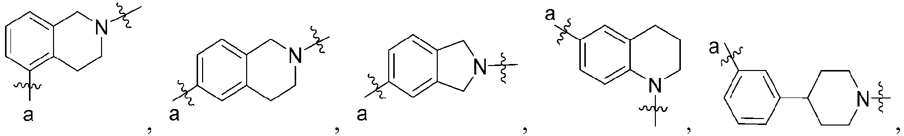 or 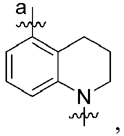 wherein the a-terminus is connected to the carbonyl group in the amide bond on the left side;
(4) R³ is -CH₂CH₂-;
(5) V¹ is a chemical bond or 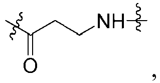 wherein the -NH- in 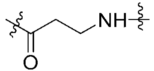 is connected to U¹;
(6) U¹ is

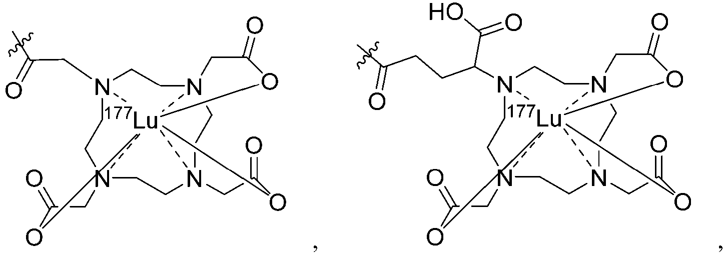

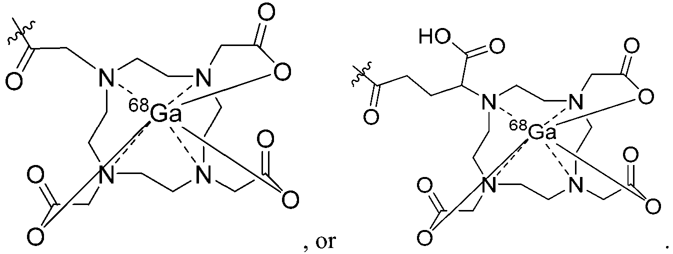

6. The compound of formula I, the pharmaceutically acceptable salt thereof, the solvate thereof, or the solvate of the pharmaceutically acceptable salt thereof according to claim 1, wherein the compound of formula I is a compound formed by chelating the radioactive metal ion according to claim 3 with compound A, wherein the structure of compound A is any one of the following: 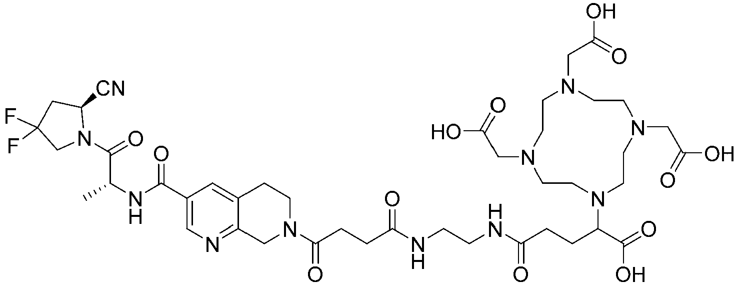 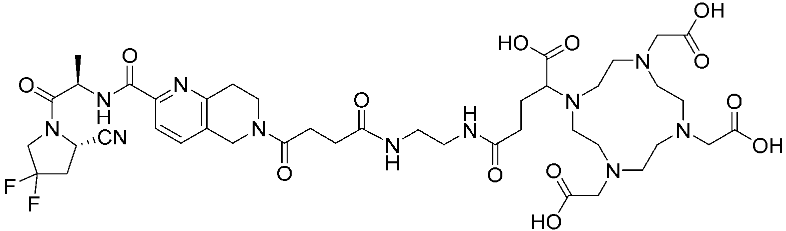 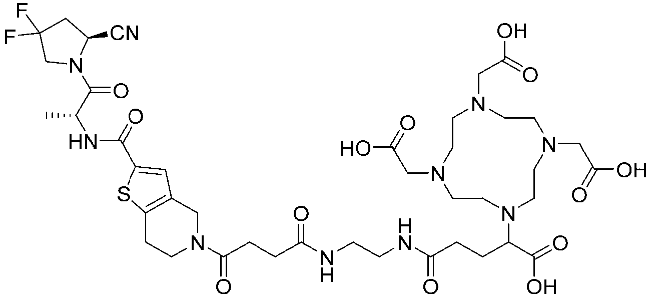 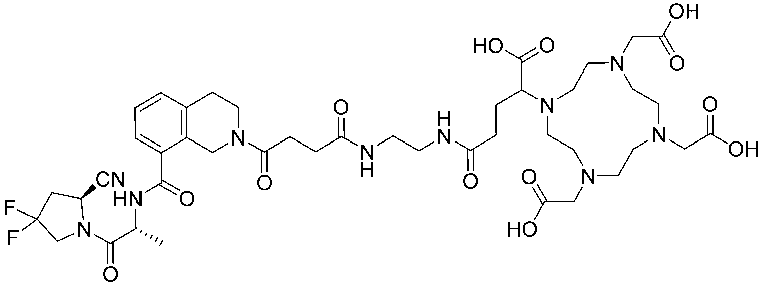 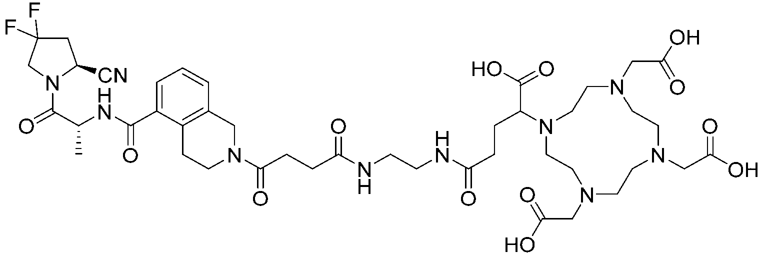 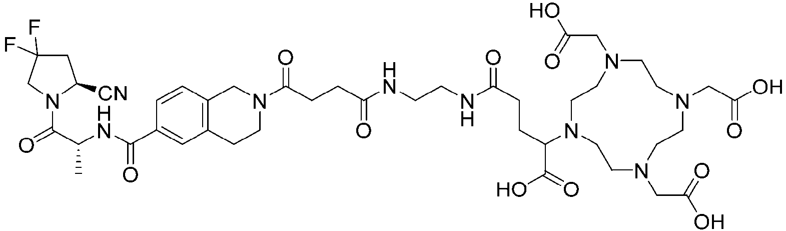 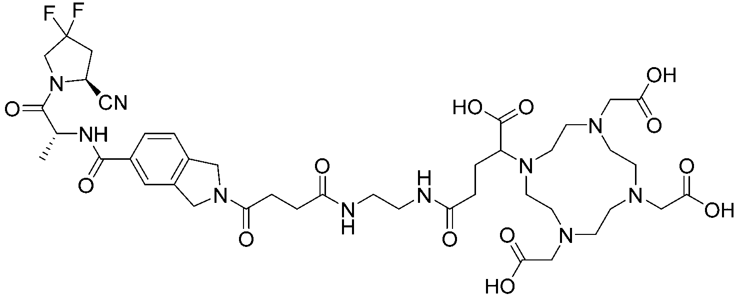 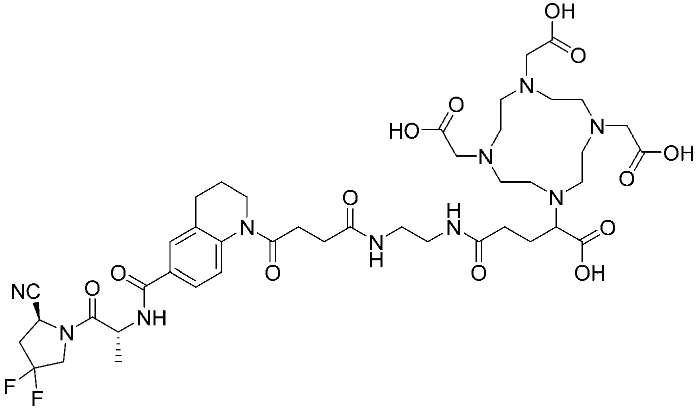 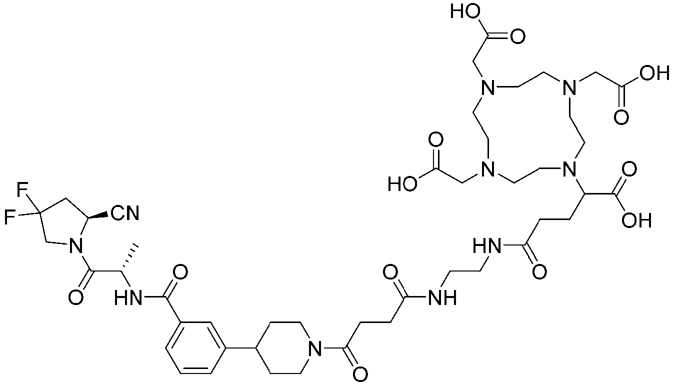 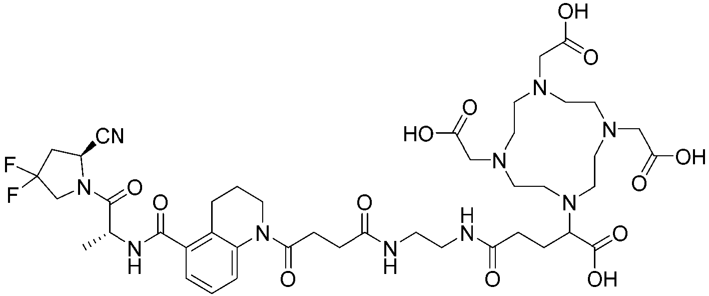 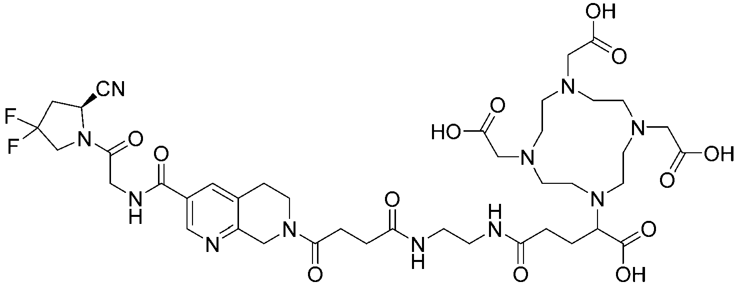 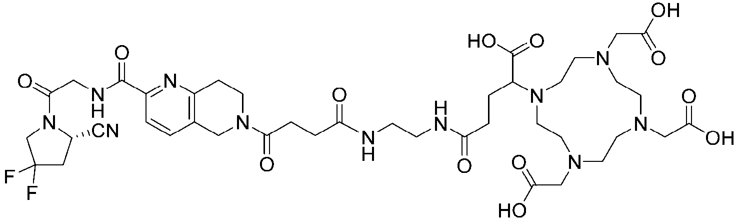 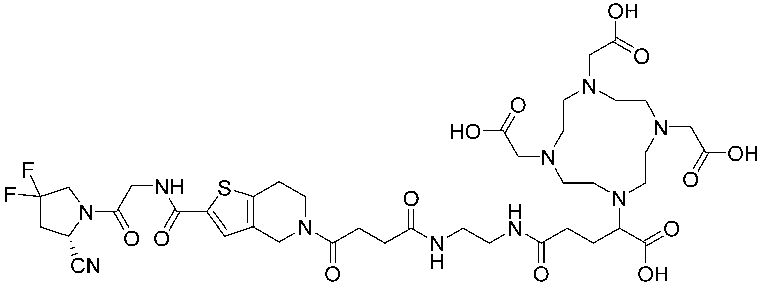 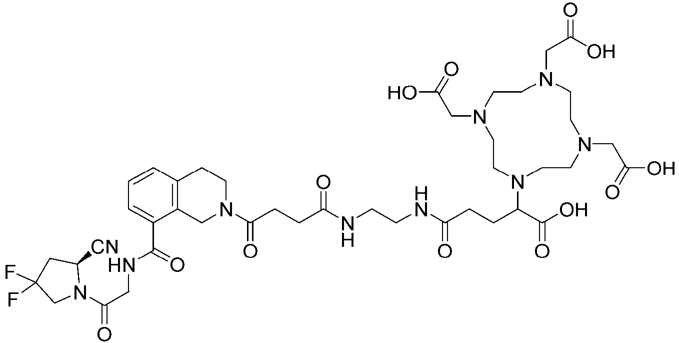 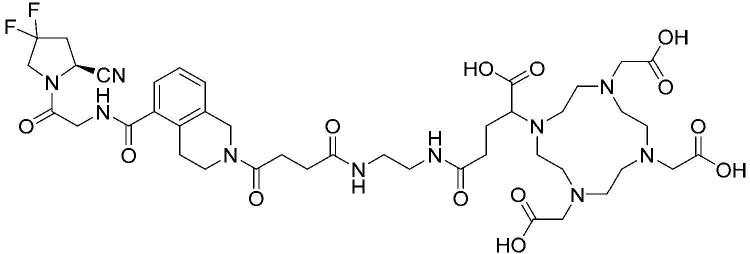 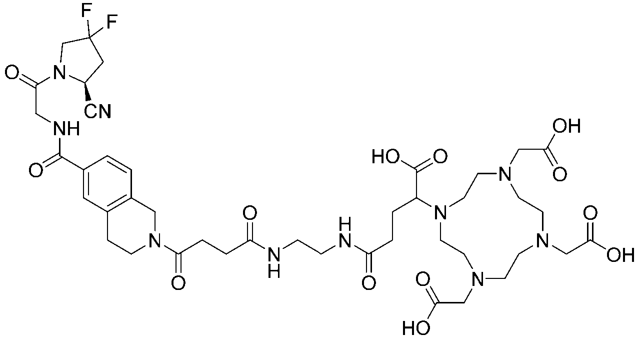 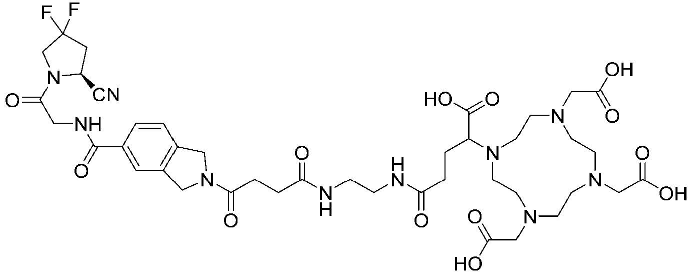 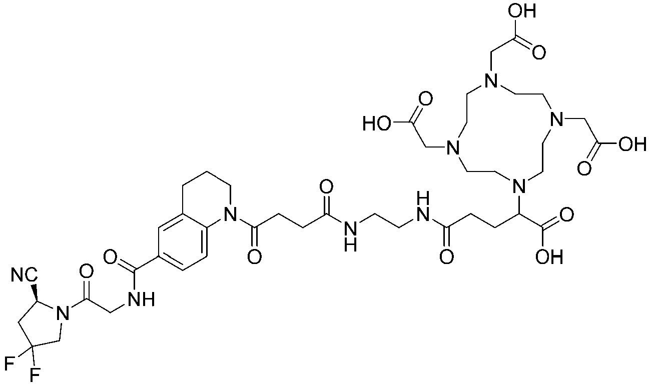 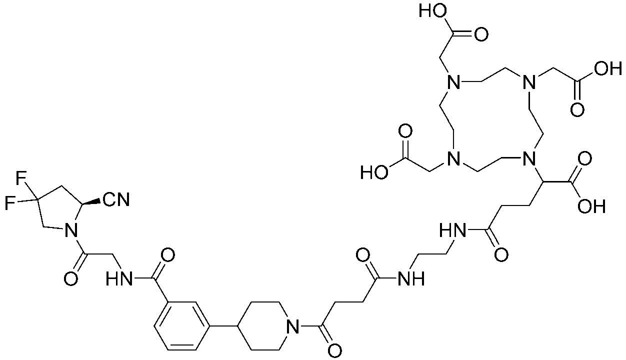 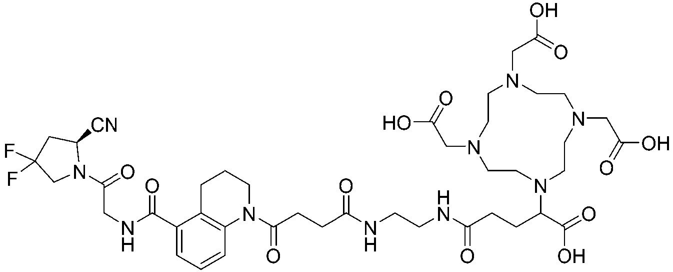 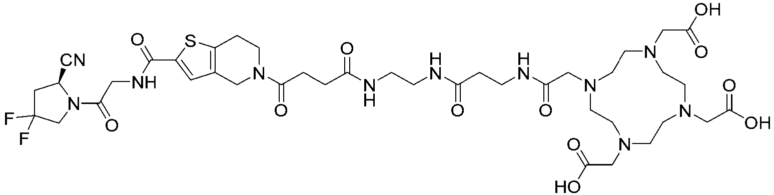 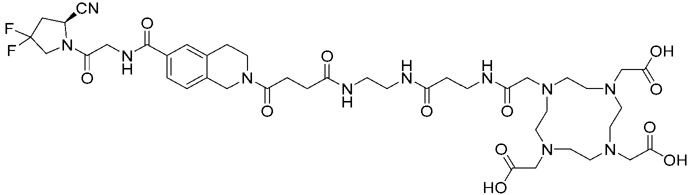 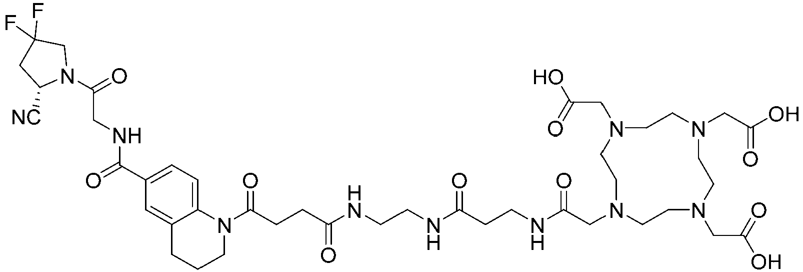 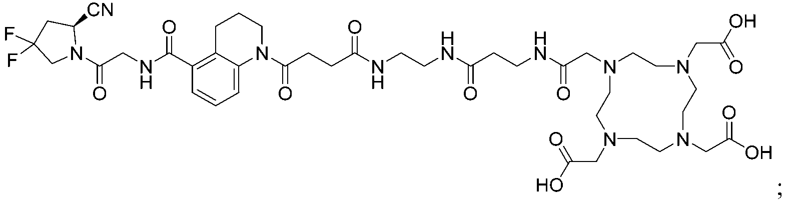
preferably, the compound of formula I is a compound formed by chelating ¹⁷⁷Lu³⁺ with compound A;
preferably, the compound of formula I is a compound formed by chelating ⁶⁸Ga³⁺ with compound A.

7. A preparation method for the compound of formula I according to any one of claims 1 to 6, comprising the following steps: chelating the radioactive metal ion according to claim 3 with a compound of formula II; 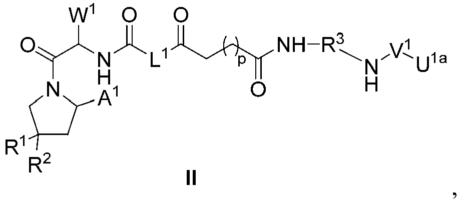 wherein U^{1a} is a group having a function of chelating a metal ion, and the group having a function of chelating a metal ion may be as defined in claim 3.

8. A compound of formula II, a pharmaceutically acceptable salt thereof, a solvate thereof, or a solvate of the pharmaceutically acceptable salt thereof, 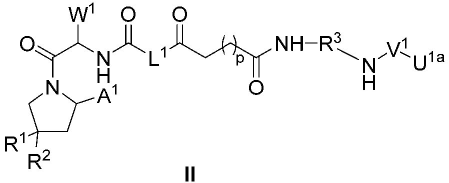 wherein U^{1a} is as defined in claim 7, and R¹, R², A¹, W¹, L¹, p, R³, and V¹ are as defined in any one of claims 1 to 5.

9. The compound of formula II, the pharmaceutically acceptable salt thereof, the solvate thereof, or the solvate of the pharmaceutically acceptable salt thereof according to claim 8, wherein the compound of formula II is a compound of formula IIa: 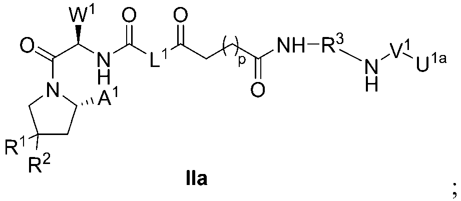 preferably, the compound of formula II is any one of compound A, wherein compound A is as defined in claim 6.

10. A compound of formula III, a pharmaceutically acceptable salt thereof, a solvate thereof, or a solvate of the pharmaceutically acceptable salt thereof; 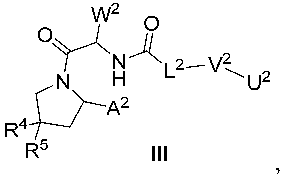
wherein R⁴ and R⁵ are independently hydrogen, halogen, C₁₋₆ alkyl, C₁₋₆ alkoxy, or hydroxyl, and R⁴ and R⁵ are not simultaneously C₁₋₆ alkyl, C₁₋₆ alkoxy, or hydroxyl;
A² is -CN, -B(OH)₂, -CONH₂, or -COOR^{d};
R^{d} is hydrogen or C₁₋₆ alkyl;
W² is hydrogen, C₁₋₄ alkyl, or HO-C₁₋₄ alkyl-;
L² is phenyl, phenyl substituted by one or more R^{e}, or 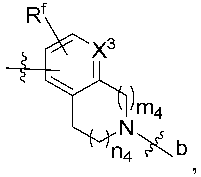 wherein the b-terminus is connected to V²;
R^{e} and R^{f} are independently hydrogen, halogen, C₁₋₆ alkyl, or C₁₋₆ alkoxy;
m₄ and n₄ are independently 0, 1, or 2;
X³ is CH or N;
V² is a chemical bond, -C₁₋₆ alkylene-NH-, or -C(=O)-C₁₋₆ alkylene-C(=O)-5- to 10-membered heterocycloalkyl, wherein the -NH-in -C₁₋₆ alkylene-NH- is connected to U², and the 5- to 10-membered heterocycloalkyl terminus in -C(=O)-C₁₋₆ alkylene-C(=O)-5- to 10-membered heterocycloalkyl is connected to U²;
U² is a group containing a radioactive metal ion or a group capable of optical imaging.

11. The compound of formula III, the pharmaceutically acceptable salt thereof, the solvate thereof, or the solvate of the pharmaceutically acceptable salt thereof according to claim 10, wherein the compound of formula III satisfies one or more of the following conditions:
(1) R⁴ and R⁵ are independently hydrogen;
(2) A² is -B(OH)₂;
(3) R^{e} and R^{f} are independently hydrogen or halogen;
(4) X³ is CH;
(5) m₄ and n₄ are independently 1;
(6) when L² is phenyl or phenyl substituted by one or more R^{e}, then V² is -C₁₋₆ alkylene-NH-, wherein the -NH- in -C₁₋₆ alkylene-NH- is connected to U²;
(7) when L² is 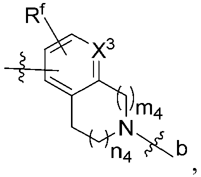 then V² is a chemical bond or -C(=O)-C₁₋₆ alkylene-C(=O)-5- to 10-membered heterocycloalkyl, wherein the 5- to 10-membered heterocycloalkyl terminus in -C(=O)-C₁₋₆ alkylene-C(=O)-5- to 10-membered heterocycloalkyl is connected to U²;
(8) the group containing a radioactive metal ion is as defined in claim 2;
(9) the group capable of optical imaging is as defined in claim 2 or 3;
(10) the compound of formula III is a compound of formula IIIa:

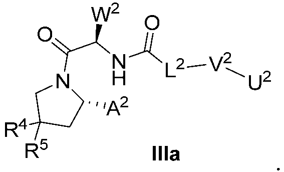

12. The compound of formula III, the pharmaceutically acceptable salt thereof, the solvate thereof, or the solvate of the pharmaceutically acceptable salt thereof according to claim 10, wherein R⁴ and R⁵ are independently hydrogen;
A² is -B(OH)₂;
W² is hydrogen, C₁₋₄ alkyl, or HO-C₁₋₄ alkyl-;
L² is phenyl, phenyl substituted by one or more R^{e}, or 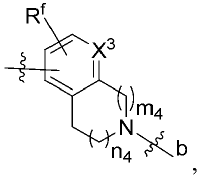 wherein the b-terminus is connected to V²;
R^{e} and R^{f} are independently hydrogen or halogen;
X³ is CH;
m₄ and n₄ are independently 1;
V² is a chemical bond, -C₁₋₆ alkylene-NH-, or -C(=O)-C₁₋₆ alkylene-C(=O)-5- to 10-membered heterocycloalkyl, wherein the -NH-in -C₁₋₆ alkylene-NH- is connected to U², and the 5- to 10-membered heterocycloalkyl terminus in -C(=O)-C₁₋₆ alkylene-C(=O)-5- to 10-membered heterocycloalkyl is connected to U²;
U² is a group containing a radioactive metal ion or a group capable of optical imaging.

13. The compound of formula III, the pharmaceutically acceptable salt thereof, the solvate thereof, or the solvate of the pharmaceutically acceptable salt thereof according to claim 10, wherein the compound of formula III satisfies one or more of the following conditions:
(1) W² is methyl, 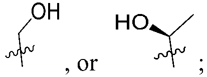
(2) L² is 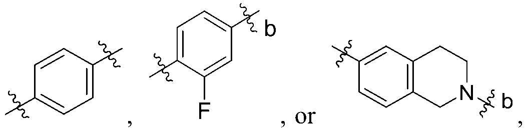 wherein the b-terminus is connected to V²;
(3) V² is a chemical bond, -CH₂-NH-, or 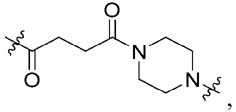 wherein the -NH- in - CH₂-NH- is connected to U², and the piperazine terminus in 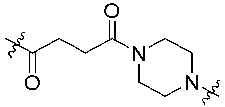 is connected to U²;
(4) U² is

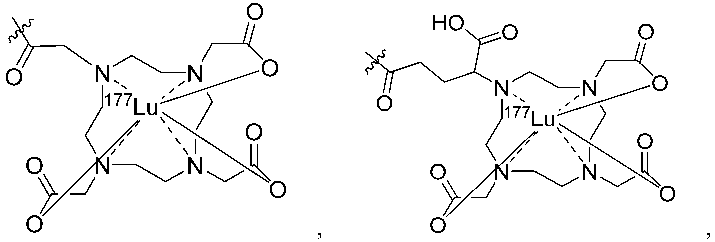

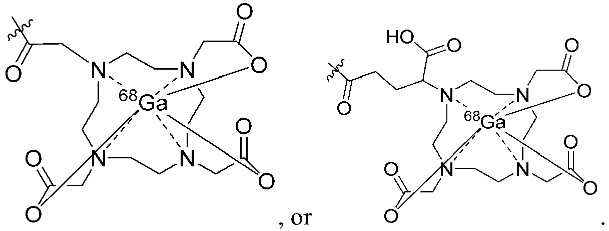

14. The compound of formula III, the pharmaceutically acceptable salt thereof, the solvate thereof, or the solvate of the pharmaceutically acceptable salt thereof according to claim 10, wherein the compound of formula III is a compound formed by chelating the radioactive metal ion according to claim 3 with compound B, wherein the structure of compound B is any one of the following: 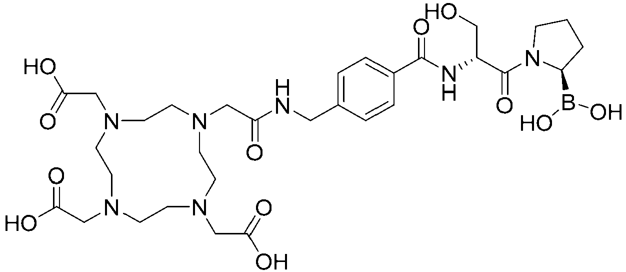 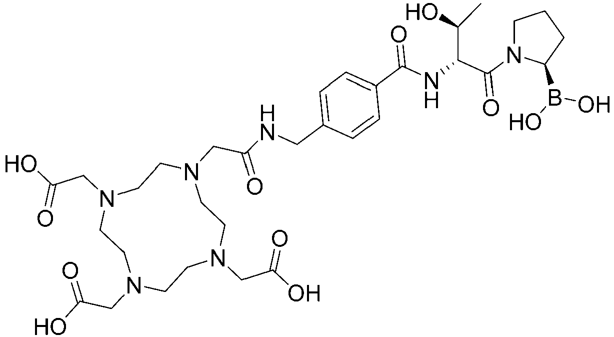 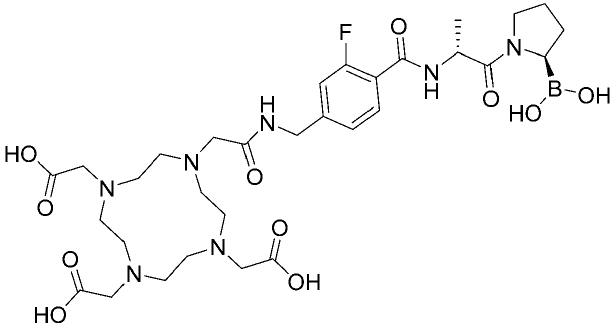 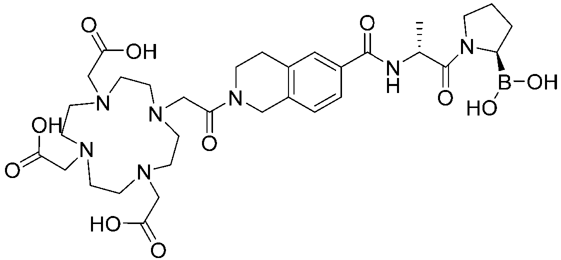 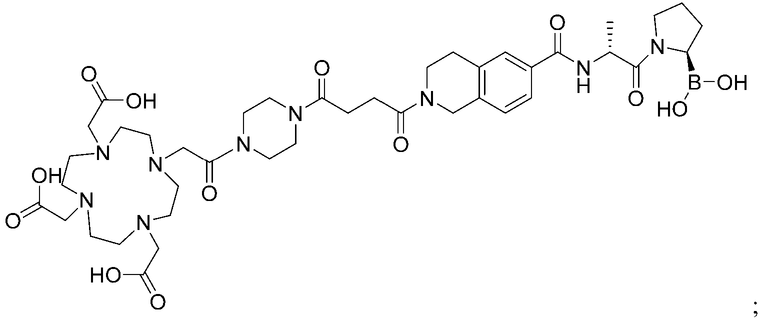
preferably, the compound of formula III is a compound formed by chelating ¹⁷⁷Lu³⁺ with compound B;
preferably, the compound of formula III is a compound formed by chelating ⁶⁸Ga³⁺ with compound B.

15. A preparation method for the compound of formula III according to any one of claims 10 to 14, comprising the following steps: chelating the radioactive metal ion according to claim 3 with a compound of formula IV; 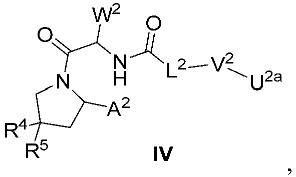 wherein u^{2a} is a group having a function of chelating a metal ion, and the group having a function of chelating a metal ion may be as defined in claim 3.

16. A compound of formula IV, a pharmaceutically acceptable salt thereof, a solvate thereof, or a solvate of the pharmaceutically acceptable salt thereof, 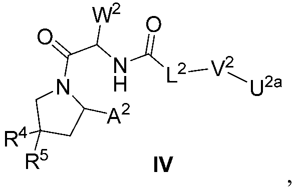 wherein R⁴, R⁵*,* A², W², L², and V² are as defined in any one of claims 10 to 13, and U^{2a} is as defined in claim 15.

17. The compound of formula IV, the pharmaceutically acceptable salt thereof, the solvate thereof, or the solvate of the pharmaceutically acceptable salt thereof according to claim 16, wherein the compound of formula IV is a compound of formula IVa: 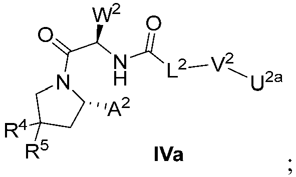 preferably, the compound of formula IV is any one of compound B, wherein compound B is as defined in claim 14.

18. A compound of formula V, a pharmaceutically acceptable salt thereof, a solvate thereof, or a solvate of the pharmaceutically acceptable salt thereof, 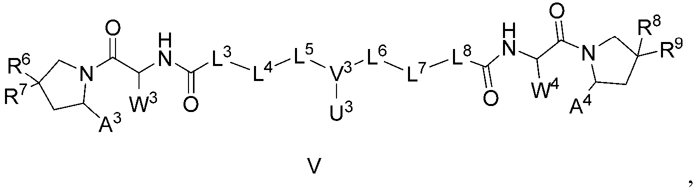
wherein R⁶, R⁷, R⁸, and R⁹ are independently hydrogen, halogen, C₁₋₆ alkyl, C₁₋₆ alkoxy, or hydroxyl, and R⁶ and R⁷ are not simultaneously C₁₋₆ alkyl, C₁₋₆ alkoxy, or hydroxyl, and R⁸ and R⁹ are not simultaneously C₁₋₆ alkyl, C₁₋₆ alkoxy, or hydroxyl;
A³ and A⁴ are independently -CN, -B(OH)₂, -CONH₂, or -COOR^{g};
R^{g} is hydrogen or C₁₋₆ alkyl;
W³ and W⁴ are independently hydrogen or C₁₋₄ alkyl;
L³ and L⁸ are independently phenyl, phenyl substituted by one or more R^{h}, 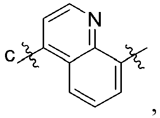 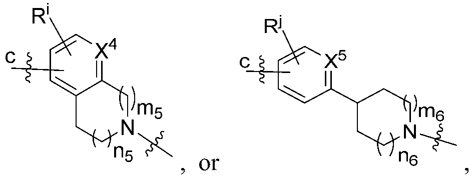 wherein the c-terminus is connected to the carbonyl group in the amide bond;
R^{h}, Rⁱ, and R^{j} are independently hydrogen, halogen, C₁₋₆ alkyl, or C₁₋₆ alkoxy; X⁴ and X⁵ are independently CH or N;
m₅, m₆, n₅, and n₆ are independently 0, 1, or 2;
L⁴ and L⁷ are independently a chemical bond or C₁₋₆ alkylene;
L⁵ and L⁶ are independently 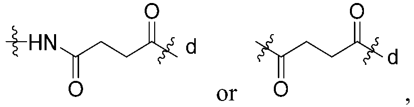 wherein the d-terminus is connected to V³;
V³ is 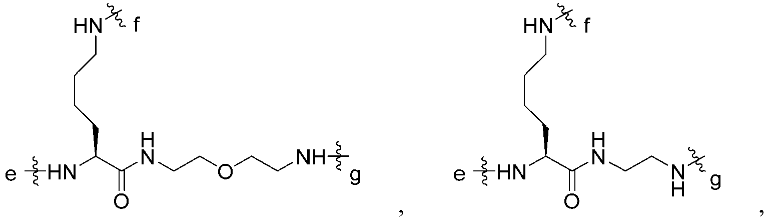 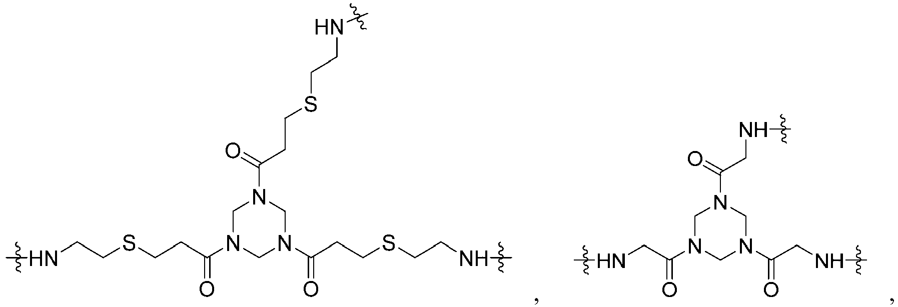 or 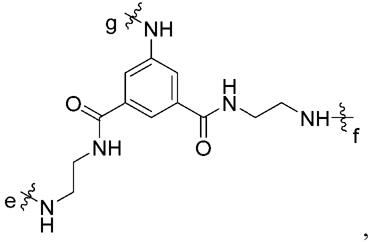 wherein the e-terminus is connected to L⁵ or L⁶, the f-terminus is connected to L⁵ or L⁶, and the g-terminus is connected to U³;
U³ is a group containing a radioactive metal ion or a group capable of optical imaging.

19. The compound of formula V, the pharmaceutically acceptable salt thereof, the solvate thereof, or the solvate of the pharmaceutically acceptable salt thereof according to claim 18, wherein the compound of formula V satisfies one or more of the following conditions:
(1) R⁶, R⁷, R⁸, and R⁹ are independently hydrogen or halogen;
(2) A³ and A⁴ are independently -CN or -B(OH)₂;
(3) Rⁱ and R^{j} are independently hydrogen;
(4) X⁴ and X⁵ are independently CH;
(5) m₅, m₆, n₅, and n₆ are independently 1;
(6) when L³ and L⁸ are independently phenyl, then L⁴ and L⁷ are independently C₁₋₆ alkylene;
(7) when L³ and L⁸ are independently 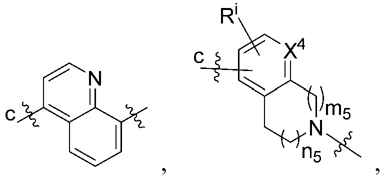 or 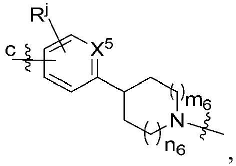 then L⁴ and L⁷ are independently a chemical bond;
(8) in the compound of formula V, the group containing a radioactive metal ion is as defined in claim 2;
(9) in the compound of formula V, the group capable of optical imaging is as defined in claim 2 or 3;
(10) the compound of formula V is a compound of formula Va:

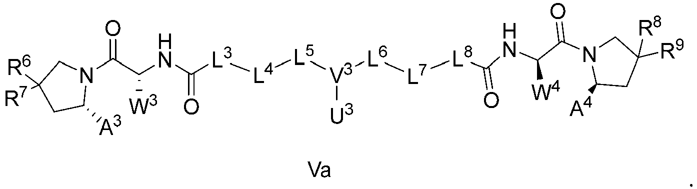

20. The compound of formula V, the pharmaceutically acceptable salt thereof, the solvate thereof, or the solvate of the pharmaceutically acceptable salt thereof according to claim 18, wherein R⁶, R⁷, R⁸, and R⁹ are independently hydrogen or halogen;
A³ and A⁴ are independently -CN or -B(OH)₂;
W³ and W⁴ are independently hydrogen or C₁₋₄ alkyl;
L³ and L⁸ are independently phenyl, 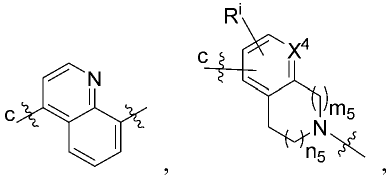 or 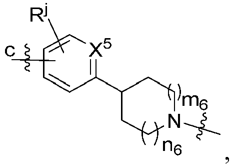 wherein the c-terminus is connected to the carbonyl group in the amide bond;
Rⁱ and R^{j} are independently hydrogen;
X⁴ and X⁵ are independently CH;
m₅, m₆, n₅, and n₆ are independently 1;
L⁴ and L⁷ are independently a chemical bond or C₁₋₆ alkylene;
L⁵ and L⁶ are independently 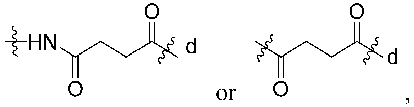 wherein the d-terminus is connected to V³;
V³ is 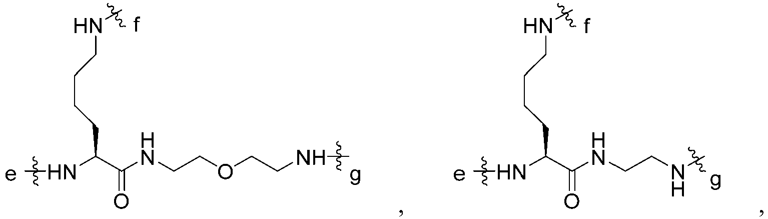 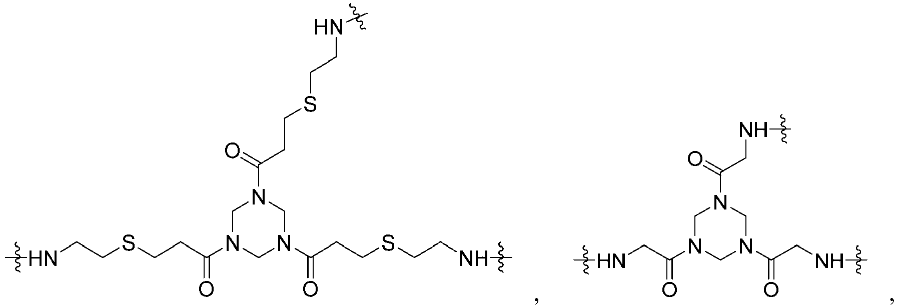 or 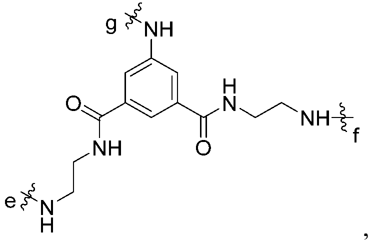 wherein the e-terminus is connected to L⁵ or L⁶, the f-terminus is connected to L⁵ or L⁶, and the g-terminus is connected to U³;
U³ is a group containing a radioactive metal ion or a group capable of optical imaging.

21. The compound of formula V, the pharmaceutically acceptable salt thereof, the solvate thereof, or the solvate of the pharmaceutically acceptable salt thereof according to claim 18, wherein the compound of formula V satisfies one or more of the following conditions:
(1) R⁶, R⁷, R⁸, and R⁹ are independently hydrogen or fluorine;
(2) W³ and W⁴ are independently hydrogen or methyl;
(3) L³ and L⁸ are independently 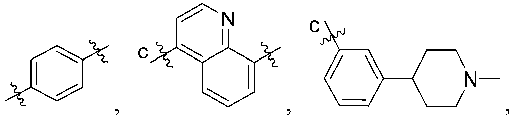 or 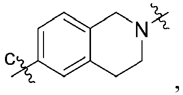 wherein the c-terminus is connected to the carbonyl group in the amide bond;
(4) U³ is

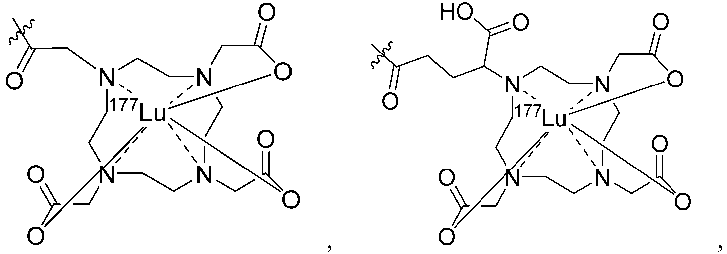

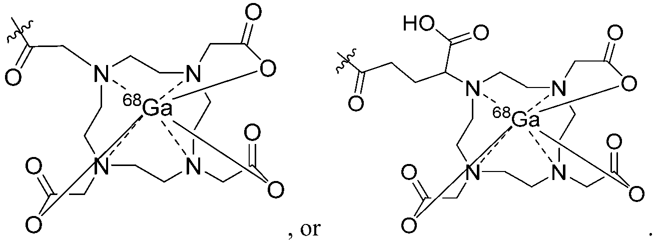

22. The compound of formula V, the pharmaceutically acceptable salt thereof, the solvate thereof, or the solvate of the pharmaceutically acceptable salt thereof according to claim 18, wherein the compound of formula V is a compound formed by chelating the radioactive metal ion according to claim 3 with compound C, wherein the structure of compound C is any one of the following: 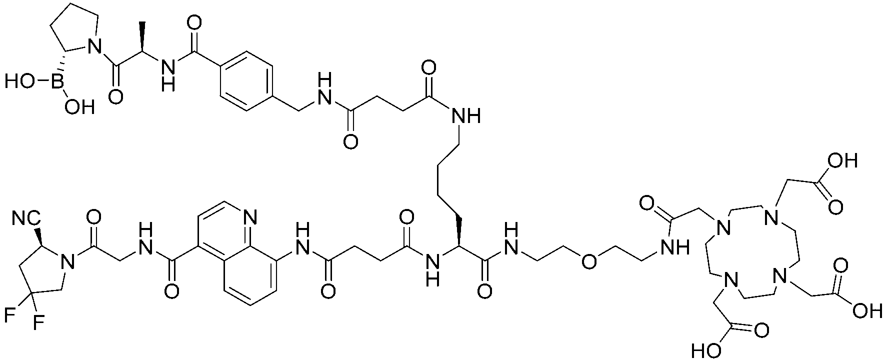 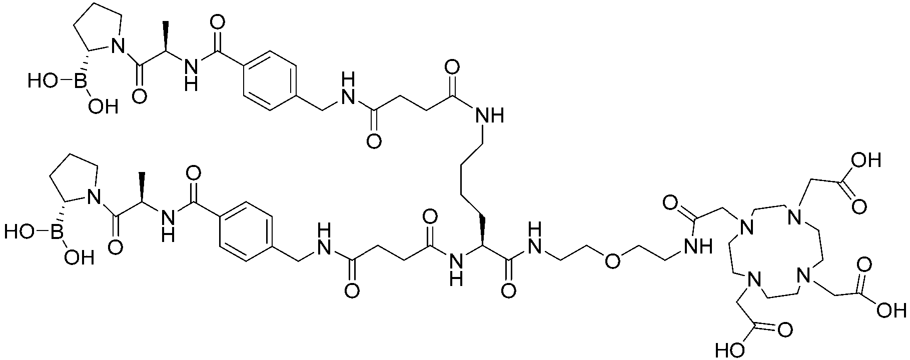 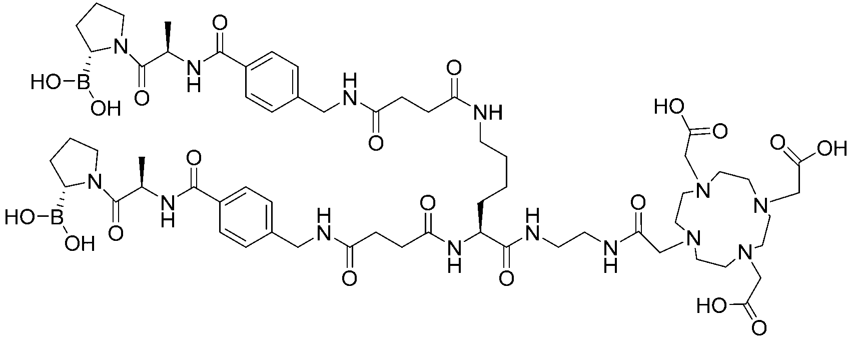 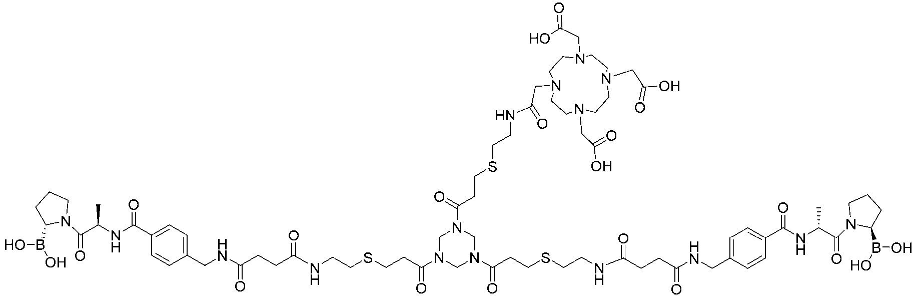 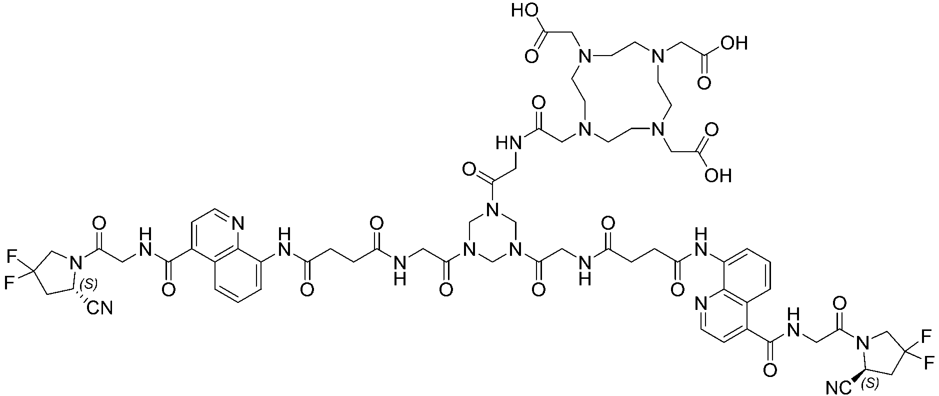 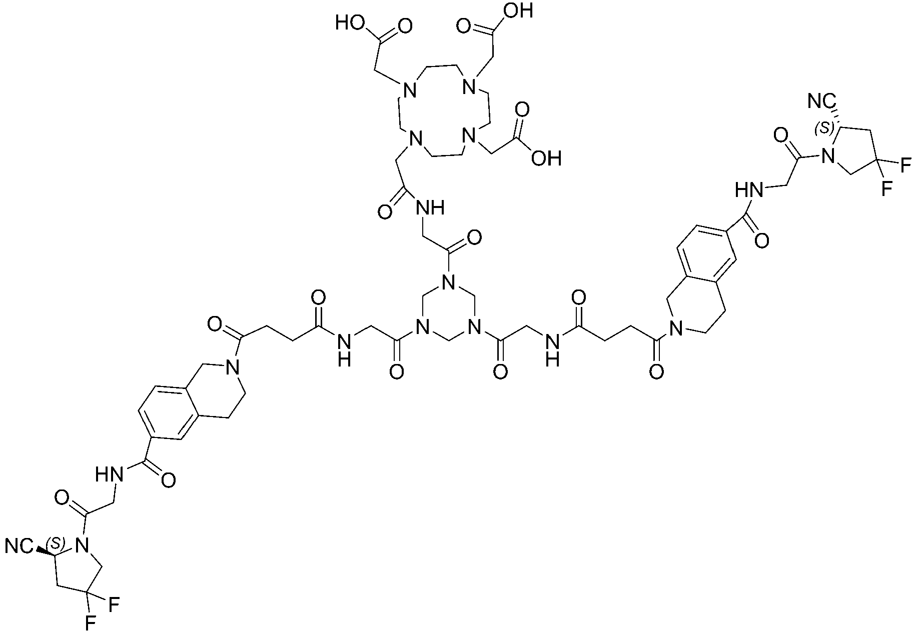 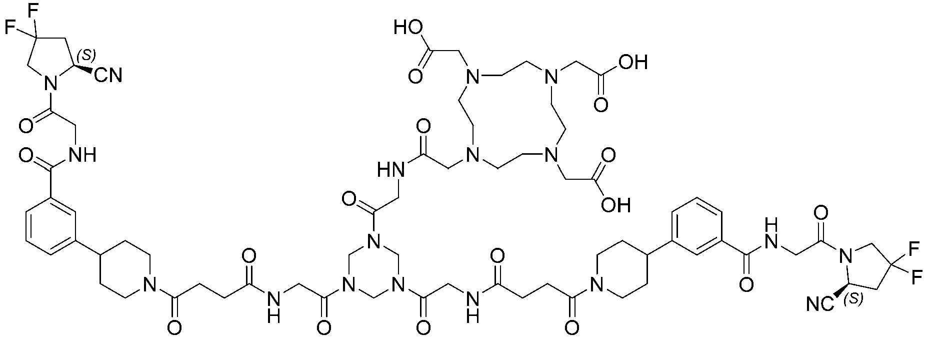 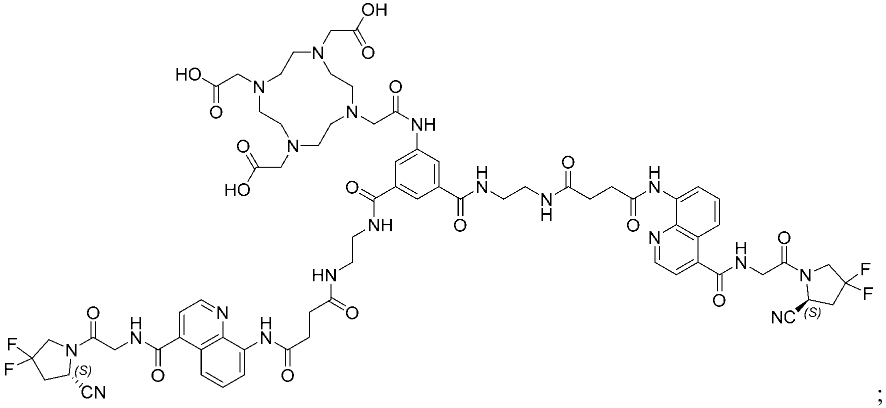
preferably, the compound of formula V is a compound formed by chelating ¹⁷⁷Lu³⁺ with compound C;
preferably, the compound of formula V is a compound formed by chelating ⁶⁸Ga³⁺ with compound C.

23. A preparation method for the compound of formula V according to any one of claims 18 to 22, comprising the following steps: chelating the radioactive metal ion according to claim 3 with a compound of formula VI; 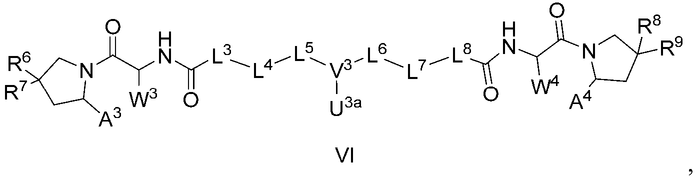 wherein U^{3a} is a group having a function of chelating a metal ion, and the group having a function of chelating a metal ion may be as defined in claim 3.

24. A compound of formula VI, a pharmaceutically acceptable salt thereof, a solvate thereof, or a solvate of the pharmaceutically acceptable salt thereof, 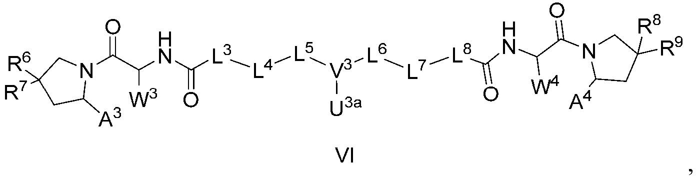 wherein R⁶, R⁷, R⁸, R⁹, A³, W³, W⁴, L³, L⁴, L⁵, L⁶, L⁷, L⁸, and V³ are as defined in any one of claims 18 to 21, and U^{3a} is as defined in claim 23.

25. The compound of formula VI, the pharmaceutically acceptable salt thereof, the solvate thereof, or the solvate of the pharmaceutically acceptable salt thereof according to claim 24, wherein the compound of formula VI is a compound of formula VIa: 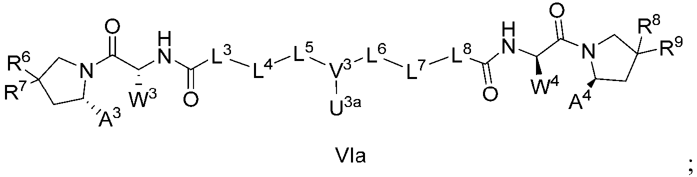 preferably, the compound of formula VI is any one of compound C, wherein compound C is as defined in claim 22.

26. A compound of formula VII, a pharmaceutically acceptable salt thereof, a solvate thereof, or a solvate of the pharmaceutically acceptable salt thereof, 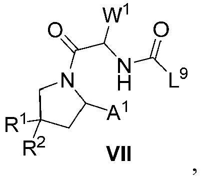
wherein L⁹ is 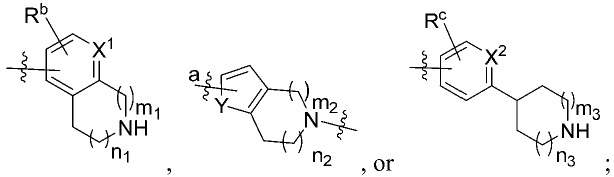
R¹, R², A¹, W¹, X¹, X², Y, R^{b}, R^{c}, m₁, m₂, m₃, n₁, n₂, and n₃ are as defined in any one of claims 1 to 5;
preferably, L⁹ is 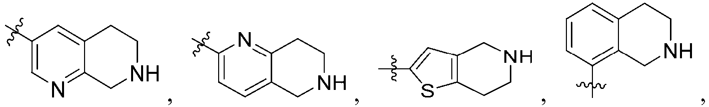 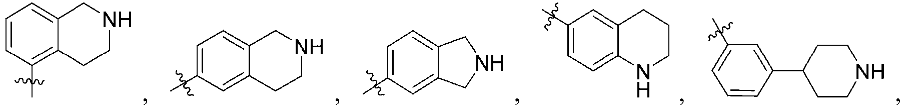 or

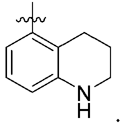

27. The compound of formula VII, the pharmaceutically acceptable salt thereof, the solvate thereof, or the solvate of the pharmaceutically acceptable salt thereof according to claim 26, wherein the compound of formula VII is a compound of formula VIIa: 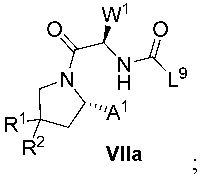 preferably, the compound of formula VII is any one of the following compounds:

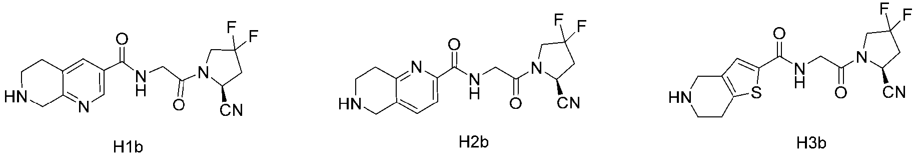

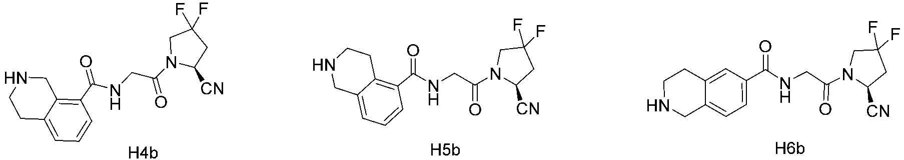

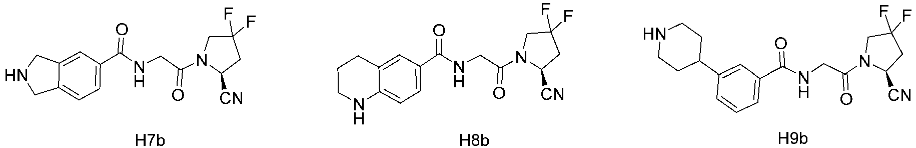

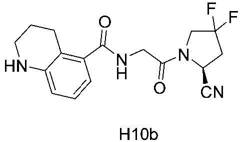

28. A pharmaceutical composition comprising substance X and a pharmaceutically acceptable excipient; wherein substance X is substance Y, a pharmaceutically acceptable salt thereof, a solvate thereof, or a solvate of the pharmaceutically acceptable salt thereof, and substance Y is the compound of formula I according to any one of claims 1 to 6, the compound of formula III according to any one of claims 10 to 14, or the compound of formula V according to any one of claims 18 to 22.

29. A use of substance X in the manufacture of a medicament; wherein substance X is substance Y, a pharmaceutically acceptable salt thereof, a solvate thereof, or a solvate of the pharmaceutically acceptable salt thereof, and substance Y is the compound of formula I according to any one of claims 1 to 6, the compound of formula III according to any one of claims 10 to 14, or the compound of formula V according to any one of claims 18 to 22;
the medicament is for treating or diagnosing a tumor, or tumor imaging.

30. The use according to claim 29, wherein the use satisfies one or more of the following conditions:
(1) when the medicament is for diagnosing a tumor, the radioactive metal ion is ⁶⁸Ga³⁺;
(2) when the medicament is for treating a tumor, the radioactive metal ion is ¹⁷⁷Lu³⁺;
(3) the tumor is breast cancer, ovarian cancer, lung cancer, colorectal cancer, gastric cancer, pancreatic cancer, or cutaneous melanoma.
